(19) Europäisches Patentamt
European Patent Office
Office européen des brevets

(11) EP 3 216 458 A1

# (12) EUROPEAN PATENT APPLICATION

(43) Date of publication:
**13.09.2017 Bulletin 2017/37**

(51) Int Cl.:
***A61K 38/18*** *(2006.01)* ***C07K 14/515*** *(2006.01)*

(21) Application number: **16159048.4**

(22) Date of filing: **07.03.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Max-Planck-Gesellschaft zur Förderung der Wissenschaften e.V.**
**80539 München (DE)**

(72) Inventors:
- **STAINIERT, Didier**
  **61381 Friedrichsdorf (DE)**
- **ROSSI, Andrea**
  **61231 Bad Nauheim (DE)**
- **MARASS, Michele**
  **61231 Bad Nauheim (DE)**
- **GAUVRIT, Sébastien**
  **61231 Bad Nauheim (DE)**

(74) Representative: **Vossius & Partner**
**Patentanwälte Rechtsanwälte mbB**
**Siebertstrasse 3**
**81675 München (DE)**

## (54) MODIFIED VASCULAR ENDOTHELIAL GROWTH FACTOR A (VEGF-A) AND ITS MEDICAL USE

(57) The present invention relates to a modified vascular endothelial growth factor A (VEGF-A) polypeptide, particularly to a modified VEGF-A 121 isoform for use in treating an angiogenic disorder. The modified vascular endothelial growth factor A (VEGF-A) comprises a hydrophobic amino acid in place of the lysine at the position corresponding to position 84 of the wild-type VEGF-A as shown in SEQ ID NO: 2, and/or the modified vascular endothelial growth factor A (VEGF-A) comprises a hydrophobic amino acid in place of the phenylalanine at the position corresponding to position 17 of the wild-type VEGF-A as shown in SEQ ID NO: 2.

EP 3 216 458 A1

SEQUENCE LISTING

<110> Max-Planck-Gesellschaft zur Förderung der Wissenschaften e.V.

<120> Modified vascular endothelial growth factor A (VEGF-A) and its medical use

<130> Y1291 EP S3

<160> 53

<170> BiSSAP 1.3

<210> 1
<211> 366
<212> DNA
<213> Homo sapiens

<220>
<223> VEGF-A isoform 121

<400> 1
gcacccatgg cagaaggagg agggcagaat catcacgaag tggtgaagtt catggatgtc 60

tatcagcgca gctactgcca tccaatcgag accctggtgg acatcttcca ggagtaccct 120

gatgagatcg agtacatctt caagccatcc tgtgtgcccc tgatgcgatg cgggggctgc 180

tgcaatgacg agggcctgga gtgtgtgccc actgaggagt ccaacatcac catgcagatt 240

atgcggatca aacctcacca aggccagcac ataggagaga tgagcttcct acagcacaac 300

aaatgtgaat gcagaccaaa gaaagataga gcaagacaag aaaaatgtga caagccgagg 360

cggtga 366

<210> 2
<211> 121
<212> PRT
<213> Homo sapiens

<220>
<223> VEGF-A isoform 121

<400> 2
```
Ala Pro Met Ala Glu Gly Gly Gly Gln Asn His His Glu Val Val Lys
1               5                   10                  15
Phe Met Asp Val Tyr Gln Arg Ser Tyr Cys His Pro Ile Glu Thr Leu
            20                  25                  30
Val Asp Ile Phe Gln Glu Tyr Pro Asp Glu Ile Glu Tyr Ile Phe Lys
        35                  40                  45
Pro Ser Cys Val Pro Leu Met Arg Cys Gly Gly Cys Cys Asn Asp Glu
    50                  55                  60
Gly Leu Glu Cys Val Pro Thr Glu Glu Ser Asn Ile Thr Met Gln Ile
65                  70                  75                  80
Met Arg Ile Lys Pro His Gln Gly Gln His Ile Gly Glu Met Ser Phe
                85                  90                  95
Leu Gln His Asn Lys Cys Glu Cys Arg Pro Lys Lys Asp Arg Ala Arg
            100                 105                 110
Gln Glu Lys Cys Asp Lys Pro Arg Arg
        115                 120
```

<210> 3
<211> 366
<212> DNA
<213> Homo sapiens

<220>
<223> modified VEGF-A K84A isoform 121

<220>
<221> unsure
<222> 252
<223> /replace="n = t,c, a or g"

<400> 3
gcacccatgg cagaaggagg agggcagaat catcacgaag tggtgaagtt catggatgtc 60

tatcagcgca gctactgcca tccaatcgag accctggtgg acatcttcca ggagtaccct 120

gatgagatcg agtacatctt caagccatcc tgtgtgcccc tgatgcgatg cgggggctgc 180

tgcaatgacg agggcctgga gtgtgtgccc actgaggagt ccaacatcac catgcagatt 240

atgcggatcg cncctcacca aggccagcac ataggagaga tgagcttcct acagcacaac 300

aaatgtgaat gcagaccaaa gaaagataga gcaagacaag aaaaatgtga caagccgagg 360

cggtga 366

<210> 4
<211> 121
<212> PRT
<213> Homo sapiens

<220>
<223> modified VEGF-A K84A isoform 121

<400> 4
Ala Pro Met Ala Glu Gly Gly Gly Gln Asn His His Glu Val Val Lys
1 5 10 15
Phe Met Asp Val Tyr Gln Arg Ser Tyr Cys His Pro Ile Glu Thr Leu
20 25 30
Val Asp Ile Phe Gln Glu Tyr Pro Asp Glu Ile Glu Tyr Ile Phe Lys
35 40 45
Pro Ser Cys Val Pro Leu Met Arg Cys Gly Gly Cys Cys Asn Asp Glu
50 55 60
Gly Leu Glu Cys Val Pro Thr Glu Glu Ser Asn Ile Thr Met Gln Ile
65 70 75 80
Met Arg Ile Ala Pro His Gln Gly Gln His Ile Gly Glu Met Ser Phe
85 90 95
Leu Gln His Asn Lys Cys Glu Cys Arg Pro Lys Lys Asp Arg Ala Arg
100 105 110
Gln Glu Lys Cys Asp Lys Pro Arg Arg
115 120

<210> 5
<211> 366
<212> DNA
<213> Homo sapiens

<220>
<223> modified VEGF-A F17A isoform 121

<220>
<221> unsure
<222> 51
<223> /replace="n = t, c, a or g"

<400> 5
gcacccatgg cagaaggagg agggcagaat catcacgaag tggtgaaggc natggatgtc 60

tatcagcgca gctactgcca tccaatcgag accctggtgg acatcttcca ggagtaccct 120

gatgagatcg agtacatctt caagccatcc tgtgtgcccc tgatgcgatg cgggggctgc 180

tgcaatgacg agggcctgga gtgtgtgccc actgaggagt ccaacatcac catgcagatt 240

atgcggatca aacctcacca aggccagcac ataggagaga tgagcttcct acagcacaac 300

aaatgtgaat gcagaccaaa gaaagataga gcaagacaag aaaaatgtga caagccgagg 360

cggtga 366

<210> 6
<211> 121
<212> PRT
<213> Homo sapiens

<220>
<223> modified VEGF-A F17A isoform 121

<400> 6
Ala Pro Met Ala Glu Gly Gly Gly Gln Asn His His Glu Val Val Lys
1               5                   10                  15
Ala Met Asp Val Tyr Gln Arg Ser Tyr Cys His Pro Ile Glu Thr Leu
            20                  25                  30
Val Asp Ile Phe Gln Glu Tyr Pro Asp Glu Ile Glu Tyr Ile Phe Lys
        35                  40                  45
Pro Ser Cys Val Pro Leu Met Arg Cys Gly Gly Cys Cys Asn Asp Glu
    50                  55                  60
Gly Leu Glu Cys Val Pro Thr Glu Glu Ser Asn Ile Thr Met Gln Ile
65                  70                  75                  80
Met Arg Ile Lys Pro His Gln Gly Gln His Ile Gly Glu Met Ser Phe
                85                  90                  95
Leu Gln His Asn Lys Cys Glu Cys Arg Pro Lys Lys Asp Arg Ala Arg
            100                 105                 110
Gln Glu Lys Cys Asp Lys Pro Arg Arg
        115                 120

<210> 7
<211> 366
<212> DNA
<213> Danio rerio

<220>
<223> VEGF-A isoform 121

<400> 7
gcccacatac ccaaagaagg gggaaagagc aaaaatgatg tgattccctt catggatgtg 60

tataaaaaga gtgcgtgcaa gacccgagag ctgctggtag acatcatcca ggagtatccc 120

gatgagatcg agcacacgta catcccgtcc tgtgtggttc tcatgcgctg tgcaggatgc 180

tgtaatgatg aggcgctcga atgcgtcccg acagagacac gaaacgtcac tatggaggtg 240

```
ctgcgggtca agcaacgcgt atcgcagcat aattttcagc tgagtttcac agaacacacc     300

aagtgtgaat gcaggccaaa ggcagaagtc aaagcaaaga aagaaaaatg tgaaaagcca     360

agatga                                                                366
```

<210> 8
<211> 121
<212> PRT
<213> Danio rerio

<220>
<223> VEGF-A isoform 121

<400> 8

```
Ala His Ile Pro Lys Glu Gly Gly Lys Ser Lys Asn Asp Val Ile Pro
1               5                   10                  15
Phe Met Asp Val Tyr Lys Lys Ser Ala Cys Lys Thr Arg Glu Leu Leu
            20                  25                  30
Val Asp Ile Ile Gln Glu Tyr Pro Asp Glu Ile Glu His Thr Tyr Ile
        35                  40                  45
Pro Ser Cys Val Val Leu Met Arg Cys Ala Gly Cys Cys Asn Asp Glu
    50                  55                  60
Ala Leu Glu Cys Val Pro Thr Glu Thr Arg Asn Val Thr Met Glu Val
65                  70                  75                  80
Leu Arg Val Lys Gln Arg Val Ser Gln His Asn Phe Gln Leu Ser Phe
                85                  90                  95
Thr Glu His Thr Lys Cys Glu Cys Arg Pro Lys Ala Glu Val Lys Ala
            100                 105                 110
Lys Lys Glu Lys Cys Glu Lys Pro Arg
        115                 120
```

<210> 9
<211> 366
<212> DNA
<213> Danio rerio

<220>
<223> modified VEGF-A K84A isoform 121

<220>
<221> unsure
<222> 252
<223> /replace="n = t, c, a or g"

<400> 9

```
gcccacatac ccaaagaagg gggaaagagc aaaaatgatg tgattccctt catggatgtg      60

tataaaaaga gtgcgtgcaa gacccgagag ctgctggtag acatcatcca ggagtatccc     120

gatgagatcg agcacacgta catcccgtcc tgtgtggttc tcatgcgctg tgcaggatgc     180

tgtaatgatg aggcgctcga atgcgtcccg acagagacac gaaacgtcac tatggaggtg     240

ctgcgggtcg cncaacgcgt atcgcagcat aattttcagc tgagtttcac agaacacacc     300

aagtgtgaat gcaggccaaa ggcagaagtc aaagcaaaga aagaaaaatg tgaaaagcca     360

agatga                                                                366
```

<210> 10
<211> 121
<212> PRT
<213> Danio rerio

<220>
<223> modified VEGF-A K84A isoform 121

<400> 10
Ala His Ile Pro Lys Glu Gly Gly Lys Ser Lys Asn Asp Val Ile Pro
1 5 10 15
Phe Met Asp Val Tyr Lys Lys Ser Ala Cys Lys Thr Arg Glu Leu Leu
20 25 30
Val Asp Ile Ile Gln Glu Tyr Pro Asp Glu Ile Glu His Thr Tyr Ile
35 40 45
Pro Ser Cys Val Val Leu Met Arg Cys Ala Gly Cys Cys Asn Asp Glu
50 55 60
Ala Leu Glu Cys Val Pro Thr Glu Thr Arg Asn Val Thr Met Glu Val
65 70 75 80
Leu Arg Val Ala Gln Arg Val Ser Gln His Asn Phe Gln Leu Ser Phe
85 90 95
Thr Glu His Thr Lys Cys Glu Cys Arg Pro Lys Ala Glu Val Lys Ala
100 105 110
Lys Lys Glu Lys Cys Glu Lys Pro Arg
115 120

<210> 11
<211> 366
<212> DNA
<213> Danio rerio

<220>
<223> modified VEGF-A F17A isoform 121

<220>
<221> unsure
<222> 51
<223> /replace="n = t, c, a or g"

<400> 11
gcccacatac ccaaagaagg gggaaagagc aaaaatgatg tgattcccgc natggatgtg 60

tataaaaaga gtgcgtgcaa gacccgagag ctgctggtag acatcatcca ggagtatccc 120

gatgagatcg agcacacgta catcccgtcc tgtgtggttc tcatgcgctg tgcaggatgc 180

tgtaatgatg aggcgctcga atgcgtcccg acagagacac gaaacgtcac tatggaggtg 240

ctgcgggtca agcaacgcgt atcgcagcat aattttcagc tgagtttcac agaacacacc 300

aagtgtgaat gcaggccaaa ggcagaagtc aaagcaaaga aagaaaaatg tgaaaagcca 360

agatga 366

<210> 12
<211> 121
<212> PRT
<213> Danio rerio

<220>
<223> modified VEGF-A F17A isoform 121

<400> 12
Ala His Ile Pro Lys Glu Gly Gly Lys Ser Lys Asn Asp Val Ile Pro
1               5                   10                  15
Ala Met Asp Val Tyr Lys Lys Ser Ala Cys Lys Thr Arg Glu Leu Leu
            20                  25                  30
Val Asp Ile Ile Gln Glu Tyr Pro Asp Glu Ile Glu His Thr Tyr Ile
        35                  40                  45
Pro Ser Cys Val Val Leu Met Arg Cys Ala Gly Cys Cys Asn Asp Glu
    50                  55                  60
Ala Leu Glu Cys Val Pro Thr Glu Thr Arg Asn Val Thr Met Glu Val
65                  70                  75                  80
Leu Arg Val Lys Gln Arg Val Ser Gln His Asn Phe Gln Leu Ser Phe
                85                  90                  95
Thr Glu His Thr Lys Cys Glu Cys Arg Pro Lys Ala Glu Val Lys Ala
            100                 105                 110
Lys Lys Glu Lys Cys Glu Lys Pro Arg
        115                 120

<210> 13
<211> 121
<212> PRT
<213> Homo sapiens

<220>
<223> modified VEGF-A K84A and F17A isoform 121

<400> 13
Ala Pro Met Ala Glu Gly Gly Gly Gln Asn His His Glu Val Val Lys
1               5                   10                  15
Ala Met Asp Val Tyr Gln Arg Ser Tyr Cys His Pro Ile Glu Thr Leu
            20                  25                  30
Val Asp Ile Phe Gln Glu Tyr Pro Asp Glu Ile Glu Tyr Ile Phe Lys
        35                  40                  45
Pro Ser Cys Val Pro Leu Met Arg Cys Gly Gly Cys Cys Asn Asp Glu
    50                  55                  60
Gly Leu Glu Cys Val Pro Thr Glu Glu Ser Asn Ile Thr Met Gln Ile
65                  70                  75                  80
Met Arg Ile Ala Pro His Gln Gly Gln His Ile Gly Glu Met Ser Phe
                85                  90                  95
Leu Gln His Asn Lys Cys Glu Cys Arg Pro Lys Lys Asp Arg Ala Arg
            100                 105                 110
Gln Glu Lys Cys Asp Lys Pro Arg Arg
        115                 120

<210> 14
<211> 121
<212> PRT
<213> Danio rerio

<220>
<223> modified VEGF-A K84A and F17A isoform 121

<400> 14
Ala His Ile Pro Lys Glu Gly Gly Lys Ser Lys Asn Asp Val Ile Pro
1               5                   10                  15
Ala Met Asp Val Tyr Lys Lys Ser Ala Cys Lys Thr Arg Glu Leu Leu
            20                  25                  30
Val Asp Ile Ile Gln Glu Tyr Pro Asp Glu Ile Glu His Thr Tyr Ile
        35                  40                  45
Pro Ser Cys Val Val Leu Met Arg Cys Ala Gly Cys Cys Asn Asp Glu
    50                  55                  60
Ala Leu Glu Cys Val Pro Thr Glu Thr Arg Asn Val Thr Met Glu Val
65                  70                  75                  80

```
Leu Arg Val Ala Gln Arg Val Ser Gln His Asn Phe Gln Leu Ser Phe
                85                  90                  95
Thr Glu His Thr Lys Cys Glu Cys Arg Pro Lys Ala Glu Val Lys Ala
            100                 105                 110
Lys Lys Glu Lys Cys Glu Lys Pro Arg
        115                 120
```

<210> 15
<211> 444
<212> DNA
<213> Homo sapiens

<220>
<223> VEGF-A isoform 121 with signal peptide

<400> 15

```
atgaactttc tgctgtcttg ggtgcattgg agccttgcct tgctgctcta cctccaccat      60

gccaagtggt cccaggctgc acccatggca gaaggaggag ggcagaatca tcacgaagtg     120

gtgaagttca tggatgtcta tcagcgcagc tactgccatc caatcgagac cctggtggac     180

atcttccagg agtaccctga tgagatcgag tacatcttca agccatcctg tgtgcccctg     240

atgcgatgcg ggggctgctg caatgacgag ggcctggagt gtgtgcccac tgaggagtcc     300

aacatcacca tgcagattat gcggatcaaa cctcaccaag gccagcacat aggagagatg     360

agcttcctac agcacaacaa atgtgaatgc agaccaaaga aagatagagc aagacaagaa     420

aaatgtgaca agccgaggcg gtga                                            444
```

<210> 16
<211> 147
<212> PRT
<213> Homo sapiens

<220>
<223> VEGF-A isoform 121 with the signal peptide

<400> 16

```
Met Asn Phe Leu Leu Ser Trp Val His Trp Ser Leu Ala Leu Leu Leu
1               5                   10                  15
Tyr Leu His His Ala Lys Trp Ser Gln Ala Ala Pro Met Ala Glu Gly
            20                  25                  30
Gly Gly Gln Asn His His Glu Val Val Lys Phe Met Asp Val Tyr Gln
        35                  40                  45
Arg Ser Tyr Cys His Pro Ile Glu Thr Leu Val Asp Ile Phe Gln Glu
    50                  55                  60
Tyr Pro Asp Glu Ile Glu Tyr Ile Phe Lys Pro Ser Cys Val Pro Leu
65                  70                  75                  80
Met Arg Cys Gly Gly Cys Cys Asn Asp Glu Gly Leu Glu Cys Val Pro
                85                  90                  95
Thr Glu Glu Ser Asn Ile Thr Met Gln Ile Met Arg Ile Lys Pro His
            100                 105                 110
Gln Gly Gln His Ile Gly Glu Met Ser Phe Leu Gln His Asn Lys Cys
        115                 120                 125
Glu Cys Arg Pro Lys Lys Asp Arg Ala Arg Gln Glu Lys Cys Asp Lys
    130                 135                 140
Pro Arg Arg
145
```

<210> 17
<211> 444
<212> DNA
<213> Homo sapiens

<220>
<223> modified VEGF-A isoform 121 with the signal peptide

<400> 17
atgaactttc tgctgtcttg ggtgcattgg agccttgcct tgctgctcta cctccaccat 60

gccaagtggt cccaggctgc acccatggca gaaggaggag ggcagaatca tcacgaagtg 120

gtgaagttca tggatgtcta tcagcgcagc tactgccatc caatcgagac cctggtggac 180

atcttccagg agtaccctga tgagatcgag tacatcttca agccatcctg tgtgcccctg 240

atgcgatgcg gggctgctg caatgacgag ggcctggagt gtgtgcccac tgaggagtcc 300

aacatcacca tgcagattat gcggatcgca cctcaccaag gccagcacat aggagagatg 360

agcttcctac agcacaacaa atgtgaatgc agaccaaaga aagatagagc aagacaagaa 420

aaatgtgaca agccgaggcg gtga 444

<210> 18
<211> 147
<212> PRT
<213> Homo sapiens

<220>
<223> modified VEGF-A isoform 121 with the signal peptide

<400> 18
Met Asn Phe Leu Leu Ser Trp Val His Trp Ser Leu Ala Leu Leu Leu
1               5                   10                  15
Tyr Leu His His Ala Lys Trp Ser Gln Ala Ala Pro Met Ala Glu Gly
            20                  25                  30
Gly Gly Gln Asn His His Glu Val Val Lys Phe Met Asp Val Tyr Gln
        35                  40                  45
Arg Ser Tyr Cys His Pro Ile Glu Thr Leu Val Asp Ile Phe Gln Glu
    50                  55                  60
Tyr Pro Asp Glu Ile Glu Tyr Ile Phe Lys Pro Ser Cys Val Pro Leu
65                  70                  75                  80
Met Arg Cys Gly Gly Cys Cys Asn Asp Glu Gly Leu Glu Cys Val Pro
                85                  90                  95
Thr Glu Glu Ser Asn Ile Thr Met Gln Ile Met Arg Ile Ala Pro His
            100                 105                 110
Gln Gly Gln His Ile Gly Glu Met Ser Phe Leu Gln His Asn Lys Cys
        115                 120                 125
Glu Cys Arg Pro Lys Lys Asp Arg Ala Arg Gln Glu Lys Cys Asp Lys
    130                 135                 140
Pro Arg Arg
145

<210> 19
<211> 444
<212> DNA
<213> Homo sapiens

<220>
<223> modified VEGF-A isoform 121 with the signal peptide

<400> 19
atgaactttc tgctgtcttg ggtgcattgg agccttgcct tgctgctcta cctccaccat 60

gccaagtggt cccaggctgc acccatggca gaaggaggag ggcagaatca tcacgaagtg 120

gtgaaggcca tggatgtcta tcagcgcagc tactgccatc caatcgagac cctggtggac 180

atcttccagg agtaccctga tgagatcgag tacatcttca agccatcctg tgtgcccctg 240

atgcgatgcg gggctgctg caatgacgag ggcctggagt gtgtgcccac tgaggagtcc 300

aacatcacca tgcagattat gcggatcaaa cctcaccaag gccagcacat aggagagatg 360

agcttcctac agcacaacaa atgtgaatgc agaccaaaga aagatagagc aagacaagaa 420

aaatgtgaca agccgaggcg gtga 444

<210> 20
<211> 147
<212> PRT
<213> Homo sapiens

<220>
<223> modified VEGF-A isoform 121 with the signal peptide

<400> 20
Met Asn Phe Leu Leu Ser Trp Val His Trp Ser Leu Ala Leu Leu Leu
1 5 10 15
Tyr Leu His His Ala Lys Trp Ser Gln Ala Ala Pro Met Ala Glu Gly
20 25 30
Gly Gly Gln Asn His His Glu Val Val Lys Ala Met Asp Val Tyr Gln
35 40 45
Arg Ser Tyr Cys His Pro Ile Glu Thr Leu Val Asp Ile Phe Gln Glu
50 55 60
Tyr Pro Asp Glu Ile Glu Tyr Ile Phe Lys Pro Ser Cys Val Pro Leu
65 70 75 80
Met Arg Cys Gly Gly Cys Cys Asn Asp Glu Gly Leu Glu Cys Val Pro
85 90 95
Thr Glu Glu Ser Asn Ile Thr Met Gln Ile Met Arg Ile Lys Pro His
100 105 110
Gln Gly Gln His Ile Gly Glu Met Ser Phe Leu Gln His Asn Lys Cys
115 120 125
Glu Cys Arg Pro Lys Lys Asp Arg Ala Arg Gln Glu Lys Cys Asp Lys
130 135 140
Pro Arg Arg
145

<210> 21
<211> 435
<212> DNA
<213> Danio rerio

<220>
<223> VEGF-A isoform 121 with the signal peptide

<400> 21
atgaacttgg ttgtttattt gatacagtta tttctcgcgg ctctcctcca tctgtctgct 60

gtaaaggctg cccacatacc caaagaaggg ggaaagagca aaaatgatgt gattcccttc 120

atggatgtgt ataaaaagag tgcgtgcaag acccgagagc tgctggtaga catcatccag 180

gagtatcccg atgagatcga gcacacgtac atcccgtcct gtgtggttct catgcgctgt 240

gcaggatgct gtaatgatga ggcgctcgaa tgcgtcccga cagagacacg aaacgtcact 300

atggaggtgc tgcgggtcaa gcaacgcgta tcgcagcata attttcagct gagtttcaca 360

gaacacacca agtgtgaatg caggccaaag gcagaagtca aagcaaagaa agaaaaatgt 420

gaaaagccaa gatga 435

<210> 22
<211> 144
<212> PRT
<213> Danio rerio

<220>
<223> VEGF-A isoform 121 with the signal peptide

<400> 22
Met Asn Leu Val Val Tyr Leu Ile Gln Leu Phe Leu Ala Ala Leu Leu
1 5 10 15
His Leu Ser Ala Val Lys Ala Ala His Ile Pro Lys Glu Gly Gly Lys
20 25 30
Ser Lys Asn Asp Val Ile Pro Phe Met Asp Val Tyr Lys Lys Ser Ala
35 40 45
Cys Lys Thr Arg Glu Leu Leu Val Asp Ile Ile Gln Glu Tyr Pro Asp
50 55 60
Glu Ile Glu His Thr Tyr Ile Pro Ser Cys Val Val Leu Met Arg Cys
65 70 75 80
Ala Gly Cys Cys Asn Asp Glu Ala Leu Glu Cys Val Pro Thr Glu Thr
85 90 95
Arg Asn Val Thr Met Glu Val Leu Arg Val Lys Gln Arg Val Ser Gln
100 105 110
His Asn Phe Gln Leu Ser Phe Thr Glu His Thr Lys Cys Glu Cys Arg
115 120 125
Pro Lys Ala Glu Val Lys Ala Lys Lys Glu Arg Cys Glu Lys Pro Arg
130 135 140

<210> 23
<211> 435
<212> DNA
<213> Danio rerio

<220>
<223> modified VEGF-A isoform 121 with the signal peptide

<400> 23
atgaacttgg ttgtttattt gatacagtta tttctcgcgg ctctcctcca tctgtctgct 60

gtaaaggctg cccacatacc caaagaaggg ggaaagagca aaaatgatgt gattcccttc 120

atggatgtgt ataaaaagag tgcgtgcaag acccgagagc tgctggtaga catcatccag 180

gagtatcccg atgagatcga gcacacgtac atcccgtcct gtgtggttct catgcgctgt 240

gcaggatgct gtaatgatga ggcgctcgaa tgcgtcccga cagagacacg aaacgtcact 300

atggaggtgc tgcgggtcgc gcaacgcgta tcgcagcata attttcagct gagtttcaca 360

gaacacacca agtgtgaatg caggccaaag gcagaagtca aagcaaagaa agaaaaatgt 420

gaaaagccaa gatga 435

<210> 24
<211> 144
<212> PRT
<213> Danio rerio

<220>
<223> modfied VEGF-A isoform 121 with the signal peptide

<400> 24
Met Asn Leu Val Val Tyr Leu Ile Gln Leu Phe Leu Ala Ala Leu Leu
1 5 10 15
His Leu Ser Ala Val Lys Ala Ala His Ile Pro Lys Glu Gly Gly Lys
20 25 30
Ser Lys Asn Asp Val Ile Pro Phe Met Asp Val Tyr Lys Lys Ser Ala
35 40 45
Cys Lys Thr Arg Glu Leu Leu Val Asp Ile Ile Gln Glu Tyr Pro Asp
50 55 60
Glu Ile Glu His Thr Tyr Ile Pro Ser Cys Val Val Leu Met Arg Cys
65 70 75 80
Ala Gly Cys Cys Asn Asp Glu Ala Leu Glu Cys Val Pro Thr Glu Thr
85 90 95
Arg Asn Val Thr Met Glu Val Leu Arg Val Ala Gln Arg Val Ser Gln
100 105 110
His Asn Phe Gln Leu Ser Phe Thr Glu His Thr Lys Cys Glu Cys Arg
115 120 125
Pro Lys Ala Glu Val Lys Ala Lys Lys Glu Arg Cys Glu Lys Pro Arg
130 135 140

<210> 25
<211> 435
<212> DNA
<213> Danio rerio

<220>
<223> modified VEGF-A isoform 121 with the signal peptide

<400> 25
atgaacttgg ttgtttattt gatacagtta tttctcgcgg ctctcctcca tctgtctgct 60

gtaaaggctg cccacatacc caaagaaggg ggaaagagca aaaatgatgt gattcccgcc 120

atggatgtgt ataaaaagag tgcgtgcaag acccgagagc tgctggtaga catcatccag 180

gagtatcccg atgagatcga gcacacgtac atcccgtcct gtgtggttct catgcgctgt 240

gcaggatgct gtaatgatga ggcgctcgaa tgcgtcccga cagagacacg aaacgtcact 300

atggaggtgc tgcgggtcaa gcaacgcgta tcgcagcata attttcagct gagtttcaca 360

gaacacacca agtgtgaatg caggccaaag gcagaagtca aagcaaagaa agaaaaatgt 420

gaaaagccaa gatga 435

<210> 26
<211> 144

<212> PRT
<213> Danio rerio

<220>
<223> modified VEGF-A isoform 121 with the signal peptide

<400> 26
```
Met Asn Leu Val Val Tyr Leu Ile Gln Leu Phe Leu Ala Ala Leu Leu
1               5                   10                  15
His Leu Ser Ala Val Lys Ala Ala His Ile Pro Lys Glu Gly Gly Lys
            20                  25                  30
Ser Lys Asn Asp Val Ile Pro Ala Met Asp Val Tyr Lys Lys Ser Ala
        35                  40                  45
Cys Lys Thr Arg Glu Leu Leu Val Asp Ile Ile Gln Glu Tyr Pro Asp
    50                  55                  60
Glu Ile Glu His Thr Tyr Ile Pro Ser Cys Val Val Leu Met Arg Cys
65                  70                  75                  80
Ala Gly Cys Cys Asn Asp Glu Ala Leu Glu Cys Val Pro Thr Glu Thr
                85                  90                  95
Arg Asn Val Thr Met Glu Val Leu Arg Val Lys Gln Arg Val Ser Gln
            100                 105                 110
His Asn Phe Gln Leu Ser Phe Thr Glu His Thr Lys Cys Glu Cys Arg
        115                 120                 125
Pro Lys Ala Glu Val Lys Ala Lys Lys Glu Arg Cys Glu Lys Pro Arg
    130                 135                 140
```

<210> 27
<211> 44
<212> PRT
<213> Homo sapiens

<220>
<223> Heparin binding domain/Nrp-1 binding domain

<400> 27
```
Asn Pro Cys Gly Pro Cys Ser Glu Arg Arg Lys His Leu Phe Val Gln
1               5                   10                  15
Asp Pro Gln Thr Cys Lys Cys Ser Cys Lys Asn Thr Asp Ser Arg Cys
            20                  25                  30
Lys Ala Arg Gln Leu Glu Leu Asn Glu Arg Thr Cys
        35                  40
```

<210> 28
<211> 44
<212> PRT
<213> Danio rerio

<220>
<223> Heparin binding domain/Nrp-1 binding domain

<400> 28
```
Asn His Cys Glu Pro Cys Ser Glu Arg Arg Lys Arg Leu Tyr Val Gln
1               5                   10                  15
Asp Pro Leu Thr Cys Lys Cys Ser Cys Lys Phe Thr Gln Met Gln Cys
            20                  25                  30
Lys Ser Arg Gln Leu Glu Leu Asn Glu Arg Thr Cys
        35                  40
```

<210> 29
<211> 191
<212> PRT

<213> Homo sapiens

<220>
<223> VEGF-A isoform 165

<400> 29
Met Asn Phe Leu Leu Ser Trp Val His Trp Ser Leu Ala Leu Leu Leu
1 5 10 15
Tyr Leu His His Ala Lys Trp Ser Gln Ala Ala Pro Met Ala Glu Gly
20 25 30
Gly Gly Gln Asn His His Glu Val Val Lys Phe Met Asp Val Tyr Gln
35 40 45
Arg Ser Tyr Cys His Pro Ile Glu Thr Leu Val Asp Ile Phe Gln Glu
50 55 60
Tyr Pro Asp Glu Ile Glu Tyr Ile Phe Lys Pro Ser Cys Val Pro Leu
65 70 75 80
Met Arg Cys Gly Gly Cys Cys Asn Asp Glu Gly Leu Glu Cys Val Pro
85 90 95
Thr Glu Glu Ser Asn Ile Thr Met Gln Ile Met Arg Ile Lys Pro His
100 105 110
Gln Gly Gln His Ile Gly Glu Met Ser Phe Leu Gln His Asn Lys Cys
115 120 125
Glu Cys Arg Pro Lys Lys Asp Arg Ala Arg Gln Glu Asn Pro Cys Gly
130 135 140
Pro Cys Ser Glu Arg Arg Lys His Leu Phe Val Gln Asp Pro Gln Thr
145 150 155 160
Cys Lys Cys Ser Cys Lys Asn Thr Asp Ser Arg Cys Lys Ala Arg Gln
165 170 175
Leu Glu Leu Asn Glu Arg Thr Cys Arg Cys Asp Lys Pro Arg Arg
180 185 190

<210> 30
<211> 188
<212> PRT
<213> Danio rerio

<220>
<223> VEGF-A isoform 165

<400> 30
Met Asn Leu Val Val Tyr Leu Ile Gln Leu Phe Leu Ala Ala Leu Leu
1 5 10 15
His Leu Ser Ala Val Lys Ala Ala His Ile Pro Lys Glu Gly Gly Lys
20 25 30
Ser Lys Asn Asp Val Ile Pro Phe Met Asp Val Tyr Lys Lys Ser Ala
35 40 45
Cys Lys Thr Arg Glu Leu Leu Val Asp Ile Ile Gln Glu Tyr Pro Asp
50 55 60
Glu Ile Glu His Thr Tyr Ile Pro Ser Cys Val Val Leu Met Arg Cys
65 70 75 80
Ala Gly Cys Cys Asn Asp Glu Ala Leu Glu Cys Val Pro Thr Glu Thr
85 90 95
Arg Asn Val Thr Met Glu Val Leu Arg Val Lys Gln Arg Val Ser Gln
100 105 110
His Asn Phe Gln Leu Ser Phe Thr Glu His Thr Lys Cys Glu Cys Arg
115 120 125
Pro Lys Ala Glu Val Lys Ala Lys Lys Glu Asn His Cys Glu Pro Cys
130 135 140
Ser Glu Arg Arg Lys Arg Leu Tyr Val Gln Asp Pro Leu Thr Cys Lys
145 150 155 160
Cys Ser Cys Lys Phe Thr Gln Met Gln Cys Lys Ser Arg Gln Leu Glu
165 170 175
Leu Asn Glu Arg Thr Cys Arg Cys Glu Lys Pro Arg

180 185

<210> 31
<211> 137
<212> PRT
<213> Homo sapiens

<220>
<223> VEGF-A isoform 111

<400> 31
Met Asn Phe Leu Leu Ser Trp Val His Trp Ser Leu Ala Leu Leu Leu
1 5 10 15
Tyr Leu His His Ala Lys Trp Ser Gln Ala Ala Pro Met Ala Glu Gly
20 25 30
Gly Gly Gln Asn His His Glu Val Val Lys Phe Met Asp Val Tyr Gln
35 40 45
Arg Ser Tyr Cys His Pro Ile Glu Thr Leu Val Asp Ile Phe Gln Glu
50 55 60
Tyr Pro Asp Glu Ile Glu Tyr Ile Phe Lys Pro Ser Cys Val Pro Leu
65 70 75 80
Met Arg Cys Gly Gly Cys Cys Asn Asp Glu Gly Leu Glu Cys Val Pro
85 90 95
Thr Glu Glu Ser Asn Ile Thr Met Gln Ile Met Arg Ile Lys Pro His
100 105 110
Gln Gly Gln His Ile Gly Glu Met Ser Phe Leu Gln His Asn Lys Cys
115 120 125
Glu Cys Arg Cys Asp Lys Pro Arg Arg
130 135

<210> 32
<211> 425
<212> DNA
<213> Danio rerio

<220>
<223> mutated endogenous vascular growth factor A

<400> 32
atgaacttgg ttgtttattt gatacagtta tttctcgcgg ctctcctcca tctgtctgct 60

gtaaaggctg cccacatacc caaagaaggg ggaaagagca aaaatgatgt gattcccttc 120

atggatgtgt ataaaaagag tgcgtgcaag acccgagagc tgctggtaga catcatccag 180

atgagatcga gcacacgtac atcccgtcct gtgtggttct catgcgctgt gcaggatgct 240

gtaatgatga ggcgctcgaa tgcgtcccga cagagacacg aaacgtcact atggaggtgc 300

tgcgggtcaa gcaacgcgta tcgcagcata attttcagct gagtttcaca gaacacacca 360

agtgtgaatg caggccaaag gcagaagtca aagcaaagaa agaaaaatgt gaaaagccaa 420

gatga 425

<210> 33
<211> 113
<212> PRT
<213> Danio rerio

<220>
<223> mutated endogenous vascular growth factor A

<400> 33
Met Asn Leu Val Val Tyr Leu Ile Gln Leu Phe Leu Ala Ala Leu Leu
1 5 10 15
His Leu Ser Ala Val Lys Ala Ala His Ile Pro Lys Glu Gly Gly Lys
20 25 30
Ser Lys Asn Asp Val Ile Pro Phe Met Asp Val Tyr Lys Lys Ser Ala
35 40 45
Cys Lys Thr Arg Glu Leu Leu Val Asp Ile Ile Gln Met Arg Ser Ser
50 55 60
Thr Arg Thr Ser Arg Pro Val Trp Phe Ser Cys Ala Val Gln Asp Ala
65 70 75 80
Val Met Met Arg Arg Ser Asn Ala Ser Arg Gln Arg His Glu Thr Ser
85 90 95
Leu Trp Arg Cys Cys Gly Ser Ser Asn Ala Tyr Arg Ser Ile Ile Phe
100 105 110
Ser

<210> 34
<211> 7
<212> PRT
<213> Homo sapiens

<220>
<223> stretch of VEGF-A

<400> 34
Met Arg Ile Lys Pro His Gln
1 5

<210> 35
<211> 7
<212> PRT
<213> Mus musculus

<220>
<223> stretch of VEGF-A

<400> 35
Met Arg Ile Lys Pro His Gln
1 5

<210> 36
<211> 7
<212> PRT
<213> Danio rerio

<220>
<223> stretch of VEGF-A

<400> 36
Leu Arg Val Lys Gln Arg Val
1 5

<210> 37
<211> 7
<212> PRT
<213> Xenopus <genus>

<220>
<223> stretch of VEGF-A

<400> 37
Met Lys Ile Lys Pro His Ile
1 5

<210> 38
<211> 7
<212> PRT
<213> Homo sapiens

<220>
<223> stretch of VEGF-A

<400> 38
Val Val Lys Phe Met Asp Val
1 5

<210> 39
<211> 7
<212> PRT
<213> Mus musculus

<220>
<223> stretch of VEGF-A

<400> 39
Val Ile Lys Phe Met Asp Val
1 5

<210> 40
<211> 7
<212> PRT
<213> Danio rerio

<220>
<223> stretch of VEGF-A

<400> 40
Val Ile Pro Phe Met Asp Val
1 5

<210> 41
<211> 7
<212> PRT
<213> Xenopus <genus>

<220>
<223> stretch of VEGF-A

<400> 41
Val Val Lys Phe Leu Lys Val
1 5

<210> 42
<211> 29
<212> DNA
<213> Artificial Sequence

<220>
<223> vegfaa fw xpcDNA

<400> 42
ccaagcttat gaacttggtt gtttatttg 29

<210> 43
<211> 27
<212> DNA
<213> Artificial Sequence

<220>
<223> vegfaa rv xpcDNA

<400> 43
ccgaattctt atcttggctt ttcacat 27

<210> 44
<211> 33
<212> DNA
<213> Artificial Sequence

<220>
<223> F17A vegfaa fw

<400> 44
aatgatgtga ttcccgcgat ggatgtgtat aaa 33

<210> 45
<211> 33
<212> DNA
<213> Artificial Sequence

<220>
<223> F17A vegfaa rv

<400> 45
tttatacaca tccatcgcgg gaatcacatc att 33

<210> 46
<211> 29
<212> DNA
<213> Artificial Sequence

<220>
<223> K84A zvegfaa fw

<400> 46
ggtgctgcgg gtcgcgcaac gcgtatcgc 29

<210> 47
<211> 29
<212> DNA
<213> Artificial Sequence

<220>
<223> K84A zvegfaa rv

<400> 47
gcgatacgcg ttgcgcgacc cgcagcacc 29

<210> 48
<211> 26

<212> DNA
<213> Artificial Sequence

<220>
<223> R165E vegfaa-165 fw

<400> 48
ccaagcttat gaacttggtt gtttat 26

<210> 49
<211> 26
<212> DNA
<213> Artificial Sequence

<220>
<223> R165E vegfaa-165rv

<400> 49
ccgaattctt attctggctt ttcaca 26

<210> 50
<211> 29
<212> DNA
<213> Artificial Sequence

<220>
<223> zPgf fw

<400> 50
ccaagcttat gagttatttg acgtcactg 29

<210> 51
<211> 29
<212> DNA
<213> Artificial Sequence

<220>
<223> zPgf rv

<400> 51
ccgaatttta cctgcgggga ggctgacac 29

<210> 52
<211> 29
<212> DNA
<213> Artificial Sequence

<220>
<223> Vegfbb fw

<400> 52
ccaagcttat gcggggtttt cttctgttt 29

<210> 53
<211> 28
<212> DNA
<213> Artificial Sequence

```
<220>
<223> Vegfbb rv

<400> 53
ccgaatttta gtctctgaac ctgcactg                                   28
```

## Description

The present invention relates to a modified vascular endothelial growth factor A (VEGF-A) polypeptide, particularly to a modified VEGF-A 121 isoform for use in treating an angiogenic disorder. The modified vascular endothelial growth factor A (VEGF-A) comprises a hydrophobic amino acid in place of the lysine at the position corresponding to position 84 of the wild-type VEGF-A as shown in SEQ ID NO: 2, and/or the modified vascular endothelial growth factor A (VEGF-A) comprises a hydrophobic amino acid in place of the phenylalanine at the position corresponding to position 17 of the wild-type VEGF-A as shown in SEQ ID NO: 2.

The present invention relates to non-naturally occurring vascular endothelial growth factor A (VEGF-A) molecules. Particularly, the invention relates to modified VEGF-A molecules that exhibit improved properties and pharmacologic effects, e.g., in the treatment of angiogenic disorders. Further, the present invention relates to nucleic acid molecules encoding such polypeptides, and vectors and hosts comprising such nucleic acids. The invention further relates to methods for producing the polypeptides of the invention, and to methods of using them in the treatment of disease, in particular, in the medical intervention of angiogenic disorders, such as neovascular pathologies. Furthermore, the invention relates to a non-human transgenic animal having a non-functional VEGF-A and a screening method using this animal for identifying modified VEGF-A molecules.

Vascular endothelial growth factor A (VEGF-A) is a master regulator of physiological and pathological angiogenesis and vasculogenesis. Accordingly, VEGF-A regulates the body's blood supply. Sufficient blood supply is crucial for tumor development. Moreover, over-expression of VEGF-A causes vascular diseases, e.g., in the retina of the eye. Therefore, the medical potential of inhibiting VEGF-A is enormous.

VEGF-A belongs to a gene family that includes placental growth factor (PLGF), VEGF-B, VEGF-C, and VEGF-D (Ferrara et al., 2003). There are multiple isoforms of VEGF-A that are a result of alternative splicing. VEGF-A pre-mRNA can for example be spliced between exons 6 and 8. Exon 7 particularly confer heparin-binding affinity (Chengzhong et al. March 2010, Anticancer research; Differential Expression of VEGF-A 121, VEGF-A 165 and VEGF-A 189 in Angiomas and Squamous Cell Carcinoma Cell Lines of the Head and Neck). VEGF-A 206, VEGF-A 189, VEGF-A 165, and VEGF-A 121 are the main isoforms of the human VEGF-A family (Arcondeguy et al., 2013). In most species, VEGF-A 121 and VEGF-A 165 are the predominant isoforms (Arcondeguy et al., 2013). The different isoforms share functions; however, they differ in their binding affinity for extracellular matrix (ECM) molecules (loc. cit. Chengzhong, 2010). For instance, the longer isoform, VEGF-A165 has a high affinity for heparin and is thus tightly associated with the ECM. By contrast, VEGF-A 121 is freely diffusible (loc. cit. Chengzhong, 2010).

Various VEGF-A molecules bind to various receptors, e.g., VEGFR-1, VEGFR-2, VEGFR-3 and Neuropilin-1 (Nrp-1) (Olsson, 2006). It is believed that the major binding site for VEGF-A is VEGFR-2. Binding of VEGF-A to VEGFR-2 promotes the dimerization of the receptor. The dimerization is additionally stabilized by low-affinity homo-typic interactions between membrane-proximal Ig-like domains. The receptor becomes activated by trans/autophosphorylation of intracellular tyrosine residues (Ruch et al., 2007; Yang et al., 2010).

Given the crucial nature of this pathway in tumorigenes, signaling activation of VEGF-A has been the focus of investigation in the last decade. The modalities under investigation or approved for use in therapy include e.g., monoclonal antibodies against VEGF-A (bevacizumab), VEGF-A decoy receptors (VEGF Trap, aflibercept), antibodies against VEGFR-2 (e.g., anti-VEGFR-2 antibody R84), and small-molecule tyrosine kinase inhibitors (TKIs) targeting VEGFR family members (sunitinib, sorafenib, pazopanib, cediranib, axitinib, etc.).

Anti-VEGF-A therapies are important in the treatment of several cancers or neo-vascular pathologies such as age-related macular degeneration (AMD). The currently accepted anti-VEGF-A agents include bevacizumab (Avastin; Genentech, San Francisco, CA, USA), ranibizumab (Lucentis; Novartis, Basel, Switzerland; and Genentech Inc., South San Francisco, CA), pegaptanib sodium (Macugen; Eyetech Pharmaceuticals/Pfizer, NY), and aflibercept (VEGF Trap-Eye, Eylea, Regeneron Pharmaceuticals, Inc., Tarrytown, NY, USA and Bayer, Basel, Switzerland).

Although inhibitors of signaling activation of VEGF-A are successfully used in the clinic, not all patients respond and some patients fail to fully respond to angiogenesis inhibitor therapy. Accordingly, there is a need for improved angiogenesis inhibitor and/or vasculogenesis inhibitor therapy.

The technical problem underlying the present invention is thus the provision of means and methods for the therapy of angiogenic diseases.

The technical problem is solved by provision of the embodiments provided herein below and as characterized in the appended claims.

The present invention relates to a modified vascular endothelial growth factor A polypeptide for use in treating an angiogenic disorder, wherein the modified vascular endothelial growth factor A (VEGF-A) is the VEGF-A 121 isoform, wherein said modified vascular endothelial growth factor A (VEGF-A) comprises a hydrophobic amino acid in place of the lysine at the position corresponding to position 84 of the wild-type VEGF-A as shown in SEQ ID NO: 2, and/or wherein said modified vascular endothelial growth factor A (VEGF-A) comprises a hydrophobic amino acid in place of the phenylalanine at the position corresponding to position 17 of the wild-type VEGF-A as shown in SEQ ID NO: 2.

In other words, the VEGF-A 121 isoform (known in the prior art and as shown in SEQ ID NO: 2) has been inventively engineered herein.

In a certain aspect, the present invention relates to a modified vascular endothelial growth factor A (VEGF-A) polypeptide for use in treating an angiogenic disorder, wherein said VEGF-A comprises a hydrophobic amino acid in place of the lysine at the position corresponding to position 84 of the wild-type VEGF-A as shown in SEQ ID NO: 2, and/or wherein said modified VEGF comprises a hydrophobic amino acid in place of the phenylalanine at the position corresponding to position 17 of the wild-type VEGF-A as shown in SEQ ID NO: 2, and wherein said VEGF-A lacks the heparin binding domain and/or the Nrp-1 binding domain.

In a certain aspect, the present invention relates to a method of treatment for an angiogenic disorder comprising the step of administering to a subject in need of such treatment a pharmaceutical effective amount of a modified vascular endothelial growth factor A (VEGF-A) polypeptide, wherein the modified vascular endothelial growth factor A (VEGF-A) is the VEGF-A 121 isoform, wherein said modified VEGF-A comprises a hydrophobic amino acid in place of the lysine at the position corresponding to position 84 of the wild-type VEGF-A as shown in SEQ ID NO: 2 , and/or wherein said modified VEGF comprises a hydrophobic amino acid in place of the phenylalanine at the position corresponding to position 17 of the wild-type VEGF-A as shown in SEQ ID NO: 2.

In a certain aspect, the present invention relates to a method of treatment for an angiogenic disorder comprising the step of administering to a subject in need of such treatment a pharmaceutical effective amount of a modified vascular endothelial growth factor A (VEGF-A) polypeptide, wherein said modified VEGF-A comprises a hydrophobic amino acid in place of the lysine at the position corresponding to position 84 of the wild-type VEGF-A as shown in SEQ ID NO: 2 , and/or wherein said modified VEGF comprises a hydrophobic amino acid in place of the phenylalanine at the position corresponding to position 17 of the wild-type VEGF-A as shown in SEQ ID NO: 2, and wherein said modified VEGF-A lacks the heparin binding domain and/or the Nrp-1 binding domain.

Angiogenic diseases or disorders are, for example, cancer or neovascular pathologies like age related macular degeneration (AMD) as defined herein below in detail.

In the appended examples, it was unexpectedly demonstrated that the modified VEGF-A molecules provided herein are strong angiogenesis and/or vasculogenesis inhibitors. The illustrative examples prove the strong efficacy of the modified VEGF-A molecules in an animal model (non-human transgenic animal). It is described in the following that the non-human transgenic animal to be used in accordance with the present invention overcomes the limitations of in vitro experiments and simultaneously allows the advantageous screening of effective modified VEGF-A molecules. In vitro experiments may fail to reflect the condition of a living organism. For example, a cell line might be used for in vitro experiments that does not reflect the true condition of patients having aberrant angiogenesis. Furthermore, VEGF-A mediates angiogenesis in vivo by binding to the VEGFRs (VEGF receptors), which are situated on the outer cell surface, and interact with the ECM (extracellular matrix). Such an environment is difficult to mimic in a cell culture, e.g. because the extracellular matrix is not present. Thus, conventional in vitro assays might not be suitable to identify modified VEGF-A molecules that are of therapeutic utility in an in vivo setting.

Therefore, it is important to demonstrate angiogenesis/vasculogenesis inhibition in an in vivo model, in particular in case of angiogenesis inhibitor therapy in which some patients fail to fully respond even to approved drugs. The animal models proposed in the prior art and their generation, respectively, is, however, disadvantageous. For example, the generation of animals comprising non-functional VEGF, e.g. having an introduced mutated version of VEGF-A, is cumbersome. For example, generating dominant negatives transgenic mice would require complicated and time consuming genome editing techniques, whereas the successful generation cannot be guaranteed. Therefore, screening of mutations in prior art animal models is time, cost and labor intensive.

The transgenic non-human animal provided herein, which is in preferred aspects of the invention a zebrafish, overcomes these obstacles. It was surprisingly demonstrated in the appended examples that a viable zebrafish could be obtained comprising an artificially introduced mutation in the endogenous VEGF-A (*vegfaa* gene in zebrafish) that results in a non-functional gene polypeptide upon expression; see illustrative Figure 1. Such a non-human transgenic animal is herein employed to identify and screen new modified VEGF-A molecules. Importantly, the therapeutic effectiveness of the modified VEGF-A molecules can, in contrast to other animal models, simply be tested by injecting mRNA to the non-human transgenic animal provided herein. Therefore, the effect of the modified VEGF-A molecules can be conveniently and reliably tested in an in vivo setting. For instance, in case the non-human transgenic animal comprises a non-functional endogenous VEGF-A, the non-human transgenic animal can be employed to test whether the modified VEGF-A polypeptide or encoding nucleic acid thereof rescues such a non-human transgenic animal comprising the additionally mutated endogenous VEGF-A.

Furthermore, a non-human animal that comprises an endogenous wild-type VEGF-A and a modified VEGF-A polypeptide can be employed to test whether the modified VEGF-A polypeptide comprised in the animal inhibits and overcomes the naturally occurring VEGF-A polypeptide. In other words, it allows to analyze whether the modified VEGF-A polypeptide has a dominant negative effect. Consequently, the non-human transgenic animals or non-human animal comprising an endogenous wild-type VEGF-A and a modified VEGF-A polypeptide provided herein are versatile tools by which the effectiveness of modified VEGF-A molecules can be conveniently tested in various environments (aberrant or healthy endogenous VEGF-A).

Furthermore, zebrafish embryos are transparent. The transparency of the embryos allows the direct visualization of cells and organs. Consistently, the effect of the modified VEGF-A polypeptides on endothelial cells or other cell types, e.g. venous, artery or lymphatic, can be directly documented. The transparency of the fish embryos also allows the analysis on a single cell level in vivo.

Consequently, the development of the animal tools provided herein allows the fast and reliable screening of effective modified VEGF-A molecules in an in vivo setting as described in the illustrative appended Examples. The utilization of such animals in accordance with the invention demonstrated that the modified VEGF-A molecules provided herein are unexpectedly strong angiogenesis and/or vasculogenesis inhibitors, which thus have high medical potential..

As mentioned above, the appended examples prove that the herein provided non-naturally occurring, modified VEGF-A molecules, i.e., polypeptides or fragments thereof or nucleic acids encoding such polypeptides, have high medical potential. As is documented in the *appended in vivo* examples, the herein provided molecules show a surprisingly strong inhibition of angiogenesis and vasculogenesis that is beneficial in diseases or disorders associated with pathological angiogenesis. Naturally-occurring VEGF-A is a bona fide stimulator of angiogenesis and/or vasculogenesis. Therefore, it was surprising to find in the non-human transgenic animal that already single mutations/modifications in VEGF-A render the mutated/modified VEGF-A to an effective inhibitor of angiogenesis and/or vasculogenesis. This surprising effect is exemplified herein by certain VEGF-A molecules comprising an artificial replacement of amino acids, e.g. at the position corresponding to 84 (K) or 17 (F), by a hydrophobic amino acid such as alanine. Yet, not all VEGF-A molecules carrying these mutations are effective inhibitors of angiogenesis and/or vasculogenesis. For example, VEGF-A variants, like the VEGF-A 165 variant carrying these mutations do not show an anti-angiogenic effect; see Example 1 and Table 2. Therefore, the anti-angiogenic effect is specific to VEGF-A-isoforms lacking the heparin and/or the Nrp1-binding domain.

The herein provided modified/non-naturally occurring VEGF-A molecules are advantageous because they inhibit angiogenesis and vasculogenesis in a non-cell autonomous fashion. Furthermore, they are highly diffusible. By contrast, conventional angiogenesis inhibitors such as anti-VEGF-A-antibodies show limited diffusion. Hence, the modified VEGF-A molecules of the invention fulfill a long felt need as inhibitors of angiogenesis and vasculogenesis with advantageous properties, and can be employed in the treatment of disorders associated with pathological angiogenesis, such as age related macular degeneration.

In the *appended in vivo* examples, it was surprisingly found that the administration of the herein provided modified/non-naturally occurring VEGF-A molecules to wild-type animals having an endogenous wild-type VEGF-A gene prevent the formation of the vascular system and patterning. Therefore, the herein provided modified/non-naturally occurring VEGF-A molecules are effective angiogenesis inhibitors as well as vasculogenesis inhibitors. As described above, this surprising effect is documented in the non-human animal model, e.g. zebrafish (*Danio rerio*), which is a widely used and important vertebrate model organism. In zebrafish, the development relies on conserved pathways that recapitulate human physiology and disease. The beneficial effect is herein exemplified by the modified VEGF-A 121 isoform, which has an artificial hydrophobic amino acid such as alanine in place of the amino acid at the position corresponding to 84 or 17, e.g., see SEQ ID NOs: 4 and 10 (VEGF-A 121 K84A) or SEQ ID NOs: 6 and 12 (VEGF-A 121 F17A). It is documented herein that these modified VEGF-A molecules inhibit upon administration to fish embryos angiogenesis and vasculogenesis during its development. It is herein demonstrated that the treated larvae show, inter alia, pericardial edema, blood pooling and lack of blood circulation, which are strong indications for vascular defects; see Figure 3B and C of the appended examples. Accordingly, the herein *provided in vivo* examples prove that the herein provided modified/non-naturally occurring VEGF-A molecules are effective inhibitors of angiogenesis as well as vasculogenesis, and can therefore be applied in a clinical setting associated with angiogenic disorders and/or diseases.

As shown in the appended *in vivo* experiments and explained herein below the herein proven beneficial anti-angiogenic properties of the inventive modified VEGF-A molecules are due to amino acid mutations/replacements, e.g. at the position 84 or 17 of wild-type VEGF-A. It is documented in the appended examples (see particularly Example 1) that the introduction of a frame shift mutation in the essential VEGF-A gene in the non-human transgenic animal (*vegfaa*$^{-/-}$ in zebrafish; see Figure 1B) results in a non-functional VEGF-A (non-functional endogenous VEGF-A). It was surprising that these non-human transgenic animals are viable. However, the mutation in the endogenous VEGF-A gene (*vegfaa* in zebrafish) causes severe defects in the vascular system and patterning in the mutant zebrafish; see e.g. Figures 1c and 1d in the appended examples. A non-human transgenic animal, e.g., zebrafish, containing such a VEGF-A null-allele shows severe pericardial edema, blood pooling and lack of circulation due to endothelial cells tubulogenesis defects. It is demonstrated in the appended Examples that these non-human animals unexpectedly survived long enough to test whether modified or naturally-occurring VEGF-A molecules could rescue these animals. Naturally occurring VEGF-A isoforms, e.g., VEGF-A 121 and VEGF-A 165, rescue this severe phenotype, i.e., these animal survived and the health condition of these transgenic animals is ameliorated, for example, in relation to the vascular system and patterning, as shown in the appended examples (see Table 1). Thus, these naturally occurring VEGF-A molecules prevent the formation of pericardial edema by inducing angiogenesis and/or vasculogenesis, e.g., the formation of a functional circulatory loop is induced (see Figure 2). Consequently, said naturally occurring VEGF-As are pro-angiogenic factors or in other words vasculogenesis and angiogenesis stimulators.

It was surprisingly found that the herein provided modifications/mutations of VEGF-A in the context of the VEGF-A that lack the heparin/Nrp1-binding domain, e.g., the VEGF-A 121 isoform, abrogate the rescue ability observed for naturally occurring VEGF-A molecules. It is herein exemplified that administration of the inventive modified/non-naturally occurring VEGF-A molecules into the transgenic animal carrying a VEGF-A null-allele does not alleviate the vascular defects. Accordingly, the demonstrated anti-angiogenic properties are due to the herein provided modification(s) of the amino acids, e.g. at the position 84 or 17, by a hydrophobic amino acid. The herein provided modifications thus render the bona fide stimulator to an inhibitor of angiogenesis and vasculogenesis.

Furthermore, the beneficial effect of the herein provided modified VEGF-A only occurs in case the heparin and/or the Nrp-1 binding domain is absent such as found in the VEGF-A 121 isoform. As exemplified in the appended examples, the replacement of the amino acids such as K84 and F17 by a hydrophobic amino acid in the context of the VEGF-A 165 isoform, which comprises the heparin and/or the Nrp-1 binding domain, rescues the transgenic animal carrying a VEGF-A null allele; Table 2 of the appended examples. These mutations in the VEGF-A 165 isoform have even higher rescue ability than naturally occurring VEGF-A 165 indicating their pro-angiogenic properties.

By contrast, as demonstrated in the appended examples and as described herein, the inventive modifications/mutations in VEGF-A lacking the heparin and/or the Nrp-1 binding domain, as for example found in the VEGF-A 121 isoform, indeed are inhibitors of angiogenesis and vasculogenesis. Thus, the herein provided inventive modifications of VEGF-A demonstrate a specific and surprising effect. Because the herein provided modifications/mutations of VEGF-A have been shown to exert their effect in the *in vivo* animal model provided herein, it is credible that they can be employed in the therapy of disorders associated with pathological angiogenesis.

Example 3 demonstrates further advantages of the modified/mutated VEGF-A polypeptides provided in accordance with the present invention. This *in vivo* experiment documents that the inventive modified/non-naturally occurring VEGF-A molecules are anti-angiogenic molecules that act in a dominant negative fashion, i.e., the modified VEGF-A molecules inhibit endogenous VEGF-A. This effect is demonstrated in several aspects of the appended *in vivo* examples. First, it is documented herein that the non-human transgenic animal, e.g. heterozygotic zebrafish embryos carrying the VEGF-A null-allele at one allele (*vegfaa*$^{+/-}$) and expressing the herein provided modified/non-naturally occurring VEGF-A polypeptides, do not follow the Mendelian segregation and exhibit severe vascular defects reminiscent of homozygous *vegfaa*$^{-/-}$ zebrafish. Second, the administration of the herein provided modified/non-naturally occurring VEGF-A polypeptides to wild-type embryos carrying the wild-type endogenous VEGF-A gene results in severe vascular defects (see e.g. Figure 3B and C). Third, wild-type embryos that contain transplanted cells expressing the modified VEGF-A polypeptides demonstrate said severe vascular defective phenotype (see e.g. Figure 3E). All *these in vivo* experiments prove that the activity of endogenous VEGF-A is inhibited and overcome by the herein provided modified/non-naturally occurring VEGF-A molecules. One possible explanation for this dominant negative effect might be that the herein provided modified VEGF-A molecule traps an endogenous VEGF-A molecule by formation of an inactive heterodimer. Accordingly, the herein provided modified VEGF-A molecules are inhibitors of angiogenesis and vasculogenesis that act in a dominant negative fashion. Further, the herein provided animal models (model organism), particularly a zebrafish model, can serve as a highly useful research tool.

Furthermore, it was surprisingly found herein that the inventive VEGF-A molecules have a high diffusion potential. The appended transplantation experiment proves such high diffusion properties and proves the cell type specificity of the herein provided modified/non-naturally occurring VEGF-A. It is herein demonstrated that the modified VEGF-A molecules cause vascular defects in the proximity of transplanted cells expressing these molecules. This anti-angiogenic effect occurs several micrometers distant from transplanted donor cells (see Figure 3D and E). Consistently, the herein provided modified VEGF-A molecules have a high diffusibility through the ECM. Conventional treatments for angiogenic disorders or diseases such as AMD encompass antibodies against VEGF-A, which have impeded diffusion properties, inter alia, due to their large size (Rosenfeld at al., 2006).

Accordingly, it is demonstrated in the appended examples that the herein provided modified VEGF-A molecules are advantageous in the therapy of disorders associated with pathological angiogenesis compared to conventional angiogenesis inhibitors such as full antibodies. Consequently, the experimental data in the appended examples provide for a clear rationale to use the inventive modified VEGF-A molecules in the therapy of disorders or diseases associated with pathological angiogenesis.

In the prior art, various modified VEGF-A variants are described as angiogenesis and vasculogenesis stimulators as well as inhibitors. These modified VEGF-A variants have also been suggested for the use in the therapy of AMD, e.g., WO 1998/016551, WO 2007/044534 A2, WO 01/53345 A1 and WO 2006/119035 A2.

WO 1998/016551 relates to VEGF-A variants that comprise amino acid modifications of at least one cysteine residue at position 51 and/or 60, wherein said modification inhibits the ability of said variant to properly dimerize with another VEGF-A monomer.

WO 01/53345 A1 is directed to a VEGF-A 148 isoform that is predicted to be *an in vivo* inhibitor of other VEGF-A isoforms.

WO 2006/119035 A2 discloses VEGF-A variants comprising modifications in the heparin binding domain that is present in the VEGF-A 164, VEGF-A 165, VEGF-A 189 and VEGF-A 206 isoforms.

WO 2007/044534 A2 relates to VEGF-A variants comprising a replacement by a basic amino acid of one or more amino acids selected from the group consisting of I83, E44, E67, E72, E73 and Q87. The modification results in a decrease in bioactivity.

WO 2010/102380 A1 is directed to modifications of the VEGF-A 165 isoform comprising a mutation at residue 159, 161, 162, 163, 164 or a combination thereof.

WO 1997/008313 is concerned with the investigation of the binding affinity of several VEGF-A variants to the VEGFR-2 and VEGFR-1 receptor. The VEGF-A variants contain alanine mutations, i.e., either single mutations or combinations of mutations, in the context of the VEGF-A 165 and VEGF-A 1-109 isoforms. WO 1997/008313 suggests using these molecules for treating indications in which vasculogenesis/angiogenesis or anti-vasculogenesis/anti-angiogenesis is desired. WO 1997/008313 discloses an alanine mutation at position 84 (lysine) or at position 17 (phenylalanine) in the VEGF-A 165 and VEGF-A 1-109 isoforms. However, WO 1997/008313 does not disclose or propose a modified VEGF-A 121 having a mutation at position 84 (lysine) or at position 17 (phenylalanine).

As it is documented in the appended examples of the present invention, the resulting effect of the modification, i.e., whether the modified molecule is a pro- angiogenic or an anti-angiogenic factor, is dependent on the isoform in which the mutation is introduced. Thus, the results provided in the present application, namely that the herein provided modifications of VEGF-A molecules render the bona fide stimulator to an inhibitor of angiogenesis and/or vasculogenesis with beneficial properties, is surprising.

In summary, the herein provided advantageous modifications/mutations in the herein provided VEGF-A molecules have not been disclosed or proposed in the prior art.

It was surprisingly found in the appended Examples that, for example, the replacement of lysine or phenylalanine at position 84 or 17 by a hydrophobic amino acid in the context of the VEGF-A 121 isoform had a surprisingly strong anti-angiogenic/vasculogenic effect in *in vivo* organisms.

The present invention has, inter alia, the following advantages over conventional anti-angiogenic agents: The inventive compounds, e.g. the modified VEGF-A proteins/polypeptides as described herein are strong inhibitors of angiogenesis and vasculogenesis. As one further advantage, the herein provided compounds prove their inhibitory effects in *in vivo* systems and thus can be potentially used in therapy of diseases or disorders associated with pathological angiogenesis. One further advantage is that the herein provided compounds act in a dominant negative fashion, i.e., the modified VEGF-A molecules inhibit endogenous VEGF-A.

One further advantage is that the herein provided compounds have a high diffusibility. The recombinant humanized antibody Bevacizumab (Avastin, Genentech) targeting VEGF-A has a molecular weight of approximately 150 kDa. The monoclonal antibody fragment, Ranibizumab (Lucentis, Genentech) neutralizing all VEGF-A isoforms has a molecular weight of approximately 50 kDa. The herein provided modified VEGF-A polypeptides are thus approximately 15 and 5 times smaller compared to full antibodies and the fragment antigen-binding domain (Fab), respectively. In general, small proteins, e.g., 28 kDa, applied topically are able to penetrate to the posterior segment, whereas large molecules such as entire antibodies are not able to penetrate across the ocular surface when delivered topically (Chen et al., 2011). Accordingly, the difference in size may render the herein disclosed inventive molecules more diffusible than conventional therapeutics. Furthermore, VEGF-A 121 lacks a heparin binding domain and is thus not immobilized to the ECM. In view of this, it is shown herein that the inventive polypeptides have a superior solubility and diffusion compared to VEGF-A molecules that comprise a heparin and/or Nrp-1 binding domain. Furthermore, the herein provided modified VEGF-A polypeptides can comprise only a modification/replacement of one amino acid and are thus highly similar to the endogenous VEGF-A polypeptide. Therefore, unwanted immunogenicity of typical recombinant proteins is avoided.

It was surprisingly shown in the appended examples that the replacement of the amino acid residue lysine by a hydrophobic amino acid at position 84 of wild-type VEGF-A as shown in SEQ ID NO: 2 renders the modified VEGF-A polypeptide as an angiogenesis and/or vasculogenesis inhibitor. It was also surprisingly shown that the replacement of the amino acid phenylalanine by a hydrophobic amino acid at position 17 of wild-type VEGF-A as shown in SEQ ID NO: 2 renders the modified VEGF-A polypeptide as an angiogenesis and/or vasculogenesis inhibitor.

Thus, the present invention relates to a modified vascular endothelial growth factor A (VEGF-A) polypeptide, wherein said modified vascular endothelial growth factor A (VEGF-A) comprises a hydrophobic amino acid in place of the lysine at the position corresponding to position 84 of the wild-type VEGF-A as shown in SEQ ID NO: 2. The invention also relates to a modified vascular endothelial growth factor A (VEGF-A) polypeptide, wherein said modified vascular endothelial growth factor A (VEGF-A) comprises a hydrophobic amino acid in place of the phenylalanine at the position corresponding to position 17 of the wild-type VEGF-A as shown in SEQ ID NO: 2. Further, the present invention relates to a modified vascular endothelial growth factor A (VEGF-A) polypeptide, wherein said modified vascular endothelial growth factor A (VEGF-A) comprises a hydrophobic amino acid in place of the lysine at the position corresponding to position 84 of the wild-type VEGF-A as shown in SEQ ID NO: 2, and wherein said modified vascular endothelial growth factor A (VEGF-A) comprises a hydrophobic amino acid in place of the phenylalanine at the position corresponding to position 17 of the wild-type VEGF-A as shown in SEQ ID NO: 2.

The present invention relates to the following items:

1. A modified vascular endothelial growth factor A (VEGF-A) polypeptide for use in treating an angiogenic disorder.

2. A method of treatment for an angiogenic disorder comprising the step of administering to a subject in need of such treatment a pharmaceutical effective amount of a modified vascular endothelial growth factor A (VEGF-A) polypeptide.

3. The polypeptide for use of item 1, or the method of item 2, wherein said angiogenic disorder is neovascular pathology.

4. The polypeptide for use of item 1 or 3, or the method of item 2 or 3, wherein said neovascular pathology is selected from the group of age related macular degeneration, neovascular age related macular degeneration, diabetic macular edema, high risk-corneal transplantation and neovascular glaucoma.

5. The polypeptide for use of any one of items 1, 3 and 4, or the method of any one of items 2 to 4, wherein said modified vascular endothelial growth factor A (VEGF-A) comprises a hydrophobic amino acid in place of the lysine at the position corresponding to position 84 of the wild-type VEGF-A as shown in SEQ ID NO: 2.

6. The polypeptide for use of any one of items 1, 3 and 4, or the method of any one of items 2 to 4, wherein said modified vascular endothelial growth factor A (VEGF-A) comprises a hydrophobic amino acid in place of the phenylalanine at the position corresponding to position 17 of the wild-type VEGF-A as shown in SEQ ID NO: 2.

7. The polypeptide for use of any one of items 1, 3 and 4, or the method of any one of items 2 to 4, wherein said modified vascular endothelial growth factor A (VEGF-A) comprises a hydrophobic amino acid in place of:

the lysine at the position corresponding to position 84 of the wild-type VEGF-A as shown in SEQ ID NO: 2; and

the phenylalanine at the position corresponding to position 17 of the wild-type VEGF-A as shown in SEQ ID NO: 2.

8. The polypeptide for use of any one of items 1 and 3 to 7, or the method of any one of items 2 to 7, wherein said modified vascular endothelial growth factor A (VEGF-A) functions as an angiogenesis inhibitor.

9. The polypeptide for use of any one of items 1 and 3 to 8, or the method of any one of items 2 to 8, wherein said hydrophobic amino acid is selected from the group consisting of alanine, valine, isoleucine, leucine and methionine.

10. The polypeptide for use of any one of items 1 and 3 to 9, or the method of any one of items 2 to 9, wherein said modified vascular endothelial growth factor A (VEGF-A) lacks the heparin binding domain and/or the Nrp-1 binding domain.

11. The polypeptide for use of any one of items 1 and 3 to 10, or the method of any one of items 2 to 10, wherein the modified vascular endothelial growth factor A (VEGF-A) is the VEGF-A 121 isoform.

12. The polypeptide for use of any one of items 1 and 3 to 11, or the method of any one of items 2 to 11, wherein said modified vascular endothelial growth factor A (VEGF-A) is human modified vascular endothelial growth factor A (VEGF-A).

13. The polypeptide for use of any one of items 1 and 3 to 12, or the method of any one of items 2 to 12, wherein said modified vascular endothelial growth factor A (VEGF-A) is selected from the group consisting of:

(a) a polypeptide encoded by a nucleic acid molecule given in SEQ ID NO: 1, wherein the nucleotides AAA at position 250 to 252 of SEQ ID NO: 1 are replaced by nucleotides encoding said hydrophobic amino acid, and/or wherein the nucleotides TTC at position 49 to 51 of SEQ ID NO: 1 are replaced by nucleotides encoding said hydrophobic amino acid;

(b) a polypeptide given in SEQ ID NO: 2, wherein the lysine residue corresponding to position 84 of SEQ ID NO: 2; and/or wherein the phenylalanine corresponding to position 17 of SEQ ID NO: 2 is replaced by said hydrophobic amino acid;
(c) a polypeptide encoded by a nucleic acid molecule hybridizing under stringent conditions to the complementary strand of a nucleic acid molecule encoding the polypeptide as defined in (a) or (b);
(d) a polypeptide having at least 55% identity to the polypeptide of any one of (a) to (c) and functioning as an inhibitor of angiogenesis; and
(e) a polypeptide that functions as an inhibitor of angiogenesis comprising an amino acid sequence encoded by a nucleic acid molecule being degenerate as a result of the genetic code to the nucleotide sequence of a nucleic acid molecule as defined in any one of (a), (c) and (d).

14. The polypeptide for use of any one of items 1 and 3 to 13, or the method of any one of items 2 to 13, wherein said hydrophobic amino acid is alanine.

15. The polypeptide for use of any one of items 1 and 3 to 14, or the method of any one of items 2 to 14, wherein said modified vascular endothelial growth factor A (VEGF-A) comprises at least one additional mutation selected from the group consisting of amino acid substitution(s), addition(s), deletions(s) and duplication(s).

16. A modified vascular endothelial growth factor A (VEGF-A) polypeptide, wherein said modified vascular endothelial growth factor A (VEGF-A) comprises a hydrophobic amino acid in place of the lysine at the position corresponding to position 84 of the wild-type VEGF-A as shown in SEQ ID NO: 2.

17. A modified vascular endothelial growth factor A (VEGF-A) polypeptide, wherein said modified vascular endothelial growth factor A (VEGF-A) comprises a hydrophobic amino acid in place of the phenylalanine at the position corresponding to position 17 of the wild-type VEGF-A as shown in SEQ ID NO: 2.

18. A modified vascular endothelial growth factor A (VEGF-A) polypeptide, wherein said modified vascular endothelial growth factor A (VEGF-A) comprises a hydrophobic amino acid in place of:

the lysine at the position corresponding to position 84 of the wild-type VEGF-A as shown in SEQ ID NO: 2; and

the phenylalanine at the position corresponding to position 17 of the wild-type VEGF-A as shown in SEQ ID NO: 2.

19. The polypeptide of any one of items 16 to 18, wherein said modified vascular endothelial growth factor A (VEGF-A) functions as an angiogenesis inhibitor.

20. The polypeptide of any one of items 16 to 19, wherein said hydrophobic amino acid is selected from the group consisting of alanine, valine, isoleucine, leucine and methionine.

21. The polypeptide of any one of items 16 to 20, wherein said modified vascular endothelial growth factor A (VEGF-A) lacks the heparin binding domain and/or the Nrp-1 binding domain.

22. The polypeptide of any one of items 16 to 21, wherein the modified vascular endothelial growth factor A (VEGF-A) is the VEGF-A 121 isoform.

23. The polypeptide of any one of items 16 to 22, wherein said modified vascular endothelial growth factor A (VEGF-A) is human modified vascular endothelial growth factor A (VEGF-A).

24. The polypeptide of any one of items 16 to 23, wherein said modified vascular endothelial growth factor A (VEGF-A) is selected from the group consisting of:

(a) a polypeptide encoded by a nucleic acid molecule given in SEQ ID NO: 1, wherein the nucleotides AAA at position 250 to 252 of SEQ ID NO: 1 are replaced by nucleotides encoding said hydrophobic amino acid, and/or wherein the nucleotides TTC at position 49 to 51 of SEQ ID NO: 1 are replaced by nucleotides encoding said hydrophobic amino acid;
(b) a polypeptide given in SEQ ID NO: 2, wherein the lysine residue corresponding to position 84 of SEQ ID NO: 2; and/or wherein the phenylalanine corresponding to position 17 of SEQ ID NO: 2 is replaced by said hydrophobic amino acid;

(c) a polypeptide encoded by a nucleic acid molecule hybridizing under stringent conditions to the complementary strand of the nucleic acid molecule encoding the polypeptide as defined in (a) or (b);
(d) a polypeptide having at least 55% identity to the polypeptide of any one of (a) to (c) and functioning as an inhibitor of angiogenesis; and
(e) a polypeptide that functions as an inhibitor of angiogenesis comprising an amino acid sequence encoded by a nucleic acid molecule being degenerate as a result of the genetic code to the nucleotide sequence of a nucleic acid molecule as defined in any one of (a), (c) and (d).

25. The polypeptide of any one of items 16 to 24, wherein said hydrophobic amino acid is alanine.

26. The polypeptide of any one of items 16 to 25, wherein said modified vascular endothelial growth factor A (VEGF-A) comprises at least one additional mutation selected from the group consisting of amino acid substitution(s), addition(s), deletions(s) and duplication(s).

27. An antibody specifically binding to the polypeptide of any one of items 16 to 26, wherein said antibody specifically binds to an epitope comprising a hydrophobic amino acid in place of:

the lysine at the position corresponding to position 84 of the wild-type VEGF-A as shown in SEQ ID NO: 2; and/or

the phenylalanine at the position corresponding to position 17 of the wild-type VEGF-A as shown in SEQ ID NO: 2;

and wherein said hydrophobic amino acid is selected from the group consisting of alanine, valine, isoleucine, leucine and methionine.

28. A nucleic acid molecule encoding the polypeptide of any one of items 16 to 26.

29. A vector comprising the nucleic acid molecule of item 28.

30. A pharmaceutical composition comprising the polypeptide of any one of items 16 to 26, the nucleic acid molecule of item 28 and/or a pharmaceutical excipient.

31. A host transformed with a vector of item 29 or comprising the nucleic acid molecule of item 28.

32. A method for producing the polypeptide of any one of items 16 to 26, wherein said method comprises culturing/raising the host of item 31 and optionally isolating the produced polypeptide.

33. The nucleic acid molecule of item 28, the vector of item 29, and/or the pharmaceutical composition of item 30 for use in treating an angiogenic disorder.

34. The nucleic acid molecule for use of item 33, the vector for use of item 33, and/or the pharmaceutical composition for use of item 33, wherein said angiogenic disease is age related macular degeneration.

35. The nucleic acid molecule for use of item 34, the vector for use of item 34, and/or the pharmaceutical composition for use of item 34, wherein said age related macular degeneration is neovascular age related macular degeneration.

36. A non-human transgenic animal, wherein said transgenic animal comprises an endogenous vascular endothelial growth factor A (VEGF-A) gene, wherein said endogenous vascular endothelial growth factor A (VEGF-A) gene comprises an artificially introduced mutation.

37. The non-human transgenic animal of item 36, wherein the expression of the mutated endogenous vascular endothelial growth factor A (VEGF-A) gene results in a non-functional vascular endothelial growth factor A (VEGF-A) polypeptide.

38. The non-human transgenic animal of item 36 or 37, wherein said transgenic animal comprises the modified vascular endothelial growth factor A (VEGF-A) polypeptide of any one of items 16 to 26.

39. The non-human transgenic animal of any one of items 36 to 38, wherein said transgenic animal is a zebrafish.

40. The non-human transgenic animal of any one of items 36 to 39, wherein said mutated endogenous vascular growth factor A (VEGF-A) gene has a sequence as shown in SEQ ID NO: 32.

41. The non-human transgenic animal of any one of items 36 to 40, wherein said transgenic animal is homozygous for said mutated endogenous vascular growth factor A (VEGF-A) gene.

42. A method for producing the non-human transgenic animal of any one of items 36 to 41, said method comprises the introduction of an artificial mutation into the endogenous vascular endothelial growth factor A (VEGF-A) gene in a non-human animal at a stage no later than the 4-cell stage.

43. The method of item 42, wherein the expression of the mutated endogenous vascular endothelial growth factor A (VEGF-A) gene results in a non-functional vascular endothelial growth factor A (VEGF-A) polypeptide.

44. A method for identifying a candidate modified vascular endothelial growth factor A (VEGF-A) as a possible agent for treating an angiogenic disorder, wherein said method comprises:

(a) contacting or otherwise exposing a non-human transgenic animal to said candidate modified vascular endothelial growth factor A (VEGF-A), wherein said transgenic animal comprises an endogenous vascular endothelial growth factor A (VEGF-A) gene with an artificially introduced mutation, wherein the expression of the mutated endogenous vascular endothelial growth factor A (VEGF-A) gene results in a non-functional vascular endothelial growth factor A (VEGF-A) polypeptide;
(b) monitoring the health condition of said transgenic animal comprising said modified vascular endothelial growth factor A (VEGF-A); and
(c) selecting said modified vascular endothelial growth factor A (VEGF-A) that results in impaired vasculogenesis of the transgenic animal, wherein said impaired vasculogenesis is indicative of the capacity of said selected modified vascular endothelial growth factor A (VEGF-A) to inhibit angiogenesis.

45. The method of item 44, wherein in step (c) the health conditions of said transgenic animal comprising said modified vascular endothelial growth factor A (VEGF-A) polypeptide and said endogenous vascular endothelial growth factor A (VEGF-A) gene with an artificially introduced mutation are compared to a transgenic animal comprising a wild-type vascular endothelial growth factor A (VEGF-A) polypeptide and said endogenous vascular endothelial growth factor A (VEGF-A) gene with an artificially introduced mutation; and wherein said modified vascular endothelial growth factor A (VEGF-A) is selected that results in impaired vasculogenesis.

It is herein understood that "vascular endothelial growth factor A", "VEGF-A", "VEGFA" or "vascular endothelial growth factor A (VEGF-A)" is used interchangeable herein. The amino acid sequence of human wild-type VEGF-A is well known in the art. An exemplary amino acid sequence of wild-type VEGF-A of the isoform 121 is herein given in SEQ ID NO: 2. The nucleic acid sequence encoding the amino acid sequence of wild-type VEGF-A of the isoform 121 is herein given in SEQ ID NO: 1. The amino acid sequence of wild-type VEGF-A can also be retrieved from public data bases such as Uniprot or NCBI. Wild-type VEGF-A has the accession number UniProtKB: P15692-9. In zebrafish, said "vascular endothelial growth factor-A" or "VEGF-A" corresponds to VEGFAA, Vegfaa or vegfaa. A corresponding exemplary amino acid sequence of wild-type zebrafish VEGF-A is given in SEQ ID NO: 8. A corresponding exemplary nucleotide sequence encoding wild-type zebrafish VEGF-A is given in SEQ ID NO: 7.

In preferred aspects of the invention, the modified VEGF-A according to the invention does not contain a signal peptide. Exemplary SEQ ID NO: 2 or 8 does not contain such a signal peptide. The signal peptide is a short stretch of amino acids usually present at the N-terminus of several proteins that are destined to the secretory pathway. In case of VEGF-A, this sequence is necessary for its secretion. An exemplary amino acid sequence of wild-type human VEGF-A 121 that has the signal peptide is given in SEQ ID NO: 16. A corresponding exemplary amino acid sequence of zebrafish VEGF-A 121 is given in SEQ ID NO: 22. Nucleotide sequences encoding such wild-type VEGF-A polypeptides with a signal peptide are given in SEQ ID NOs: 15 and 21. In preferred aspects of the invention, the modified VEGF-A is a modified VEGF-A 121 isoform lacking the signal peptide, wherein said modified VEGF-A comprises a hydrophobic amino acid in place of the lysine at the position corresponding to position 84 of the wild-type VEGF-A as shown in SEQ ID NO: 2, and/or wherein said modified vascular endothelial growth factor A (VEGF-A) comprises a hydrophobic amino acid in place of the phenylalanine at the position corresponding to position 17 of the wild-type VEGF-A as shown in SEQ ID NO: 2.

The present invention relates to a modified vascular endothelial growth factor A (VEGF-A) polypeptide. The modified VEGF-A, mutated VEGF-A or non-naturally occurring VEGF-A comprises (a) specific amino acid replacement(s)/mutation(s), which is/are not found in the naturally occurring VEGF-A or wild-type VEGF-A. As used herein the term "modified vascular endothelial growth factor A (VEGF-A)", "modified VEGF-A", "mutated VEGF-A", "genetically modified VEGF-A", "non-natural VEGF-A" or "non-naturally occurring VEGF-A" in accordance with the present invention refers to a modified VEGF-A polypeptide, wherein said modified vascular endothelial growth factor A (VEGF-A) comprises a hydrophobic amino acid in place of:

the lysine at the position corresponding to position 84 of the wild-type VEGF-A as shown in SEQ ID NO: 2; and/or the phenylalanine at the position corresponding to position 17 of the wild-type VEGF-A as shown in SEQ ID NO: 2, wherein preferably said hydrophobic amino acid is selected from the group consisting of alanine, valine, isoleucine, leucine and methionine.

In preferred aspects of the invention, the lysine or the phenylalanine corresponding to position 84 or 17, respectively, that is replaced/changed/mutated in the modified VEGF-A as herein provided is selected by comparison of homology. Therefore, in preferred aspects, the invention relates to a modified VEGF-A polypeptide, wherein said modified vascular endothelial growth factor A (VEGF-A) comprises a hydrophobic amino acid in place of:

the lysine at the position that by comparison of homology corresponds to position 84 of the wild-type VEGF-A as shown in SEQ ID NO: 2; and/or
the phenylalanine at the position that by comparison of homology corresponds to position 17 of the wild-type VEGF-A as shown in SEQ ID NO: 2, wherein preferably said hydrophobic amino acid is selected from the group consisting of alanine, valine, isoleucine, leucine and methionine.

In other words, the modified VEGF-A comprises an amino acid sequence, wherein the lysine at the position 84 of the wild-type VEGF-A as shown in SEQ ID NO: 2 or an amino acid residue corresponding by comparison of homology to said lysine at position 84 of the wild-type VEGF-A as shown in SEQ ID NO: 2 is substituted/changed/mutated to a hydrophobic amino acid; and/or
wherein the phenylalanine at the position 17 of the wild-type VEGF-A as shown in SEQ ID NO: 2 or an amino acid residue corresponding by comparison of homology to said phenylalanine at position 17 of the wild-type VEGF-A as shown in SEQ ID NO: 2 is substituted/changed/mutated to a non-aromatic hydrophobic amino acid.
The amino acid residue corresponding to (preferably by comparison of homology to) said lysine at position 84 of the wild-type VEGF-A as shown in SEQ ID NO: 2 is preferably a lysine. The amino acid residue corresponding to (preferably by comparison of homology to) said phenylalanine at position 17 of the wild-type VEGF-A as shown in SEQ ID NO: 2 is preferably a phenylalanine.
In other words, the modified VEGF-A of the present invention comprises (an) amino acid mutation(s), i.e. (an) alanine mutation(s), at residue(s) 17 and/or 84 or residue(s) being homologous to said residues.
In most preferred aspects of the invention, the modified VEGF-A of the present invention comprises the amino acid substitution(s) F17A and/or K84A. Thus, in most preferred aspects of the invention, the modified VEGF-A or the functional fragment thereof that functions as angiogenesis and/or vasculogenesis inhibitor comprises a hydrophobic amino acid in place of the lysine at the position 84 of the wild-type VEGF-A as shown in SEQ ID NO: 2; and/or in place of the the phenylalanine at the position 17 of the wild-type VEGF-A as shown in SEQ ID NO: 2, wherein preferably said hydrophobic amino acid is selected from the group consisting of alanine, valine, isoleucine, leucine and methionine.
As used herein, the term "comprises a hydrophobic amino acid in place of" means that the specific amino acid residue that is present in the naturally occurring VEGF-A (e.g. lysine or phenylalanine or the corresponding amino acid residue thereto) is mutated to the hydrophobic amino acid, preferably to a non-aromatic hydrophobic amino acid, in the modified VEGF-A. Accordingly, the modified VEGF-A comprises this mutated hydrophobic amino acid.
As used herein, the term "the lysine at the position corresponding to position 84 of the wild-type VEGF-A as shown in SEQ ID NO: 2" means the lysine at position 84 of SEQ ID NO: 2 or a specific amino acid residue in a known wild-type sequence corresponding to said specific lysine at position 84 of SEQ ID NO: 2. The corresponding amino acid residue at the corresponding position can be selected by standard homology screenings or PCR-mediated screening techniques for related sequences as detailed below. In preferred aspects of the invention, the corresponding amino acid residue is selected by comparison of homology. Accordingly, as used herein, the term "the lysine at the position that by comparison of homology corresponds to position 84 of the wild-type VEGF-A as shown in SEQ ID NO: 2" means the lysine at position 84 of SEQ ID NO: 2 or a specific amino acid residue in a known wild-type sequence that is homologous to said specific lysine at position 84 of SEQ ID NO: 2. The homologous amino acid residue can be identified by e.g. amino acid sequence alignment, such as multiple protein alignment. Exemplary homologous amino acids residues can be lysine, arginine or histidine. Most preferably, the homologous amino acid residue is lysine.
In preferred aspects, said lysine lies between I83 and P85 as found in the human wild-type VEGF-A as shown in SEQ ID NO: 2 or said homologous amino acid or lysine corresponding to position 84 of SEQ ID NO: 2 lies between an isoleucine and proline. More preferably, said homology exist over a stretch of amino acids, e.g. 10, more preferably 20, more preferably 30, more preferably 50, or more preferably 100 amino acid residues, or most preferably the homology exist over the whole amino acid stretch, e.g. VEGF-A. Exemplary alignments of the amino acid stretches of interest are shown in tables 3 and 5. The illustrative amino acid residue that is mutated or replaced according to the invention by the hydrophobic amino acid is illustrated in bold.

As used herein the term "the phenylalanine at the position corresponding to position 17 of the wild-type VEGF-A as shown in SEQ ID NO: 2" means the phenylalanine at position 17 of SEQ ID NO: 2 or a specific amino acid residue in a known wild-type sequence corresponding to said phenylalanine at position 17 of SEQ ID NO: 2. As mentioned above, the corresponding amino acid residue at the corresponding position can be selected by standard homology screenings or PCR-mediated screening techniques for related sequences as detailed below. In preferred aspects, the term "the phenylalanine at the position that by comparison of homology corresponds to position 17 of the wild-type VEGF-A as shown in SEQ ID NO: 2" means the phenylalanine at position 17 of SEQ ID NO: 2 or a specific amino acid residue in a known wild-type sequence that is homologous to said phenylalanine at position 17 of SEQ ID NO: 2. Exemplary homologous amino acids residues can be phenylalanine, tryptophan or tyrosine. Most preferably, the homologous amino acid residue is phenylalanine. In preferred aspects, said phenylalanine lies between K16 and M18 as found in the human wild-type VEGF-A as shown in SEQ ID NO: 2 or said homologous amino acid residue lies between an lysine and methionine.

Table 3. Exemplary lysines at the position corresponding to position 84 of wild-type VEGF-A as given in SEQ ID NO: 2 that are replaced by a hydrophobic amino acid in the modified VEGF-A.

| | | | | | | | **VEGF-A and SEQ ID NO** |
|---|---|---|---|---|---|---|---|
| M | R | I | **K** | P | H | Q | Human VEGF-A SEQ ID NO: 34 |
| M | R | I | **K** | P | H | Q | Mouse VEGF-A SEQ ID NO: 35 |
| L | R | V | **K** | Q | R | V | Zebrafish VEGF-A SEQ ID NO: 36 |
| M | K | I | **K** | P | H | I | Frog VEGF-A SEQ ID NO: 37 |

**Table 4.** Exemplary phenylalanines at the position corresponding to position 17 of wild-type VEGF-A as given in SEQ ID NO: 2 that are replaced by a hydrophobic amino acid in the modified VEGF-A.

| | | | | | | | **VEGF-A and SEQ ID NO** |
|---|---|---|---|---|---|---|---|
| V | V | K | **F** | M | D | V | Human VEGF-A SEQ ID NO: 38 |
| V | I | K | **F** | M | D | V | Mouse VEGF-A SEQ ID NO: 39 |
| V | I | P | **F** | M | D | V | Zebrafish VEGF-A SEQ ID NO: 40 |
| V | V | K | **F** | L | K | V | Frog VEGF-A SEQ ID NO: 41 |

For example, said lysine corresponding to position 84 of the wild-type VEGF-A as shown in SEQ ID NO: 2 or said amino acid residue that is homologous to the lysine at position 84 of SEQ ID NO: 2 is also found at position 84 in zebrafish VEGF-A as given in SEQ ID NO: 8. Thus, said position 84 of the human VEGF-A corresponds to position 84 of the zebrafish VEGF-A. Similarly, said position 17 of the human VEGF-A corresponds to position 17 of the zebrafish VEGF-A as given in SEQ ID NO: 8.

An exemplary sequence of the modified VEGF-A polypeptides comprising an alanine at the position that by comparison of homology corresponds to position 84 of the wild type human VEGF-A as shown in SEQ ID NO: 2 is given in SEQ ID NO: 4. The encoding nucleotide sequence is given in SEQ ID NO: 3. An exemplary sequence of the modified VEGF-A polypeptides comprising an alanine at the position that by comparison of homology corresponds to position 17 of the wild type human VEGF-A as shown in SEQ ID NO: 2 is given in SEQ ID NO: 6. The encoding nucleotide sequence is given in SEQ ID NO: 5. An exemplary sequence of the modified VEGF-A polypeptides comprising an alanine at the position that by comparison of homology corresponds to position 84 of the wild type zebrafish VEGF-A as shown in SEQ ID NO: 8 is given in SEQ ID NO: 10. The encoding nucleotide sequence is given in SEQ ID NO: 9. An exemplary sequence of the modified VEGF-A polypeptides comprising an alanine at the position that by comparison of homology corresponds to position 17 of the wild type zebrafish VEGF-A as shown in SEQ ID NO: 8 is given in SEQ ID NO: 12. The encoding nucleotide sequence is given in SEQ ID NO: 11. Furthermore, in case the modified VEGF-A comprises a signal peptide, said corresponding lysine or said corresponding amino acid residue, which is homologous to the lysine at position 84 of SEQ ID NO: 2 and which is replaced to generate an inventive modified VEGF-A, is at position 110 of SEQ ID NO: 18. Similarly, said corresponding phenylalanine or said corresponding amino acid residue, which is homologous to the phenylalanine at position 17 of SEQ ID NO: 2 and which is replaced to generate an inventive modified VEGF-A, is at position 43 of SEQ ID NO: 20. Accordingly, exemplary modified VEGF-A polypeptides according to the invention that have a signal peptide are given in SEQ ID NO: 18 or 20. Nucleotide sequences encoding such modified VEGF-A polypeptides are given in SEQ ID NOs: 17 and 19. Exemplary modified zebrafish VEGF-A polypeptides with a signal peptide are given in SEQ ID NOs: 24 and 26. Nucleotide sequences encoding such modified VEGF-A polypeptides are given in SEQ ID NOs: 23 and 25.

As described above, wild-type VEGF-A sequences are e.g. given in SEQ ID NOs: 1 and 7 (nucleotide sequences) or 2 and 8 (amino acid sequences). For further wild-type sequences of VEGF-A, for example from other species, that will be isolated in the future the position corresponding to K84 or F17 of the human VEGF-A is easily deducible employing the sequence information that is already available due to the high homology between VEGF-A polypeptides. Methods to be employed in order to elucidate further wild-type sequences of VEGF-A and methods to identify the lysine or phenylalanine, which corresponds to the lysine on position 84 of human VEGF-A or the phenylalanine on position 17 of human VEGF-A, are, inter alia, standard homology screenings as mentioned above and PCR-mediated screening techniques for related sequences. For the identification of further wild-type sequences of VEGF-A, as well as for the detection of the relevant specific amino acid residues corresponding to the lysine on position 84 or to the phenylalanine on position 17 of the human VEGF-A, computer programs can also be utilized.

For example, BLAST2.0, which stands for Basic Local Alignment Search Tool (Altschul, Nucl. Acids Res. 25 (1997), 3389-3402; Altschul, J. Mol. Evol. 36 (1993), 290-300; Altschul, J. Mol. Biol. 215 (1990), 403-410), can be used to search for local sequence alignments. Furthermore, CLUSTAL Omega (Sievers (2014) Curr Protoc Bioinformatics. 48:3.13.1-3.13.16) can be used. BLAST produces alignments of both nucleotide and amino acid sequences to determine sequence similarity. Because of the local nature of the alignments, BLAST is especially useful in determining exact matches or in identifying similar sequences and (a) corresponding amino acid residue(s) that is/are homologous to e.g. lysine or phenylalanine at position 84 or 17, respectively. Analogous computer techniques using BLAST (Altschul, 1997, 1993 and 1990, supra) are used to search for identical or related molecules in nucleotide databases such as GenBank or EMBL. This analysis is much faster than multiple membrane-based hybridizations. In addition, the sensitivity of the computer search can be modified to determine whether any particular match is categorized as exact or similar. The basis of the search is the product score which is defined as:

$$\frac{\%\text{sequence identity x } \% \text{ maximum BLAST score}}{100}$$

and it takes into account both the degree of similarity between two sequences and the length of the sequence match. For example, with a product score of 40, the match will be exact within a 1-2% error; and at 70, the match will be exact. Similar molecules are usually identified by selecting those which show product scores between 15 and 40, although lower scores may identify related molecules.

The term "by comparison of homology" means, in accordance with this invention, that amino acid sequences of other VEGF-A polypeptides are compared with the amino acid sequence of the amino acid sequence of the VEGF-A (as depicted, inter alia, in SEQ ID NOs: 2 and 8). As described above, the specific amino acid residue that is replaced/mutated/changed in the modified VEGF-A can be identified by comparison of sequence homology. For example, two segments of nucleotide sequences or amino acid sequences can have shared ancestry because of either a speciation event (orthologs) or a duplication event (paralogs). Homology among polypeptides or nucleotide seqeunces is typically inferred from their sequence similarity. Alignments of multiple sequences can herein be used to indicate which regions or specific amino acids of each sequence are homologous. In general, a sequence alignment is a way of arranging the sequences of DNA, RNA, or protein to identify regions of similarity or a specific amino acid that may be a consequence of functional, structural, or evolutionary relationships between the sequences. Aligned sequences of nucleotide or amino acid residues are typically represented as rows within a matrix. Tables 3 and 4 show exemplary aligned amino acid stretches of illustrative VEGF-A polypeptides. In the aspects or embodiments according to the invention, the corresponding amino acid residue or the corresponding position can optionally be selected by comparison of homology. In preferred aspects of the present invention, the corresponding amino acid residue or the corresponding position is selected by comparison of homology. However, the skilled person understands that the corresponding amino acid residue or corresponding position can be selected by any means and methods available in the art.

The present invention, also relates to modified VEGF-A polypeptides or nucleotide sequences encoding such modified VEGF-A polypeptides that further comprise deletion(s), addition(s), insertion(s), duplication(s), inversion(s) and/or recombination(s). Those deviations may naturally occur or be produced via recombinant DNA techniques well known in the art; see, for example, the techniques described in Sambrook (Molecular Cloning; A Laboratory Manual, 2nd Edition, Cold Spring Harbour Laboratory Press, Cold Spring Harbour, NY (1989)) and Ausubel, "Current Protocols in Molecular Biology", Green Publishing Associates; and Wiley Interscience, N.Y. (1989). The allelic variations may be naturally occurring allelic variants as well as synthetically produced or genetically engineered variants. The polypeptides, peptides or protein fragments encoded by the various derivatives, allelic variants, homologues or analogues of the above-described nucleic acid molecules encoding VEGF-A may share specific common characteristics, such as molecular weight, immunological reactivity, conformation etc., as well as physical properties, such as electrophoretic mobility, chromatographic behavior, sedimentation coefficients, pH optimum, stability, solubility, spectroscopic properties etc.

The invention also relates a nucleic acid molecule comprising a nucleic acid molecule encoding the herein provided modified VEGF-A polypeptide.

Modified VEGF-A polypeptides, genetically modified VEGF-A proteins or related polypeptides (functional fragments thereof and/or fusion proteins comprising said VEGF-A proteins/polypeptides having an identity of at least 55% to the specific VEGF-A proteins provided and defined herein, and the like) have primarily the activity as an angiogenesis inhibitor and/or vasculogenesis inhibitor according to the present invention. Furthermore, the modified VEGF-A polypeptides, genetically modified VEGF-A proteins or related polypeptides have a dominant negative function. A dominant negative molecule adversely affects the naturally-occurring polypeptide.

The skilled person is aware of the term angiogenesis or vasculogenesis inhibitor. For example an angiogenesis inhibitor is a substance that inhibits the growth of new blood vessels (angiogenesis). An inhibitor of angiogenesis prevents the formation of new blood vessels, thereby stopping or slowing the growth or spread of diseases or disorders such as tumors and neovascular pathologies. A vasculogenesis inhibitor is a substance that inhibits the process of blood vessel formation occurring by a *de novo* production of endothelial cells.

In preferred aspects, the nucleic acid molecule encoding for the herein provided modified VEGF-A is preferably at least 55% homologous/identical to the nucleic acid sequence as shown, inter alia, in SEQ ID NO: 1 or 7, wherein such a homologous nucleic acid sequence comprises the inventive modification(s) herein provided. It is herein understood that such nucleic acid sequences can also include orthologous/homologous/identical (and thus related) sequences. More preferably, the nucleic acid sequence encoding the herein provided modified VEGF-A is at least 56%, 57%, 58%, 59%, 60%, 65%, 70%, 75%, 80%, 85%, 90%, 91%, 92%, 93%, 94%, 95%, 96%, 97% or 98% homologous/identical to the nucleic acid sequence as shown in e.g., SEQ ID NO: 1 or 7, wherein the higher values of sequence identity are preferred.

More preferably, the inventive nucleic acid sequence encoding the herein provided modified VEGF-A is at least 60% homologous/identical to the nucleic acid sequence as shown in SEQ ID NO: 1 or 7. More preferably, the nucleic acid sequence encoding the herein provided modified VEGF-A is at least 70% homologous/identical to the nucleic acid sequence as shown in SEQ ID NO: 1 or 7. Even more preferably, the nucleic acid sequence encoding the herein provided modified VEGF-A is at least 80% homologous/identical to the nucleic acid sequence as shown in SEQ ID NO: 1 or 7. Most preferably, the nucleic acid sequence encoding the herein provided modified VEGF-A is at least 90% homologous/identical to the nucleic acid sequence as shown in SEQ ID NO: 1 or 7. The above defined orthologous/homologous/identical sequences can also be encompassed in longer or shorter isoforms, spliced variants and fusion transcripts so long as the herein described beneficial effect as an angiogenesis inhibitor is achieved. The term "orthologous protein" or "orthologous gene" as used herein refers to proteins and genes, respectively, in different species that are similar to each other because they originated from a common ancestor.

Hybridization assays for the characterization of orthologs or other related sequences of known nucleic acid sequences are well known in the art; see e.g. Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989). The term "hybridization" or "hybridizing", as used herein in connection with nucleic acids, relates to hybridizations under conditions of any degree of stringency. In general, hybridizations of nucleic acids, such as Southern or Northern hybridizations, can be performed under experimental conditions of various degrees of stringency. Usually a nucleic acid immobilized on a solid support such as a membrane is contacted with a liquid containing another, similar nucleic acid (called probe) under suitable buffer and temperature conditions in order to selectively allow the interaction of the probe with the immobilized nucleic acid, wherein the probe has a certain degree of sequence identity to the immobilized nucleic acid to be tested. Commonly the buffer used for the hybridization, in particular the washing steps after hybridization, is standard sodium citrate buffer (SSC; also referred to as saline sodium citrate buffer). A 20-fold concentrated SSC buffer contains 3 M NaCl and 0.3 M sodium citrate, adjusted to pH 7.0 using HCl, and is commercially available, e.g., from Sigma Aldrich. The Na+ concentration of a corresponding 20-fold SSC buffer is 3.3 M (i.e., 3.3 mol/L), and it is 1.65 M for a 10-fold SSC buffer, 0.825 M for a 5-fold SSC buffer, 0.33 M for a 2-fold SSC buffer, 0.165 M for a 1-fold SSC buffer, and 0.0165 M for a 0.1-fold SSC buffer. Formamide or sodium dodecyl sulfate (SDS) can be added to the SSC buffer to reduce unspecific binding of the probe. The stringency of the hybridization depends on the percentage of the nucleotides G and C present in the sequence of the probe (%G+C) and the hybridization conditions, particularly the temperature, the concentration of Na+ and the concentration of formamide or SDS (if present). In general, the higher the hybridization temperature and the lower the sodium (Na+) concentration, the higher will be the stringency. The stringency of the hybridization can thus be controlled by appropriately choosing the temperature for the hybridization, the concentration of the SSC buffer (and thereby the sodium concentration) and optionally the concentration of formamide (or SDS) added to the SSC buffer. If different concentrations of SSC buffer are used in different steps of the hybridization procedure, the concentrations of sodium and formamide in the most concentrated SSC buffer (which is typically the buffer used for the final washing step) are decisive.

Accordingly, at a given GC-content of the probe and at specific concentrations of sodium and formamide (if present) in the most concentrated buffer used for the washing after hybridization (typically the buffer used for the final washing step), the stringency of the hybridization can be controlled by adjusting the hybridization temperature to a specific number of degrees Celsius (e.g., 25°C or less) below the effective melting temperature (Tm) which can be calculated using the following formula (for DNA-DNA-hybridizations):

$$Tm = 81.5 + 16.6(\log M\, [Na^+]) + 0.41(\%G{+}C) - 0.72(\%\ formamide)$$

In the above formula, "Tm" is the temperature under which the sequence of the immobilized nucleic acid to be tested needs to match 100% of the sequence of the probe in order for both sequences to hybridize to each other; "log M [$Na^+$]" is the logarithm to base 10 ($\log_{10}$) of the concentration of sodium ($Na^+$) in mol/L in the buffer; "%G+C" is the percentage of the nucleotides G and C in the sequence of the probe (GC-content); and "% formamide" is the concentration of formamide in %(volume/volume) in the buffer. As is well known, the length of the probe to be determined constitutes further parameters of the hybridization conditions. The farther the hybridization temperature is below the Tm, the lower will be the stringency of the hybridization. In particular, for each 1.4°C the hybridization temperature is below the calculated Tm, the hybridization will still occur in the presence of 1% sequence mismatch, i.e., a mismatch of x% of the sequences of the probe and the immobilized nucleic acid to be tested will still lead to hybridization if the hybridization temperature is at least x • 1.4°C below the calculated Tm. For example, if the Tm is calculated to be 90°C and the hybridization experiment is conducted at 55°C (i.e., 35°C below the Tm), nucleic acid sequences matching at least 82.1% of the sequence of the probe will hybridize to the probe (i.e., 35°C/1.4°C = 25.0, meaning that 100% - 25.0% = 75.0%).

As used herein, hybridization under "stringent conditions" preferably means that the hybridization temperature is about 35°C or less below the Tm (calculated using the formula explained above), which corresponds to a minimum sequence identity of about 75.0% required for hybridization to occur (i.e., 100% - (35°C/1.4°C)%). More preferably, hybridization under stringent conditions means that the hybridization temperature is about 30°C or less below the Tm (corresponding to a minimum sequence identity of about 78.6% required for hybridization), even more preferably, hybridization under stringent conditions means that the hybridization temperature is about 25°C or less below the Tm (corresponding to a minimum sequence identity of about 82.1% required for hybridization), even more preferably, hybridization under stringent conditions means that the hybridization temperature is about 20°C or less below the Tm (corresponding to a minimum sequence identity of about 85.7% required for hybridization), even more preferably about 15°C or less below the Tm (corresponding to a minimum sequence identity of about 89.3% required for hybridization), even more preferably about 10°C or less below the Tm (corresponding to a minimum sequence identity of about 92.9% required for hybridization), even more preferably about 7°C or less below the Tm (corresponding to a minimum sequence identity of about 95.0% required for hybridization), yet even more preferably about 5°C or less below the Tm (corresponding to a minimum sequence identity of about 96.4% required for hybridization), and still more preferably about 3°C or less below the Tm (corresponding to a minimum sequence identity of about 97.9% required for hybridization). Conversely, hybridization under "non-stringent conditions" means that the hybridization temperature is below the above-defined temperature required for stringent hybridization. If not further specified, the conditions are preferably non-stringent. Said hybridization conditions may be established according to conventional protocols described, e.g., in Sambrook (2001) loc. cit.; Ausubel (1989) loc. cit., or Higgins and Hames (Eds.) "Nucleic acid hybridization, a practical approach" IRL Press Oxford, Washington DC, (1985). The setting of conditions is well within the skill of the artisan and can be determined according to protocols described in the art.

In accordance with the present invention, the terms "homology" or "percent homology" or "identical" or "percent identity" or "percentage identity" or "sequence identity" in the context of two or more nucleic acid sequences refers to two or more sequences or subsequences that are the same, or that have a specified percentage of nucleotides that are the same, when compared and aligned for maximum correspondence over the window of comparison (preferably over the full length), or over a designated region as measured using a sequence comparison algorithm as known in the art, or by manual alignment and visual inspection. Sequences having, for example, 70% to 90% or greater sequence identity may be considered to be substantially identical. Such a definition also applies to the complement of a test sequence. Preferably the described identity exists over a region that is at least about 15 to about 25 nucleotides in length, more preferably, over a region that is at least about 50 to about 100 nucleotides in length, even more preferably, over a region that is at least about 150 to about 350 nucleotides in length and most preferably, over the full length. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

Although the FASTDB algorithm typically does not consider internal non-matching deletions or additions in sequences, i.e., gaps, in its calculation, this can be corrected manually to avoid an overestimation of the % identity. CLUSTALW, however, does take sequence gaps into account in its identity calculations. Also available to those having skill in this art are the BLAST and BLAST 2.0 algorithms (Altschul, (1997) Nucl. Acids Res. 25:3389-3402; Altschul (1993) J. Mol. Evol. 36:290-300; Altschul (1990) J. Mol. Biol. 215:403-410). The BLASTN program for nucleic acid sequences uses as defaults a word length (W) of 11, an expectation (E) of 10, M=5, N=4, and a comparison of both strands. The BLOSUM62 scoring matrix (Henikoff (1989) PNAS 89:10915) uses alignments (B) of 50, expectation (E) of 10, M=5, N=4, and a comparison of both strands.

In order to determine whether a nucleotide in a nucleic acid sequence corresponds to a certain position in the nucleotide sequence of e.g. SEQ ID NO: 1 or 7, the skilled person can use means and methods well-known in the art, e.g., alignments, either manually or by using computer programs such as those mentioned herein, e.g., BLAST 2.0 as described above.

The explanations and definitions given herein above in respect of "homology/identity of nucleic acid sequences" apply, mutatis mutandis, to "amino acid sequences" of members of the modified VEGF-A polypeptide, in particular, the amino acid sequence as depicted in SEQ ID NO: 2 (Homo sapiens VEGF-A 121).

The modified VEGF-A polypeptides have of at least 55% homology/identity to a wild-type VEGF-A polypeptide having the amino acid sequence as, for example, depicted in SEQ ID NO: 2 or 8, wherein such sequences comprise the inventive modification(s) provided herein and function as an inhibitor of angiogenesis and/or vasculogenesis.
In preferred aspects, the provided modified VEGF-A polypeptides of the invention is preferably at least 55% homologous/identical to the amino acid sequence as shown in SEQ ID NO: 2 or 8. It is understood that such amino acid sequences can also include orthologous/homologous/identical (and thus related) sequences. More preferably, the amino acid sequence encoding the provided modified VEGF-A polypeptide is at least 56%, 57%, 58%, 59%, 60%, 65%, 70%, 75%, 80%, 85%, 90%, 91%, 92%, 93%, 94%, 95%, 96%, 97% or 98% homologous/identical to the amino acid sequence as shown SEQ ID NO: 2 or 8, wherein the higher values of sequence identify are preferred.

The herein provided modified VEGF-A polypeptide or the functional fragment thereof that functions as an angiogenesis and/or vasculogenesis inhibitor is selected from the group consisting of:

(a) a polypeptide encoded by a nucleic acid molecule given in SEQ ID NO: 1, wherein the nucleotides AAA at position 250 to 252 of SEQ ID NO: 1 are replaced by nucleotides encoding said hydrophobic amino acid, and/or wherein the nucleotides TTC at position 49 to 51 of SEQ ID NO: 1 are replaced by nucleotides encoding said hydrophobic amino acid;
(b) a polypeptide given in SEQ ID NO: 2, wherein the lysine residue corresponding to position 84 of SEQ ID NO: 2; and/or wherein the phenylalanine corresponding to position 17 of SEQ ID NO: 2 is replaced by said hydrophobic amino acid;
(c) a polypeptide encoded by a nucleic acid molecule hybridizing under stringent conditions to the complementary strand of a nucleic acid molecule encoding the polypeptide as defined in (a) or (b);
(d) a polypeptide having at least 55% identity to the polypeptide of any one of (a) to (c) and functioning as an inhibitor of angiogenesis; and
(e) a polypeptide that functions as an inhibitor of angiogenesis comprising an amino acid sequence encoded by a nucleic acid molecule being degenerate as a result of the genetic code to the nucleotide sequence of a nucleic acid molecule as defined in any one of (a), (c) and (d).

In certain aspects, the herein provided modified VEGF-A polypeptide or the functional fragment thereof that functions as an angiogenesis and/or vasculogenesis inhibitor is selected from the group consisting of:

(a) a polypeptide encoded by a nucleic acid molecule given in SEQ ID NO: 7, wherein the nucleotides AAG at position 250 to 252 of SEQ ID NO: 7 are replaced by nucleotides encoding said hydrophobic amino acid, and/or wherein the nucleotides TTC at position 49 to 51 of SEQ ID NO: 7 are replaced by nucleotides encoding said hydrophobic amino acid;

(b) a polypeptide given in SEQ ID NO: 8, wherein the lysine residue corresponding to position 84 of SEQ ID NO: 8; and/or wherein the phenylalanine corresponding to position 17 of SEQ ID NO: 8 is replaced by said hydrophobic amino acid;

(c) a polypeptide encoded by a nucleic acid molecule hybridizing under stringent conditions to the complementary strand of a nucleic acid molecule encoding the polypeptide as defined in (a) or (b);

(d) a polypeptide having at least 55% identity to the polypeptide of any one of (a) to (c) and functioning as an inhibitor of angiogenesis; and

(e) a polypeptide that functions as an inhibitor of angiogenesis comprising an amino acid sequence encoded by a nucleic acid molecule being degenerate as a result of the genetic code to the nucleotide sequence of a nucleic acid molecule as defined in any one of (a), (c) and (d).

Further, the invention relates to nucleic acid molecules encoding the modified VEGF-A polypeptides or the functional fragments thereof.

The terms "complement", "reverse complement" and "reverse sequence" referred to herein are described in the following example: For sequence 5'AGTGAAGT3', the complement is 3'TCACTTCA5', the reverse complement is 3'ACTTCACT5' and the reverse sequence is 5'TGAAGTGA3'.

In certain aspects, the modified VEGF-A or the functional fragment thereof that functions as an angiogenesis and/or vasculogenesis inhibitor is encoded by a nucleic acid molecule comprising:

(b) a nucleic acid molecule given in SEQ ID NO: 1,
wherein the nucleotides AAA at position 250 to 252 of SEQ ID NO: 1 are replaced by nucleotides encoding said hydrophobic amino acid, and/or wherein the nucleotides TTC at position 49 to 51 of SEQ ID NO: 1 are replaced by nucleotides encoding said hydrophobic amino acid;

(b) a nucleic acid molecule encoding a polypeptide given in SEQ ID NO: 2, wherein the lysine residue corresponding to position 84 of SEQ ID NO: 2; and/or wherein the phenylalanine corresponding to position 17 of SEQ ID NO: 2 is replaced by said hydrophobic amino acid;

(c) a nucleic acid molecule hybridizing under stringent conditions to the complementary strand of a nucleic acid molecule as defined in (a) or (b);

(d) a nucleic acid molecule having at least 55% identity to any one of the polypeptides referred to in (b) and functioning as an inhibitor of angiogenesis; and

(e) a nucleic acid molecule being degenerate as a result of the genetic code to the nucleotide sequence of a nucleic acid molecule as defined in any one of (a) to (d).

In certain aspects, the modified VEGF-A or the functional fragment thereof that functions as an angiogenesis and/or vasculogenesis inhibitor is encoded by a nucleic acid molecule comprising:

(b) a nucleic acid molecule given in SEQ ID NO: 7,
wherein the nucleotides AAG at position 250 to 252 of SEQ ID NO: 7 are replaced by nucleotides encoding said hydrophobic amino acid, and/or wherein the nucleotides TTC at position 49 to 51 of SEQ ID NO: 7 are replaced by nucleotides encoding said hydrophobic amino acid;

(b) a nucleic acid molecule encoding a polypeptide given in SEQ ID NO: 8, wherein the lysine residue corresponding to position 84 of SEQ ID NO: 8; and/or wherein the phenylalanine corresponding to position 17 of SEQ ID NO: 8 is replaced by said hydrophobic amino acid;

(c) a nucleic acid molecule hybridizing under stringent conditions to the complementary strand of a nucleic acid molecule as defined in (a) or (b);

(d) a nucleic acid molecule having at least 55% identity to any one of the polypeptides referred to in (b) and functioning as an inhibitor of angiogenesis; and

(e) a nucleic acid molecule being degenerate as a result of the genetic code to the nucleotide sequence of a nucleic acid molecule as defined in any one of (a) to (d).

In preferred aspects of the invention, the nucleic acid molecule encodes a modified VEGF-A polypeptide or the functional fragment thereof, wherein the modified VEGF-A polypeptide is selected from the group consisting of SEQ ID NO: 4, SEQ ID NO: 6, SEQ ID NO: 10, SEQ ID NO: 12, SEQ ID NO: 13, SEQ ID NO: 14, SEQ ID NO: 18, SEQ ID NO: 20, SEQ ID NO: 24, and SEQ ID NO: 26, and wherein the fragment functions as an angiogenesis and/or vasculogenesis inhibitor. In more preferred aspects, the nucleic acid molecule encodes a modified vascular endothelial growth factor A (VEGF-A) or the functional fragment thereof, wherein the modified VEGF-A polypeptide is SEQ ID NO: 4 or SEQ ID NO: 6, and wherein the fragment functions as an angiogenesis and/or vasculogenesis inhibitor.

Furthermore, in preferred aspects of the invention, the nucleic acid molecule comprises a nucleic acid sequence or a fragment thereof, wherein the nucleic acid sequence is selected from the group consisting of SEQ ID NO: 3, SEQ ID NO: 5, SEQ ID NO: 9, SEQ ID NO: 11, SEQ ID NO: 17, SEQ ID NO: 19, SEQ ID NO: 23, and SEQ ID NO: 25. In more preferred aspects, the nucleic acid nucleic acid sequence is SEQ ID NO: 3 or SEQ ID NO: 5.

As used herein, the term "functional fragment" relates to the modified VEGF-A polypeptide or a nucleic acid encoding such a polypeptide, wherein said polypeptide has a deletion of amino acid stretches, such as 1, 2, 3, 4, 5, 6, 7, 8, 9, 10, 11 or more amino acids. The size of the functional fragment of the herein provided polypeptide is not limited as long as it has the inventive properties described herein, e.g., it is an inhibitor of angiogenesis and/or vasculogenesis, and comprises the inventive modifications described herein, i.e., has a hydrophobic amino acid in place of the lysine at position 84 and/or has a hydrophobic amino acid in place of the phenylalanine at position 17.

As used herein, a "hydrophobic amino acid" is a hydrophobic amino acid residue. Such a hydrophobic amino acid can be an amino acid selected from the group consisting of alanine (A/Ala), valine (V/Val), isoleucine (I/Ile), leucine (L/Leu) and methionine (M/Met). In most preferred aspects, the hydrophobic amino acid is alanine. It is herein understood, that the phenylalanine at the position 17 of SEQ ID NO: 2 or an amino acid that corresponds to the phenylalanine at position 17 of SEQ ID NO: 2 is substituted by a hydrophobic amino acid, wherein this hydrophobic amino acid is a non-aromatic amino acid. Aromatic amino acids include an aromatic ring such as tyrosine or tryptophan. Accordingly, the modified VEGF-A of the present invention has not an aromatic amino at position 17. The herein described modification/replacement of lysine on position 84 and/or phenylalanine on position 17 by a hydrophobic amino acid renders the modified VEGF-A as angiogenesis and/or vasculogenesis inhibitor. The skilled person understands that in principle any other amino acid can be used that shows the herein described beneficial properties as documented in the appended examples. It might thus also be envisaged that glycine can be used instead of the hydrophobic amino acid. Furthermore, it is thus envisaged herein that non-proteinogenic or a non-standard $\alpha$-amino acid can be used to achieve such beneficial effects.

In the appended examples, it was surprisingly shown that the replacement of the amino acid residue lysine by alanine at position 84 of wild-type VEGF-A as shown in SEQ ID NO: 2 renders the modified VEGF-A polypeptide as an angiogenesis inhibitor. It was also surprisingly shown that the replacement of the amino acid phenylalanine by alanine at position 17 of wild-type VEGF-A as shown in SEQ ID NO: 2 renders the modified VEGF-A polypeptide as an angiogenesis inhibitor. Therefore, in a most preferred aspect of the invention, the hydrophobic amino acid is alanine.

In preferred aspects, the invention relates to a modified vascular endothelial growth factor A (VEGF-A) polypeptide, wherein said modified vascular endothelial growth factor A (VEGF-A) comprises alanine in place of the lysine at the position corresponding to position 84 of the wild-type VEGF-A as shown in SEQ ID NO: 2. An exemplary sequence is given SEQ ID NO: 4 of VEGF-A 121 that comprises an alanine at position 84.

In further preferred aspects, the invention relates to a modified vascular endothelial growth factor A (VEGF-A) polypeptide, wherein said modified vascular endothelial growth factor A (VEGF-A) comprises alanine in place of the phenylalanine at the position corresponding to position 17 of the wild-type VEGF-A as shown in SEQ ID NO: 2. An exemplary sequence is given in SEQ ID NO: 6 of VEGF-A 121 that comprises an alanine at position 17.

In certain aspects of the invention, the invention relates to a modified vascular endothelial growth factor A (VEGF-A) polypeptide, wherein said modified vascular endothelial growth factor A (VEGF-A) comprises alanine in place of the lysine at the position corresponding to position 84 of the wild-type VEGF-A as shown in SEQ ID NO: 2, and wherein said modified vascular endothelial growth factor A (VEGF-A) comprises alanine in place of the phenylalanine at the position corresponding to position 17 of the wild-type VEGF-A as shown in SEQ ID NO: 2. An exemplary amino acid sequence is given in SEQ ID NO: 13 or 14 of VEGF-A 121 that comprises an alanine at position 17 and 84.

As used herein, the term "nucleotides encoding the hydrophobic amino acid" refers to a codon encoding a hydrophobic amino acid. A codon encoding a hydrophobic amino acid means in accordance with the present invention, a codon, which according to the standard genetic code (as illustrated, inter alia, in Stryer (1995), "Biochemistry", Freeman and Company, ISBN 0-7167-2009-4) codes for a "hydrophobic amino acid". In certain aspects, A is encoded by the codon selected from the group consisting of GCC, GCA, GCG and GCT. In particular preferred aspects, A is encoded by the codon GCC or GCA. The degeneracy of the genetic code permits the same amino acid sequence to be encoded and translated in many different ways. For example, leucine, serine and arginine are each encoded by six different codons, while alanine, valine, proline, threonine and glycine are each encoded by four different codons. However, the frequency of use of such synonymous codons varies from genome to genome among eukaryotes and prokaryotes. For example, synonymous codon-choice patterns among mammals are very similar, while evolutionarily distant organisms such as yeast (S. cerevisiae), bacteria (such as E. coli) and insects (such as D. melanogaster) reveal a clearly different pattern of genomic codon use frequencies. Therefore, codon optimized genes can be used in the present invention. The design of codon optimized genes should take into account a variety of factors, including the frequency of codon usage in an organism, nearest neighbor frequencies, RNA stability, the potential for secondary structure formation, the route of synthesis and the intended future DNA manipulations of that gene. It is contemplated herein that codon optimized nucleic acid sequences can be employed.

In preferred aspects, the herein provided modified VEGF-A polypeptide or the functional fragment thereof that functions as an angiogenesis and/or vasculogenesis inhibitor is selected from the group consisting of:

(a) a polypeptide encoded by a nucleic acid molecule given in SEQ ID NO: 1,
wherein the nucleotides AAA at position 250 to 252 of SEQ ID NO: 1 are replaced by nucleotides encoding alanine, and/or
wherein the nucleotides TTC at position 49 to 51 of SEQ ID NO: 1 are replaced by nucleotides encoding alanine;
(b) a polypeptide given in SEQ ID NO: 2, wherein the lysine residue corresponding to position 84 of SEQ ID NO: 2; and/or wherein the phenylalanine corresponding to position 17 of SEQ ID NO: 2 is replaced by alanine;
(c) a polypeptide encoded by a nucleic acid molecule hybridizing under stringent conditions to the complementary strand of a nucleic acid molecule encoding the polypeptide as defined in (a) or (b);
(d) a polypeptide having at least 55% identity to the polypeptide of any one of (a) to (c) and functioning as an inhibitor of angiogenesis; and
(e) a polypeptide that functions as an inhibitor of angiogenesis comprising an amino acid sequence encoded by a nucleic acid molecule being degenerate as a result of the genetic code to the nucleotide sequence of a nucleic acid molecule as defined in any one of (a), (c) and (d).

In preferred aspects, the herein provided modified VEGF-A polypeptide or the functional fragment thereof that functions as an angiogenesis and/or vasculogenesis inhibitor is selected from the group consisting of:

(a) a polypeptide encoded by a nucleic acid molecule given in SEQ ID NO: 7,
wherein the nucleotides AAG at position 250 to 252 of SEQ ID NO: 7 are replaced by nucleotides encoding alanine, and/or
wherein the nucleotides TTC at position 49 to 51 of SEQ ID NO: 7 are replaced by nucleotides encoding alanine;
(b) a polypeptide given in SEQ ID NO: 8, wherein the lysine residue corresponding to position 84 of SEQ ID NO: 8; and/or wherein the phenylalanine corresponding to position 17 of SEQ ID NO: 8 is replaced by alanine;
(c) a polypeptide encoded by a nucleic acid molecule hybridizing under stringent conditions to the complementary strand of a nucleic acid molecule encoding the polypeptide as defined in (a) or (b);
(d) a polypeptide having at least 55% identity to the polypeptide of any one of (a) to (c) and functioning as an inhibitor of angiogenesis; and
(e) a polypeptide that functions as an inhibitor of angiogenesis comprising an amino acid sequence encoded by a nucleic acid molecule being degenerate as a result of the genetic code to the nucleotide sequence of a nucleic acid molecule as defined in any one of (a), (c) and (d).

The appended examples demonstrate that the beneficial effect of the herein provided modified VEGF-A occurs in case the heparin and/or Nrp-1 binding domain is absent in the VEGF-A isoforms provided herein, such as found in the VEGF-A 121 isoform. In preferred aspects, the herein provided modified VEGF-A polypeptide lacks the heparin binding domain and/or the Nrp-1 binding domain. In the appended examples, it is documented that the herein described modifications in VEGF-A isoforms lacking the heparin binding domain, e.g. VEGF-A 121 isoform, render the polypeptide to an angiogenesis and/or vasculogenesis inhibitor. The heparin binding/Nrp-1 binding domain is a C-terminal sub-domain in the longer VEGF-A-isoforms such as the VEGF-A 165 isoform. The heparin binding domain can be removed from VEGF-A 165 by alternative splicing or by plasmin cleavage. As described above, exons 6 and 7 confer heparin-binding affinity (loc cit. Chengzhong et al.). Therefore, in certain aspects, the herein provided modified VEGF-A nucleic acid molecule encoding the modified VEGF-A polypeptide lacks the exons 6 and 7.

Structural coordinates of the heparin/Nrp-1 binding domain are determined, e.g., in the crystal structure of VEGF-A (Muller et al., 1997, Structure 5:1325-1338; Stauffer et al., 2002, Journal of Biomolecular NMR, 23:57-61). The heparin/Nrp-1 binding domain comprises two sub-domains. Each of the sub-domains contain a small two-stranded anti-parallel $\beta$-sheet and two disulfide bonds. The C-terminal sub-domain also contains a short $\alpha$-helix that packs against the $\beta$-sheet (loc. cit Stauffer et al.). Within this domain, NRP1 binding is mediated by E154 and R165 and heparin binding is mediated by R149, R150, K151, K166, R171, R175, R182. An exemplary "Heparin binding domain/Nrp-1 binding domain" has an amino acid sequence from position 141 to 184 of human VEGF-A 165 as given in SEQ ID NO: 29. An exemplary human amino acid sequence of a Heparin binding domain is given in SEQ ID NO: 27. In zebrafish VEGF-A 165, an exemplary "Heparin binding domain/Nrp-1 binding domain" has an amino acid sequence from position 139 to 182 of zebrafish VEGF-A 165 as given in SEQ ID NO: 30. An exemplary zebrafish heparin/Nrp-1 binding domain is given in SEQ ID NO: 28.

In certain aspects, the invention relates to a modified vascular endothelial growth factor A (VEGF-A) polypeptide, wherein said modified vascular endothelial growth factor A (VEGF-A) lacks the heparin binding domain and/or the Nrp-1 binding domain and wherein said modified vascular endothelial growth factor A (VEGF-A) comprises a hydrophobic amino acid in place of the lysine at the position corresponding to position 84 of the wild-type VEGF-A as shown in SEQ ID NO: 2.

In preferred aspects, the invention relates to a modified vascular endothelial growth factor A (VEGF-A) polypeptide, wherein said modified vascular endothelial growth factor A (VEGF-A) lacks the heparin binding domain and/or the Nrp-1 binding domain and wherein said modified vascular endothelial growth factor A (VEGF-A) comprises alanine in place of the lysine at the position corresponding to position 84 of the wild-type VEGF-A as shown in SEQ ID NO: 2.

In certain aspects, the invention relates to a modified vascular endothelial growth factor A (VEGF-A) polypeptide, wherein said modified vascular endothelial growth factor A (VEGF-A) lacks the heparin binding domain and/or the Nrp-1 binding domain, and wherein said modified vascular endothelial growth factor A (VEGF-A) comprises a hydrophobic amino acid in place of the phenylalanine at the position corresponding to position 17 of the wild-type VEGF-A as shown in SEQ ID NO: 2.

In preferred aspects, the invention relates to a modified vascular endothelial growth factor A (VEGF-A) polypeptide, wherein said modified vascular endothelial growth factor A (VEGF-A) lacks the heparin binding domain and/or the Nrp-1 binding domain, and wherein said modified vascular endothelial growth factor A (VEGF-A) comprises alanine in place of the phenylalanine at the position corresponding to position 17 of the wild-type VEGF-A as shown in SEQ ID NO: 2.

In certain aspects, the invention relates to a modified vascular endothelial growth factor A (VEGF-A) polypeptide, wherein said modified vascular endothelial growth factor A (VEGF-A) lacks the heparin binding domain and/or the Nrp-1 binding domain as given in SEQ ID NO: 27, and wherein said modified vascular endothelial growth factor A (VEGF-A) comprises a hydrophobic amino acid in place of the lysine at the position corresponding to position 84 of the wild-type VEGF-A as shown in SEQ ID NO: 2.

In certain aspects, the invention relates to a modified vascular endothelial growth factor A (VEGF-A) polypeptide, wherein said modified vascular endothelial growth factor A (VEGF-A) lacks the heparin binding domain and/or the Nrp-1 binding domain as given in SEQ ID NO: 27, and wherein said modified vascular endothelial growth factor A (VEGF-A) comprises alanine in place of the lysine at the position corresponding to position 84 of the wild-type VEGF-A as shown in SEQ ID NO: 2.

In certain aspects, the invention relates to a modified vascular endothelial growth factor A (VEGF-A) polypeptide, wherein said modified vascular endothelial growth factor A (VEGF-A) lacks the heparin/Nrp-1 binding domain given in SEQ ID NO: 27 and wherein said modified vascular endothelial growth factor A (VEGF-A) comprises a hydrophobic amino acid in place of the phenylalanine at the position corresponding to position 17 of the wild-type VEGF-A as shown in SEQ ID NO: 2.

In certain aspects, the invention relates to a modified vascular endothelial growth factor A (VEGF-A) polypeptide, wherein said modified vascular endothelial growth factor A (VEGF-A) lacks the heparin/Nrp-1 binding domain given in SEQ ID NO: 27 and wherein said modified vascular endothelial growth factor A (VEGF-A) comprises alanine in place of the phenylalanine at the position corresponding to position 17 of the wild-type VEGF-A as shown in SEQ ID NO: 2.

In certain aspects, the invention relates to a modified vascular endothelial growth factor A (VEGF-A) polypeptide, wherein said modified vascular endothelial growth factor A (VEGF-A) lacks the heparin/Nrp-1 binding domain, wherein said modified vascular endothelial growth factor A (VEGF-A) comprises a hydrophobic amino acid in place of the lysine at the position corresponding to position 84 of the wild-type VEGF-A as shown in SEQ ID NO: 2, and wherein said modified vascular endothelial growth factor A (VEGF-A) comprises a hydrophobic amino acid in place of the phenylalanine at the position corresponding to position 17 of the wild-type VEGF-A as shown in SEQ ID NO: 2.

In certain aspects, the invention relates to a modified vascular endothelial growth factor A (VEGF-A) polypeptide, wherein said modified vascular endothelial growth factor A (VEGF-A) lacks the heparin/Nrp-1 binding domain, wherein said modified vascular endothelial growth factor A (VEGF-A) comprises alanine in place of the lysine at the position corresponding to position 84 of the wild-type VEGF-A as shown in SEQ ID NO: 2, and wherein said modified vascular endothelial growth factor A (VEGF-A) comprises alanine in place of the phenylalanine at the position corresponding to position 17 of the wild-type VEGF-A as shown in SEQ ID NO: 2.

In certain aspects, the invention relates to a modified vascular endothelial growth factor A (VEGF-A) polypeptide, wherein said modified vascular endothelial growth factor (VEGF-A) lacks the heparin/Nrp-1 binding domain given in SEQ ID NO: 27, wherein said modified vascular endothelial growth factor A (VEGF-A) comprises a hydrophobic amino acid in place of the lysine at the position corresponding to position 84 of the wild-type VEGF-A as shown in SEQ ID NO: 2, and wherein said modified vascular endothelial growth factor A (VEGF-A) comprises a hydrophobic amino acid in place of the phenylalanine at the position corresponding to position 17 of the wild-type VEGF-A as shown in SEQ ID NO: 2.

In certain aspects, the invention relates to a modified vascular endothelial growth factor A (VEGF-A) polypeptide, wherein said modified vascular endothelial growth factor A (VEGF-A) lacks the heparin/Nrp-1 binding domain, wherein said modified vascular endothelial growth factor A (VEGF-A) comprises a hydrophobic amino acid in place of the lysine at the position corresponding to position 84 of the wild-type VEGF-A as shown in SEQ ID NO: 8, and/or wherein said modified vascular endothelial growth factor A (VEGF-A) comprises a hydrophobic amino acid in place of the phenylalanine at the position corresponding to position 17 of the wild-type VEGF-A as shown in SEQ ID NO: 8, and wherein preferably the hydrophobic amino acid is alanine.

In certain aspects, the modified vascular endothelial growth factor A (VEGF-A) polypeptide comprises the amino acids from position 1 to position 110, preferably the amino acids from position 1 to position 111, more preferably the amino acids from position 1 to position 112, more preferably the amino acids from position 1 to position 113, more preferably the amino acids from position 1 to position 114, or most preferably the amino acids from position 1 to position 115 as given in SEQ ID NO: 2, wherein said modified VEGF-A comprises a hydrophobic amino acid in place of the lysine at the position corresponding to position 84 of the wild-type VEGF-A as shown in SEQ ID NO: 2, and/or wherein said modified VEGF-A comprises a hydrophobic amino acid in place of the phenylalanine at the position corresponding to position 17 of the wild-type VEGF-A as shown in SEQ ID NO: 2, and optionally wherein said modified VEGF-A polypeptide comprises the amino acids from position 116 to position 121 as given in SEQ ID NO: 2.

In preferred aspects, the nucleic acid molecule encoding said modified VEGF-A comprises at least exons 1, 2, 3, 4, 5 and 8 of the VEGF-A gene. Furthermore, the modified VEGF-A lacks exon 7 conferring the heparin and/or Nrp-1 binding. In preferred aspects, the modified VEGF-A lacks exons 6 and 7. For example, the nucleic acid molecule encoding VEGF-A 165 comprises exons 1, 2, 3, 4, 5, 7 and 8. The nucleic acid molecule encoding VEGF-A 121 comprises exons 1, 2, 3, 4, 5 and 8. Thus, VEGF-A 121 lacks the heparin binding domain and/or Nrp-1 binding domain.

As used herein, "VEGF-A 121" or "VEGFA121" refers to the isoform 121 of VEGF-A that is encoded by the exons 1, 2, 3, 4, 5 and 8 of the VEGF-A gene. The VEGF-A 121 isoform can also be encoded by the exons 1, 2, 3, 4, 5 and 8a or 8b of the VEGF-A gene. In general, the VEGF-A 121 isoforms are well known in the prior art e.g. (UniProtKB: P15692-9).When the signal peptide is cleaved off, the VEGF-A 121 isoform has 121 amino acids. Therefore, the active polypeptide within a cell has no signal peptide. "VEGF-A 165" refers to the isoform 165 of VEGF-A, which is encoded by exons 1, 2, 3, 4, 5, 7 and 8.

As described above and documented in the appended examples, the replacement of lysine at the position 84 or phenylalanine at the position 17 of SEQ ID NO: 2 by the hydrophobic amino acid in a VEGF-A isoform lacking the heparin and/or the Nrp-1 binding domain, e.g., VEGF-A 121, confers the herein anti-angiogenic and/or anti-vasculogenic properties. Therefore, in particular preferred embodiments, the herein provided modified VEGF-A is the VEGF-A 121 isoform. An exemplary amino acid sequence of a human wild-type VEGF-A 121 is given in SEQ ID NO: 2. A corresponding nucleic acid molecule encoding the human wild-type VEGF-A 121 is given in SEQ ID NO: 1. An exemplary amino acid sequence of zebrafish wild-type VEGF-A-121 is given in SEQ ID NO: 8. A corresponding nucleic acid molecule encoding the zebrafish wild-type VEGF-A-121 is given in SEQ ID NO: 7.

In particular preferred embodiments, the invention relates to a modified vascular endothelial growth factor A (VEGF-A) polypeptide, wherein said vascular endothelial growth factor A (VEGF-A) is the VEGF-A 121 isoform and wherein said modified vascular endothelial growth factor A (VEGF-A) comprises a hydrophobic amino acid in place of the lysine at the position corresponding to position 84 of the wild-type VEGF-A as shown in SEQ ID NO: 2.

In further particular preferred embodiments, the invention relates to a modified vascular endothelial growth factor A (VEGF-A) polypeptide, wherein said vascular endothelial growth factor A (VEGF-A) is the VEGF-A 121 isoform and wherein said modified vascular endothelial growth factor A (VEGF-A) comprises a hydrophobic amino acid in place of the phenylalanine at the position corresponding to position 17 of the wild-type VEGF-A as shown in SEQ ID NO: 2.

In certain aspects, the invention relates to a modified vascular endothelial growth factor A (VEGF-A) polypeptide, wherein said vascular endothelial growth factor A (VEGF-A) is the VEGF-A 121 isoform, wherein said modified vascular endothelial growth factor A (VEGF-A) comprises a hydrophobic amino acid in place of the lysine at the position corresponding to position 84 of the wild-type VEGF-A as shown in SEQ ID NO: 2, and wherein said modified vascular endothelial growth factor A (VEGF-A) comprises a hydrophobic amino acid in place of the phenylalanine at the position corresponding to position 17 of the wild-type VEGF-A as shown in SEQ ID NO: 2.

In most preferred embodiments, the invention relates to a modified vascular endothelial growth factor A (VEGF-A) polypeptide, wherein said vascular endothelial growth factor A (VEGF-A) is the VEGF-A 121 isoform and wherein said modified vascular endothelial growth factor A (VEGF-A) comprises alanine in place of the lysine at the position corresponding to position 84 of the wild-type VEGF-A as shown in SEQ ID NO: 2.

An exemplary amino acid sequence of the modified VEGF-A 121 isoform comprising an alanine in place of the lysine at the position corresponding to position 84 of the wild-type VEGF-A as shown in SEQ ID NO: 2 is given in SEQ ID NO: 4. Exemplary nucleic acid sequences encoding an amino acid sequence of the modified VEGF-A 121 isoform comprising an alanine in place of the lysine at the position corresponding to position 84 of the wild-type VEGF-A as shown in SEQ ID NO: 2 is given in SEQ ID NO: 3 or 9.

In most preferred embodiments, the invention relates to a modified vascular endothelial growth factor A (VEGF-A) polypeptide, wherein said vascular endothelial growth factor A (VEGF-A) is the VEGF-A-121 isoform and wherein said modified vascular endothelial growth factor A (VEGF-A) comprises alanine in place of the phenylalanine at the position that corresponding to position 17 of the wild-type VEGF-A as shown in SEQ ID NO: 2.

An exemplary amino acid sequence of the modified VEGF-A 121 isoform comprising an alanine in place of the phenylalanine at the position corresponding to position 17 of the wild-type VEGF-A as shown in SEQ ID NO: 2 is given in SEQ ID NO: 6. Exemplary nucleic acid sequences encoding an amino acid sequence of the modified VEGF-A 121 isoform comprising an alanine in place of the phenylalanine at the position corresponding to position 17 of the wild-type VEGF-A as shown in SEQ ID NO: 2 is given in SEQ ID NO: 5 or 11.

In most preferred embodiments, the invention relates to a modified vascular endothelial growth factor A (VEGF-A) polypeptide, wherein said vascular endothelial growth factor A (VEGF-A) is the VEGF-A 121 isoform and wherein said modified vascular endothelial growth factor A (VEGF-A) comprises alanine in place of the lysine at the position corresponding to position 84 of the wild-type VEGF-A as shown in SEQ ID NO: 8.

An exemplary amino acid sequence of the modified VEGF-A 121 isoform comprising an alanine in place of the lysine at the position corresponding to position 84 of the wild-type VEGF-A as shown in SEQ ID NO: 8 is given in SEQ ID NO: 10.

In most preferred embodiments, the invention relates to a modified vascular endothelial growth factor A (VEGF-A) polypeptide, wherein said vascular endothelial growth factor A (VEGF-A) is the VEGF-A 121 isoform and wherein said modified vascular endothelial growth factor A (VEGF-A) comprises alanine in place of the phenylalanine at the position corresponding to position 17 of the wild-type VEGF-A as shown in SEQ ID NO: 8.

An exemplary amino acid sequence of the modified VEGF-A 121 isoform comprising an alanine in place of the phenylalanine at the position corresponding to position 17 of the wild-type VEGF-A as shown in SEQ ID NO: 8 is given in SEQ ID NO: 12.

In certain aspects, the invention relates to a modified vascular endothelial growth factor A (VEGF-A) polypeptide, wherein said vascular endothelial growth factor A (VEGF-A) is the VEGF-A-121 isoform, wherein said modified vascular endothelial growth factor A (VEGF-A) comprises alanine in place of the lysine at the position corresponding to position 84 of the wild-type VEGF-A as shown in SEQ ID NO: 2, and wherein said modified vascular endothelial growth factor A (VEGF-A) comprises alanine in place of the phenylalanine at the position corresponding to position 17 of the wild-type VEGF-A as shown in SEQ ID NO: 2. An exemplary amino acid sequence of the modified VEGF-A 121 isoform comprising alanine in place of the lysine at the position corresponding to position 84 of the wild-type VEGF-A as shown in SEQ ID NO: 2 and alanine in place of the phenylalanine at the position corresponding to position 17 of the wild-type VEGF-A as shown in SEQ ID NO: 2 is given in SEQ ID NO: 13.

In certain aspects, the invention relates to a modified vascular endothelial growth factor A (VEGF-A) polypeptide, wherein said vascular endothelial growth factor A (VEGF-A) is the VEGF-A 121 isoform, wherein said modified vascular endothelial growth factor A (VEGF-A) comprises alanine in place of the lysine at the position corresponding to position 84 of the wild-type VEGF-A as shown in SEQ ID NO: 8, and wherein said modified vascular endothelial growth factor A (VEGF-A) comprises alanine in place of the phenylalanine at the position corresponding to position 17 of the wild-type VEGF-A as shown in SEQ ID NO: 8. An exemplary amino acid sequence of the modified VEGF-A 121 isoform comprising alanine in place of the lysine at the position corresponding to position 84 of the wild-type VEGF-A as shown in SEQ ID NO: 8 and alanine in place of the phenylalanine at the position corresponding to position 17 of the wild-type VEGF-A as shown in SEQ ID NO: 8 is given in SEQ ID NO: 14.

In certain aspects, the polypeptides of the present invention further comprises at least one additional mutation selected from the group consisting of amino acid substitution(s), addition(s), deletions(s) and duplication(s). Further, the invention relates to the polypeptide comprising the modified VEGF-A, wherein said VEGF-A comprises said hydrophobic amino acid at position 84 and/or 17 of SEQ ID NO: 2 and comprises at least one additional mutation selected from the group consisting of amino acid substitution(s), addition(s), deletions(s), inversion(s) and duplication(s). It also envisaged herein that the herein provided modified polypeptide comprises deletions of amino acid stretches, such as 1, 2, 3, 4, 5, 6, 7, 8, 9, 10 or 11 amino acids. In other words, the herein provided polypeptide can be a functional fragment of the herein provided inventive polypeptide. The size of the functional fragment of the herein provided polypeptide is not limited as long as it has the inventive properties described herein and comprises the inventive modifications described herein, i.e., has a hydrophobic amino acid in place of the lysine at position 84 and/or has a hydrophobic amino acid in place of the phenylalanine at position 17.

As a further embodiment, the inventive amino acid modification(s) can also be included in the VEGF-A 111 isoform (uniprot P15692-10) as given in SEQ ID NO: 31. Therefore, the invention also relates to a modified vascular endothelial growth factor A (VEGF-A) polypeptide, wherein said VEGF-A is the VEGF-A 111 isoform, and wherein said modified VEGF-A comprises a hydrophobic amino acid in place of the lysine at the position corresponding to position 110 of the wild-type VEGF-A as shown in SEQ ID NO: 31, and/or wherein said modified VEGF-A comprises a hydrophobic amino acid in place of the phenylalanine at the position corresponding to position 43 of the wild-type VEGF-A as shown in SEQ ID NO: 31.

In the appended examples, it was surprisingly shown that already a single amino acid residue substitution results in the strong anti-vasculogenic and/or anti-angiogenic effects.

Therefore, the present invention also relates to a modified VEGF-A polypeptide or a fragment thereof, wherein the fragment thereof functions as angiogenesis inhibitors, wherein said modified VEGF-A corresponds to a wild-type VEGF-A or a fragment thereof that has solely a replacement of the lysine at the position corresponding to position 84 of the wild-type VEGF-A as shown in SEQ ID NO: 2, and/or a replacement of the phenylalanine at the position corresponding to position 17 of the wild-type VEGF-A as shown in SEQ ID NO: 2 by a hydrophobic amino acid residue, such as alanine. In these aspects, the modified VEGF-A has a hydrophobic amino acid at the position 17 and/or at the position 84 of the wild type VEGF-A as shown in SEQ ID NO: 2, or a position that corresponds thereto, wherein the other amino acids, i.e., not phenylalanine at position 17 or lysine at position 84, correspond to the amino acids as found in the wild-type VEGF-A sequence.
In preferred aspects, the present invention relates to a modified VEGF-A polypeptide or a fragment thereof, wherein the fragment thereof functions as angiogenesis inhibitor, wherein the modified VEGF-A is the VEGF-A 121 isoform, wherein said modified VEGF-A corresponds to a wild-type VEGF-A or a fragment thereof that has solely a replacement of the lysine at the position corresponding to position 84 of the wild-type VEGF-A as shown in SEQ ID NO: 2, and/or a replacement of the phenylalanine at the position corresponding to position 17 of the wild-type VEGF-A as shown in SEQ ID NO: 2 by a hydrophobic amino acid residue, such as alanine. Such exemplary modified VEGF-A polypeptides are given in SEQ ID NOs: 4 and 10 (VEGF-A 121 K84A); SEQ ID NOs: 6 and 12 (VEGF-A 121 F17A); or SEQ ID NOs: 13 and 14 (VEGF-A 121 K84A F17A).

In such preferred aspects of the invention, the modified VEGF-A comprises an amino acid sequence that is selected from the group consisting of SEQ ID NO: 4, SEQ ID NO: 6, SEQ ID NO: 10, SEQ ID NO: 12, SEQ ID NO: 13, SEQ ID NO: 14, SEQ ID NO: 18, SEQ ID NO: 20, SEQ ID NO: 24, and SEQ ID NO: 26. The invention further relates to a functional fragment of the modified VEGF-A and wherein the fragment functions as an angiogenesis and/or vasculogenesis inhibitor. In more preferred aspects of the invention, the modified VEGF-A is selected from the group consisting of SEQ ID NO: 4, SEQ ID NO: 6, SEQ ID NO: 10, SEQ ID NO: 12, SEQ ID NO: 13, SEQ ID NO: 14, SEQ ID NO: 18, SEQ ID NO: 20, SEQ ID NO: 24, and SEQ ID NO: 26. In more preferred aspects of the invention, the modified VEGF-A is selected from the group consisting of SEQ ID NO: 4, SEQ ID NO: 6, SEQ ID NO: 10, SEQ ID NO: 12, SEQ ID NO: 13 and SEQ ID NO: 14. In more preferred aspects, said modified VEGF-A comprises an amino acid sequence that is SEQ ID NO: 4 or SEQ ID NO: 6 or a functional fragment thereof, and wherein optionally the fragment functions as an angiogenesis and/or vasculogenesis inhibitor. In most preferred aspects, said modified VEGF-A is SEQ ID NO: 4 or SEQ ID NO: 6.

VEGFR-2 receptor activation is promoted by dimerization of the VEGF-2 receptor. The VEGF-A polypeptide forms a dimer. It is herein understood that a "dimer" is an oligomer consisting of two structural similar monomers joined by bonds that can be either weak or strong, i.e., intermolecular or covalent. The two monomers that form the dimer can be comprised in the same fusion protein. The present invention relates to a fusion protein that comprises two modified VEGF-A polypeptides described herein. In certain aspects, the fusion protein comprises a first modified vascular endothelial growth factor A (VEGF-A) and a second modified vascular endothelial growth factor A (VEGF-A), wherein said first modified VEGF-A comprises a hydrophobic amino acid in place of:

the lysine at the position corresponding to position 84 of the wild-type VEGF-A as shown in SEQ ID NO: 2; and/or

the phenylalanine at the position corresponding to position 17 of the wild-type VEGF-A as shown in SEQ ID NO: 2,

wherein said second modified VEGF-A comprises a hydrophobic amino acid in place of:

the lysine at the position corresponding to position 84 of the wild-type VEGF-A as shown in SEQ ID NO: 2; and/or

the phenylalanine at the position corresponding to position 17 of the wild-type VEGF-A as shown in SEQ ID NO: 2.

The fusion protein as described herein can be a heterologous protein conjugated/linked together via amino acid linkers, e.g., serine-glycine linkers. Such linkers are known in the art and can be for example short peptide sequences that occur between protein domains. The linkers are often composed of flexible residues like glycine and serine so that the adjacent protein domains are free to move relative to one another. Longer linkers are used when it is necessary to ensure that two adjacent domains do not sterically interfere with one another. Non-peptide bonds are also envisaged herein. Such non-peptide bonds may include disulfide bonds, e.g. between Cys side chains, thioether bonds or non-peptide covalent bonds induced by chemical cross-linkers, such as disuccinimidyl suberate (DSS) or sulfosuccinimidyl 4-[p-maleimidophenyl] butyrate (Sulfo-SMPB), metal-chelating/complexing groups, as well as non-covalent protein-protein interactions. These are merely embodiments of the present invention and it is evident for the skilled artisan that modifications can easily be made within the fusion proteins and used without deferring from the gist of the present invention.

It is also envisaged herein that the stability of the modified VEGF-A can be optimized by adding, for example, immunoglobulin-like domains and to simultaneously enhance pharmacokinetic properties like prolonged half-life in serum and protection from proteolytic digestion by proteases. Moreover, stability of the formats can be enhanced by optimizing the production. Since linker sequences that are utilized to covalently join domains often leads to aggregates, production lines have been established that first produce two or three polypeptides that can be easily reassembled in order to generate a functional drug. Such techniques utilize directed disulphide-bridges or cross-linking reagents to covalently join two different polypeptides. Other techniques make use of hetero- or homo-dimerization domains like leucine-zipper domains, Fc-domains and others like knob into hole technologies (see, for example, WO 2007/062466).

The present invention also relates to an antibody specifically binding to the herein provided modified VEGF-A polypeptide. In particular, said antibody specifically binds to an epitope comprising a hydrophobic amino acid in place of:

the lysine at the position corresponding to position 84 of the wild-type VEGF-A as shown in SEQ ID NO: 2; and/or the phenylalanine at the position corresponding to position 17 of the wild-type VEGF-A as shown in SEQ ID NO: 2; and wherein said hydrophobic amino acid is selected from the group consisting of alanine, valine, isoleucine, leucine and methionine. The term "antibody", in accordance with the present invention, comprises polyclonal and monoclonal antibodies as well as derivatives or fragments thereof, which still retain the binding specificity. Techniques for the production of antibodies are well known in the art and described, e.g. in Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The term "antibody" in accordance with the invention also includes embodiments such as chimeric, single chain and humanized antibodies, as well as antibody fragments, like, inter alia, Fab fragments, fusion proteins consisting of Eph receptors, ephrin or phosphatase extracellular domains and Fc. Antibody fragments or derivatives further comprise $F(ab')_2$, Fv fragments, scFvs, single domain $V_H$ or V-like domains, such as VhH or V-NAR-domains, as well as multimeric formats such as minibodies, diabodies, tribodies, tetrabodies or chemically conjugated Fab'-multimers; see, for example, Harlow and Lane (1988) and (1999), Altshuler (2010) Biochemistry (Moscow) 75, 1584-605 or Holliger (2005) Nature Biotechnology 23, 1126-36. Various procedures are known in the art and may be used for the production of such antibodies and/or fragments. Thus, the (antibody) derivatives can be produced by peptidomimetics. Further, techniques described for the production of single chain antibodies (see, inter alia, US Patent 4,946,778) can be adapted to produce single chain antibodies specific for polypeptide(s) and fusion proteins of this invention. Also, transgenic animals may be used to express humanized antibodies specific for polypeptides and fusion proteins of this invention. Most preferably, the antibody of this invention is a monoclonal antibody. For the preparation of monoclonal antibodies, any technique, which provides antibodies produced by continuous cell line cultures, can be used. Examples for such techniques include the original hybridoma technique (Köhler and Milstein (1975) Nature 256, 495) as further developed by the art, the trioma technique, the human B-cell hybridoma technique (Kozbor (1983) Immunology Today 4, 72) and the EBV-hybridoma technique to produce human monoclonal antibodies (Cole et al. (1985) Monoclonal Antibodies and Cancer Therapy, Alan R. Liss, Inc., 77). The term antibody also relates to humanized antibodies. "Humanized" forms of non-human (e.g. murine or rabbit) antibodies are chimeric immunoglobulins, immunoglobulin chains or fragments thereof (such as Fv, Fab, Fab', F(ab')2 or other antigen-binding subsequences of antibodies), which contain minimal sequence derived from non-human immunoglobulin. Often, humanized antibodies are human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Furthermore, humanized antibody may comprise residues, which are found neither in the recipient antibody nor in the imported CDR or framework sequences. These modifications are made to further refine and optimize antibody performance. In general, the humanized antibody will comprise substantially all of at least one, and typically two variable domains, in which all or substantially all of the CDR regions correspond to those of a non-human immunoglobulin and all or substantially all of the FR regions are those of a human immunoglobulin consensus sequence. The humanized antibody may also comprise at least a portion of an immunoglobulin constant region (Fc), typically that of a human immunoglobulin. For further details, see: Jones Nature 321 (1986), 522-525; Reichmann Nature 332 (1998), 323-327 and Presta Curr Op Struct Biol 2 (1992), 593-596.

A popular method for humanization of antibodies involves CDR grafting, where a functional antigen-binding site from a non-human 'donor' antibody is grafted onto a human 'acceptor' antibody. CDR grafting methods are known in the art and described, for example, in US 5,225,539, US 5,693,761 and US 6,407,213. Another related method is the production of humanized antibodies from transgenic animals that are genetically engineered to contain one or more humanized immunoglobulin loci, which are capable of undergoing gene rearrangement and gene conversion (see, for example, US 7,129,084). Further methods for designing and producing humanized antibodies are described in US 07/290,975, US 07/310,252 and US 2003/0229208 or by Queen PNAS (1989), 10029-10033. Surface plasmon resonance as employed in the BIAcore system can be used to increase the efficiency like efficiency and/or selectivity of phage antibodies which bind to an epitope of a polypeptide of the invention (Schier (1996) Human Antibodies Hybridomas 7, 97; Malmborg (1995) J. Immunol. Methods 183, 7). It is also envisaged in the context of this invention that the term "antibody" comprises antibody constructs, which may be expressed in cells, e.g. antibody constructs which may be transfected and/or transduced via, amongst others, viruses or plasmid vectors. The antibody described in the context of the invention is capable to specifically bind/interact with an epitope of the mentioned polypeptide or the modified VEGF-A as defined herein. The term "specifically binding/interacting with" as used in accordance with the present invention means that the antibody does not or essentially does not cross-react with an epitope of similar structure. Cross-reactivity of a panel of antibodies under investigation may be tested, for example, by assessing binding of said panel of antibodies under conventional conditions to the epitope of interest as well as to a number of more or less (structurally and/or functionally) closely related epitopes. Only those antibodies that bind to the epitope of interest in its relevant context (e.g. a specific motif in the structure of modified VEGF-A or the functional fragment thereof) but do not or do not essentially bind to any of the other epitopes are considered specific for the epitope of interest and thus to be antibodies in accordance with this invention. Corresponding methods are described e.g. in Harlow and Lane, 1988 and 1999, loc cit. The antibody specifically binds to/interacts with conformational or continuous epitopes, which are unique for the mentioned modified VEGF-A polypeptide. A conformational or discontinuous epitope is characterized for polypeptide antigens by the presence of two or more discrete amino acid residues which are separated in the primary sequence, but come together on the surface of the molecule when the polypeptide folds into the native protein/antigen (Sela (1969) Science 166, 1365; Laver (1990) Cell 61, 553). The two or more discrete amino acid residues contributing to the epitope are present on separate sections of one or more polypeptide chain(s). These residues come together on the surface of the molecule when the polypeptide chain(s) fold(s) into a three-dimensional structure to constitute the epitope. In contrast, a continuous or linear epitope consists of two or more discrete amino acid residues, which are present in a single linear segment of a polypeptide chain.

Various techniques have been developed for the production of antibody fragments. Traditionally, these fragments were derived via proteolytic digestion of intact antibodies (see Morimoto et al (1992) Journal of Biochemical and Biophysical Methods 24:107-117; and Brennan et al (1985) Science 229:81). Antibody fragments can also be produced directly by recombinant host cells and the antibody phage libraries discussed above. Fab'-SH fragments can be directly recovered from E. coli and chemically coupled to form F(ab')2 fragments (Carter et al (1992) Bio/Technology 10:163-167). According to another approach, F(ab')2 fragments can be isolated directly from recombinant host cell culture. Other techniques for the production of antibody fragments will be apparent to the skilled practitioner. In other embodiments, the antibody of choice is a single chain Fv fragment (scFv). See WO 93/16185; U.S. Pat. No. 5,571,894; and U.S. Pat. No. 5,587,458. The antibody fragment may also be a "linear antibody", e.g., as described in U.S. Pat. No. 5,641,870. Such linear antibody fragments may be monospecific or bispecific. Bispecific antibodies with binding specificities for at least two different epitopes (Millstein et al (1983), Nature 305:537-539) may bind to two different epitopes of the modified VEGF-A. Techniques for generating bispecific antibodies from antibody fragments have also been described, such as using chemical linkage wherein intact antibodies are proteolytically cleaved to generate F(ab')2 fragments (Brennan et al (1985) Science 229:81). Fab'-SH fragments can be recovered from E. coli and chemically coupled to form bispecific antibodies (Shalaby et al (1992) J. Exp. Med. 175:217-225. The "diabody" technology provides an alternative method for making bispecific antibody fragments (Hollinger et al (1993) Proc. Natl. Acad. Sci. USA 90:6444-6448). A "Fab fragment" generally is comprised of one light chain and the $C_H1$ and variable regions of one heavy chain. The heavy chain of a Fab molecule cannot form a disulfide bond with another heavy chain molecule. An "Fc" region generally contains two heavy chain fragments comprising the $C_H2$ and $C_H3$ domains of an antibody. The two heavy chain fragments are held together by two or more disulfide bonds and by hydrophobic interactions of the $C_H3$ domains. A "Fab' fragment" generally contains one light chain and a portion of one heavy chain that contains the $V_H$ domain and the $C_H1$ domain and also the region between the $C_H1$ and $C_H2$ domains, such that an interchain disulfide bond can be formed between the two heavy chains of two Fab' fragments to form a $F(ab')_2$ molecule. A "$F(ab')_2$ fragment" generally contains two light chains and two heavy chains containing a portion of the constant region between the $C_H1$ and $C_H2$ domains, such that an interchain disulfide bond is formed between the two heavy chains. A $F(ab')_2$ fragment thus is composed of two Fab' fragments that are held together by a disulfide bond between the two heavy chains. The "Fv region" generally comprises the variable regions from both the heavy and light chains, but lacks the constant regions. Antibodies with more than two valencies are contemplated. Multivalent, "Octopus" antibodies with three or more antigen binding sites and two or more variable domains can be readily produced by recombinant expression of nucleic acid encoding the polypeptide chains of the antibody (US 2002/0004586; WO01/77342). For example, trispecific antibodies can be prepared (Tutt et al (1991) J. Immunol. 147:60.). Binding molecules/antibodies/antigen-binding fragments provided herein are preferably in the IgG1 or IgG3 framework, more preferred human IgG1 or IgG3 framework. Binding molecules, antibodies or antigen-binding fragments of these antibodies in the IgG1 or IgG3, preferably human IgG1 or IgG3, framework, are particularly preferred for use in vaccine therapy.

The following exemplary assay can be used to determine that a candidate antibody is indeed specifically binding to the modified VEGF-A or a functional fragment thereof in accordance with the present invention. Antibodies that selectively and specifically bind to the epitope comprising a hydrophobic amino acid in place of:

the lysine at the position corresponding to position 84 of the wild-type VEGF-A as shown in SEQ ID NO: 2; and/or

the phenylalanine at the position corresponding to position 17 of the wild-type VEGF-A as shown in SEQ ID NO: 2;

and wherein said hydrophobic amino acid is selected from the group consisting of alanine, valine, isoleucine, leucine and methionine can be identified by, e.g., capture ELISA assays. In order to perform such an assay, Corning 96 Well Clear Polystyrene High Bind Stripwell Microplate (product #2592) are coated overnight with increasing amounts (0.02 nM to 3.5 nM) of the affinity purified modified VEGF-A or (the functional fragment thereof) or the purified wild type VEGF-A (or the functional fragment thereof) in 0.05 M $Na_2CO_3$ (pH 9.6) at 4 °C. Subsequently, the reaction is blocked with PBS containing 0.05% Tween-20 (PBS-T) with 5% BSA for 2 hours at room temperature. Solutions containing equal amounts of generated antibodies are captured by overnight incubation at 4 °C. Unbound material is removed by extensive washing with PBS-T. The binding of anti- modified VEGF-A antibodies is detected by incubating wells with appropriate horseradish peroxidase-conjugated secondary antibodies in PBS-T containing 1% BSA for 1 h at 4 °C. Following further washing, modified VEGF-A (or functional fragment thereof-bound) or wild type VEGF-Abound (or functional fragment thereof-bound) anti-modified VEGF-A antibodies are detected by a chromogenic reaction such as with ortho-phenylenediamine. Antibodies that specifically bind to the modified VEGF-A (or the functional fragment thereof), but not to the wild type VEGF-A (or the functional fragment thereof) are selected.

The present invention also relates to a vector comprising the nucleic acid sequence(s) of the present invention.

Many suitable vectors are known to those skilled in molecular biology, the choice of which would depend on the function desired and include plasmids, cosmids, viruses, bacteriophages and other vectors used conventionally in genetic engineering. Methods which are well known to those skilled in the art can be used to construct various plasmids and vectors; see, for example, the techniques described in Sambrook et al. (loc cit.) and Ausubel, Current Protocols in Molecular Biology, Green Publishing Associates and Wiley Interscience, N.Y. (1989), (1994). Alternatively, the polynucleotides and vectors of the invention can be reconstituted into liposomes for delivery to target cells. Relevant sequences can be transferred into expression vectors where expression of a particular polypeptide is required. Typical cloning vectors include pBluescript SK, pGEM, pUC9, pBR322 and pGBT9. Typical expression vectors include pTRE, pCAL-n-EK, pESP-1, or pOP13CAT.

It is also envisaged herein that said vector is a gene targeting vector and/or a gene transfer vector. Gene therapy, which is based on introducing therapeutic genes (for example for vaccination) into cells by *ex vivo* or *in vivo* techniques, is one of the most important applications of gene transfer. In the aspects of gene therapy, the nucleic acid molecule encoding the modified VEGF-A comprises preferably nucleic acids encoding the signal peptide. Such a modified VEGF-A can be selected from the group consisting of SEQ ID NO: 17, SEQ ID NO: 19, SEQ ID NO: 23 and SEQ ID NO: 25. Suitable vectors, vector systems and methods for *in vitro* or *in vivo* gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813, Isner, Lancet 348 (1996), 370-374; Muhlhauser, Circ. Res. 77 (1995), 1077-1086; Wang, Nature Medicine 2 (1996), 714-716; WO 94/29469; WO 97/00957, Schaper, Current Opinion in Biotechnology 7 (1996), 635-640 or Verma, Nature 389 (1997), 239-242 and references cited therein. In certain aspects, the vector is an adeno-associated-virus (AAV) vector. AAV vectors are attractive for gene therapy. The AAV system has several advantages including long-term gene expression, the inability to autonomously replicate without a helper virus, transduction of dividing and nondividing cells, and the lack of pathogenicity from wild-type infections. It is envisaged herein that AAV serotypes display different organ tropism. Accordingly, different AAV serotypes can be employed to target the proteins/polypeptides of the invention to cancers of different organs. It is envisaged herein that different AAV vectors can be employed in gene therapy according to standard protocols (Grieger et al., 2012 and Asokan et al., 2012).

The nucleic acid molecules of the invention and vectors as described herein above may be designed for direct introduction or for introduction via liposomes, or viral vectors (e.g. adenoviral, retroviral) into the cell. Additionally, baculoviral systems or systems based on vaccinia virus or Semliki Forest Virus can be used as eukaryotic expression system for the nucleic acid molecules of the invention. In addition to recombinant production, fragments of the protein, the fusion protein or antigenic fragments of the invention may be produced by direct peptide synthesis using solid-phase techniques (cf Stewart et al. (1969) Solid Phase Peptide Synthesis, WH Freeman Co, San Francisco; Merrifield, J. Am. Chem. Soc. 85 (1963), 2149-2154). *In vitro* protein synthesis may be performed using manual techniques or by automation. Automated synthesis may be achieved, for example, using Applied Biosystems 431A Peptide Synthesizer (Perkin Elmer, Foster City CA) in accordance with the instructions provided by the manufacturer. Various fragments may be chemically synthesized separately and combined using chemical methods to produce the full length molecule.

In certain aspects, the vector comprises a nucleic acid sequence, which is a regulatory sequence operably linked to said nucleic acid sequence defined herein. The term "regulatory sequence" refers to DNA sequences, which are necessary to effect the expression of coding sequences to which they are ligated. The nature of such control sequences differs depending upon the host organism. In prokaryotes, control sequences generally include promoter, ribosomal binding site, and terminators. In eukaryotes general control sequences include promoters, terminators and, in some instances, enhancers, transactivators or transcription factors. The term "control sequence" is intended to include, at a minimum, all components the presence of which are necessary for expression, and may also include additional advantageous components.

The term "operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences. In case the control sequence is a promoter, it is obvious for a skilled person that double-stranded nucleic acid is preferably used.

The recited vector can also be an expression vector. An "expression vector" is a construct that can be used to transform a selected host and provides for expression of a coding sequence in the selected host. Expression vectors can for instance be cloning vectors, binary vectors or integrating vectors. Expression comprises transcription of the nucleic acid molecule preferably into a translatable mRNA. Regulatory elements ensuring expression in prokaryotes and/or eukaryotic cells are well known to those skilled in the art. In the case of eukaryotic cells they comprise normal promoters ensuring initiation of transcription and optionally poly-A signals ensuring termination of transcription and stabilization of the transcript. Possible regulatory elements permitting expression in prokaryotic host cells comprise, e.g., the $P_L$, *lac, trp* or *tac* promoter in *E. coli,* and examples of regulatory elements permitting expression in eukaryotic host cells are the *AOX1* or *GAL1* promoter in yeast or the CMV-, SV40-, RSV-promoter (Rous sarcoma virus), CMV-enhancer, SV40-enhancer or a globin intron in mammalian and other animal cells.

Beside elements, which are responsible for the initiation of transcription such regulatory elements may also comprise transcription termination signals, such as the SV40-poly-A site or the tk-poly-A site, downstream of the polynucleotide. Furthermore, depending on the expression system used leader sequences capable of directing the polypeptide to a cellular compartment or secreting it into the medium may be added to the coding sequence of the recited nucleic acid sequence and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including an N-terminal identification peptide imparting desired characteristics, e.g., stabilization or simplified purification of expressed recombinant product; see supra. In this context, suitable expression vectors are known in the art such as Okayama-Berg cDNA expression vector pcDV1 (Pharmacia), pCDM8, pRc/CMV, pcDNA1, pcDNA3 (Invitrogen), pEF-DHFR, pEF-ADA or pEF-neo (Mack et al. PNAS (1995) 92, 7021-7025 and Raum et al. Cancer Immunol Immunother (2001) 50(3), 141-150) or pSPORT1 (GIBCO BRL).

Preferably, the expression control sequences will be eukaryotic promoter systems in vectors capable of transforming of transfecting eukaryotic host cells, but control sequences for prokaryotic hosts may also be used. Once the vector has been incorporated into the appropriate host, the host is maintained under conditions suitable for high level expression of the nucleotide sequences, and as desired, the collection and purification of the polypeptide of the invention may follow; see, e.g., the appended examples.

The present invention relates to a host transformed with a vector of the present invention or to a host comprising the nucleic acid molecule of this invention. Said host may be any prokaryotic or eukaryotic cell. Suitable prokaryotic/bacterial cells are those generally used for cloning like E. coli or Bacillus subtilis. Said eukaryotic host may be a mammalian cell. In certain aspects, said mammalian cell is a neuronal cell and/or a cultured cell like, inter alia, a HEK 293 (human embryonic kidney) cell, a CHO, HeLa, NIH3T3, BHK or a PC12 cell. The HEK293 cell line can stably express the Epstein-Barr virus nuclear antigen-1 (HEK293-EBNA1, or 293E). In general, this is the most commonly used cell line for large-scale transfection (CSH Protocols; 2008; doi:10.1101/pdb.prot4976).

The term "cell" or "mammalian cell" as used in this context may also comprise a plurality of cells as well as cells comprised in a tissue. The cell to be used in the screening or validation method may be obtained from samples from a (transgenic) non-human animal, e.g., zebrafish, or human suffering from a disease, e.g. angiogenic disease, such as neovascular pathology, macular pathology, cancer or a disease associated with VEGF-A receptor activation. The cell (e.g. a tumor cell and the like) may also be obtained or derived from patient samples (e.g. biopsies), in particular a biopsy/biopsies from a patient/subject suffering from a disease as defined herein above or below. Accordingly, the cell may be a human cell. Again, such a cell to be used in the present screening or validation methods may be comprised in a tissue or tissue sample, like in a sample biopsy. The invention also provides for a host transformed or transfected with a vector of the invention. Said host may be produced by introducing the above described vector of the invention or the above described nucleic acid molecule of the invention into the host. The presence of at least one vector or at least one nucleic acid molecule in the host may mediate the expression of a gene encoding the above described modified VEGF-A or the fragment thereof. The described nucleic acid molecule or vector of the invention, which is introduced in the host, may either integrate into the genome of the host or it may be maintained extrachromosomally. The host can be any prokaryote or eukaryotic cell.

An alternative expression system is the insect system or the insect cell expression system. In one such system, *Autographa californica* nuclear polyhedrosis virus (AcNPV) is used as a vector to express foreign genes in *Spodoptera frugiperda* cells or in *Trichoplusia* larvae. The coding sequence of a recited nucleic acid molecule may be cloned into a nonessential region of the virus, such as the polyhedrin gene, and placed under control of the polyhedrin promoter. Successful insertion of said coding sequence will render the polyhedrin gene inactive and produce recombinant virus lacking coat protein coat. The recombinant viruses are then used to infect *S. frugiperda* cells or *Trichoplusia* larvae in which the protein of the invention is expressed (Smith, J. Virol. 46 (1983), 584; Engelhard, Proc. Nat. Acad. Sci. USA 91 (1994), 3224-3227). In certain aspects, the pFBDM vector can be used for expression. The insertion into the MultiBac baculoviral DNA is mediated via the Tn7 transposition sequence upon transformation in DH10 MultiBac E. coli cells (Berger et al., 2004; Fitzgerald et al., 2006). Virus amplification and expression can be performed in Sf21 (Spodoptera frugiperda) (Gibco, Invitrogen) and/or High Five (Trichoplusia ni) (Gibco, Invitrogen) cell.

Additional regulatory elements may include transcriptional as well as translational enhancers. Advantageously, the above-described vectors of the invention comprise a selectable and/or scorable marker. Selectable marker genes useful for the selection of transformed cells and, e.g., plant tissue and plants are well known to those skilled in the art and comprise, for example, antimetabolite resistance as the basis of selection for dhfr, which confers resistance to methotrexate (Reiss, Plant Physiol. (Life Sci. Adv.) 13 (1994), 143-149); npt, which confers resistance to the aminoglycosides neomycin, kanamycin and paromycin (Herrera-Estrella, EMBO J. 2 (1983), 987-995) and hygro, which confers resistance to hygromycin (Marsh, Gene 32 (1984), 481-485). Additional selectable genes have been described, namely trpB, which allows cells to utilize indole in place of tryptophan; hisD, which allows cells to utilize histinol in place of histidine (Hartman, Proc. Natl. Acad. Sci. USA 85 (1988), 8047); mannose-6-phosphate isomerase which allows cells to utilize mannose (WO 94/20627) and ODC (ornithine decarboxylase) which confers resistance to the ornithine decarboxylase inhibitor, 2-(difluoromethyl)-DL-ornithine, DFMO (McConlogue, 1987, In: Current Communications in Molecular Biology, Cold Spring Harbor Laboratory ed.) or deaminase from Aspergillus terreus which confers resistance to Blasticidin S (Tamura, Biosci. Biotechnol. Biochem. 59 (1995), 2336-2338). Useful scorable markers are also known to those skilled in the art and are commercially available. Advantageously, said marker is a gene encoding luciferase (Giacomin, Pl. Sci. 116 (1996), 59-72; Scikantha, J. Bact. 178 (1996), 121), green fluorescent protein (Gerdes, FEBS Lett. 389 (1996), 44-47) or ß-glucuronidase (Jefferson, EMBO J. 6 (1987), 3901-3907). This embodiment is particularly useful for simple and rapid screening of cells, tissues and organisms containing a recited vector.

As described above, the recited nucleic acid molecule can be used alone or as part of a vector to express the polypeptide of the invention in cells, for, e.g., purification but also for gene therapy purposes. The nucleic acid molecules or vectors containing the DNA sequence(s) encoding any one of the above described polypeptide of the invention is introduced into the cells which in turn produce the polypeptide of interest. Gene therapy, which is based on introducing therapeutic genes into cells by ex vivo or in vivo techniques, is one of the most important applications of gene transfer. Suitable vectors, methods or gene-delivery systems for in-vitro or in-vivo gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813; Verma, Nature 389 (1994), 239; Isner, Lancet 348 (1996), 370-374; Muhlhauser, Circ. Res. 77 (1995), 1077-1086; Onodera, Blood 91 (1998), 30-36; Verma, Gene Ther. 5 (1998), 692-699; Nabel, Ann. N.Y. Acad. Sci. 811 (1997), 289-292; Verzeletti, Hum. Gene Ther. 9 (1998), 2243-51; Wang, Nature Medicine 2 (1996), 714-716; WO 94/29469; WO 97/00957, US 5,580,859; US 5,589,466; or Schaper, Current Opinion in Biotechnology 7 (1996), 635-640. The recited nucleic acid molecules and vectors may be designed for direct introduction or for introduction via liposomes, or viral vectors (e.g., adenoviral, retroviral) into the cell. Preferably, said cell is a germ line cell, embryonic cell, or egg cell or derived there from, most preferably said cell is a stem cell. An example for an embryonic stem cell can be, inter alia, a stem cell as described in Nagy, Proc. Natl. Acad. Sci. USA 90 (1993), 8424-8428.

The term "prokaryote" is meant to include all bacteria, which can be transformed or transfected with DNA or RNA molecules for the expression of a protein of the invention. Prokaryotic hosts may include gram negative as well as gram positive bacteria such as, for example, E. coli, S. typhimurium, Serratia marcescens and Bacillus subtilis. The term "eukaryotic" is meant to include yeast, higher plant, insect and preferably mammalian cells. Depending upon the host employed in a recombinant production procedure, the protein encoded by the polynucleotide of the present invention may be glycosylated or may be non-glycosylated. Especially preferred is the use of a plasmid or a virus containing the coding sequence of the polypeptide of the invention and genetically fused thereto to a Protein A tag. An above described polynucleotide can be used to transform or transfect the host using any of the techniques commonly known to those of ordinary skill in the art. Furthermore, methods for preparing fused, operably linked genes and expressing them in, e.g., mammalian cells and bacteria are well-known in the art (Sambrook, loc cit.).

Herein provided is also a process for the production of a polypeptide to be used in accordance with the present invention, said process comprising culturing/raising the host of the invention under conditions allowing the expression of the polypeptide of the invention and optionally recovering/isolating the produced polypeptide from the culture.

The transformed hosts can be grown in fermenters and cultured according to techniques known in the art to achieve optimal cell growth. The polypeptide of the invention can then be isolated from the growth medium, cellular lysates, or cellular membrane fractions. The isolation and purification of the, e.g., microbially expressed polypeptides of the invention may be by any conventional means such as, for example, preparative chromatographic separations and immunological separations such as those involving the use of monoclonal or polyclonal antibodies directed, e.g., against a tag of the polypeptide of the invention. The conditions for the culturing of a host, which allow the expression, are known in the art to depend on the host system and the expression system/vector used in such process. The parameters to be modified in order to achieve conditions allowing the expression of a recombinant polypeptide are known in the art. Thus, suitable conditions can be determined by the person skilled in the art in the absence of further inventive input.
Once expressed, the polypeptide of the invention can be purified according to standard procedures of the art, including ammonium sulfate precipitation, affinity columns, column chromatography, gel electrophoresis and the like; see, Scopes, "Protein Purification", Springer-Verlag, N.Y. (1982). Substantially pure polypeptides of at least about 90 to 95% homogeneity are preferred, and 98 to 99% or more homogeneity are most preferred, for pharmaceutical uses. Once purified, partially or to homogeneity as desired, the polypeptide of the invention may then be used therapeutically (including extracorporeally) or in developing and performing assay procedures.

Furthermore, the present invention relates to a non-human transgenic animal, wherein said transgenic animal comprises an endogenous vascular endothelial growth factor A (VEGF-A) gene, wherein said endogenous vascular endothelial growth factor A (VEGF-A) gene comprises an artificially introduced mutation. In preferred aspects, the expression of the mutated endogenous vascular endothelial growth factor A (VEGF-A) gene results in a non-functional vascular endothelial growth factor A (VEGF-A) polypeptide.

In further aspects, the present invention relates to a non-human transgenic animal, wherein said transgenic animal comprises an endogenous vascular endothelial growth factor A (VEGF-A) gene, wherein said endogenous vascular endothelial growth factor A (VEGF-A) gene comprises an artificially introduced mutation, and wherein said transgenic animal comprises the modified VEGF-A polypeptide, fragment thereof or a nucleic acid molecule encoding such a polypeptide.

In further aspects, the present invention relates to a non-human animal, wherein said animal comprises the modified VEGF-A polypeptide, fragment thereof or a nucleic acid molecule encoding such a polypeptide.

In certain aspects, the present invention relates to a method for identifying a candidate molecule, preferably a modified vascular endothelial growth factor A (VEGF-A). Preferably, the non-human transgenic animal provided herein or the non-human animal comprising an endogenous wild-type VEGF-A and a modified VEGF-A polypeptide is employed in such a method. In the appended examples, it is shown that such animals are employed to analyze and/or identify the herein provided modified VEGF-A polypeptide.
Furthermore, the present invention relates to the use of the non-human transgenic animal provided herein or the non-human animal comprising an endogenous wild-type VEGF-A and comprising a modified VEGF-A polypeptide. For example, the present invention relates to the non-human transgenic animal or the non-human animal comprising an endogenous wild-type VEGF-A and a modified VEGF-A polypeptide for use in identifying a possible agent for treating an angiogenic disorder or a candidate molecule as a possible inhibitor or stimulator of angiogenesis and/or vasculogenesis. Preferably, the present invention relates to the non-human transgenic animal or the non-human animal comprising an endogenous wild-type VEGF-A and a modified VEGF-A polypeptide for use in identifying a candidate VEGF-A as a possible agent for treating an angiogenic disorder.

"Transgenic animal" or "non-human transgenic animal" is intended herein to include any non-naturally occurring non-human animal in which one or more of the cells of the animal comprises an endogenous vascular endothelial growth factor A (VEGF-A) gene, wherein said endogenous vascular endothelial growth factor A (VEGF-A) gene comprises an artificially introduced mutation. In preferred aspects, the expression of the mutated endogenous vascular endothelial growth factor A (VEGF-A) gene results in a non-functional vascular endothelial growth factor A (VEGF-A) polypeptide.

Furthermore, the "transgenic animal" or "non-human transgenic animal" is intended herein to include any non-naturally occurring non-human animal in which one or more of the cells of the animal comprise an endogenous VEGF-A gene, wherein said endogenous VEGF-A gene comprises an artificially introduced mutation and wherein one or more of the cells further comprise the modified VEGF-A polypeptide, fragment thereof or a nucleic acid molecule encoding such a polypeptide.

For example, an exemplary non-human transgenic animal comprising an artificially introduced mutation, e.g., a frame shift mutation, at the endogenous VEGF-A gene is shown in the appended examples. Furthermore, exemplary non-human transgenic animals comprising the frame shift mutation at the endogenous VEGF-A gene and comprising the modified VEGF-A polypeptide, e.g. VEGF-A K84A, are shown in the appended examples.

In this exemplary mode, the non-human transgenic animal comprises a mutated endogenous VEGF-A gene resulting in a non-functional VEGF-A polypeptide. In such an animal the angiogenesis/vasculogenesis is disturbed. The injection of modified VEGF-A polypeptides or encoding nucleic acid molecules thereof can ameliorate the health condition (rescue), in particular the health condition associated with angiogenesis and/or vasculogenesis, of the non-human transgenic animal; see also below. Such a modified VEGF-A is an angiogenesis/vasculogenesis stimulator. Alternatively, the injection of the modified VEGF-A can deteriorate the health condition of the non-human transgenic animal or leave it unchanged. Thus, such modified VEGF-A molecules fail to rescue the animal; see also below. Such a modified VEGF-A is an angiogenesis/vasculogenesis inhibitor according to the invention.

Furthermore, the "non-human animal comprising an endogenous wild-type VEGF-A and a modified VEGF-A polypeptide" or the like is intended herein to include any non-naturally occurring non-human animal in which one or more of the cells of the animal comprise a modified VEGF-A polypeptide, fragment thereof or a nucleic acid molecule encoding such a polypeptide. It is also herein understood that the non-human animal comprising an endogenous wild-type VEGF-A and a modified VEGF-A polypeptide is a non-human transgenic animal. It is understood in the context of such aspects that the modified VEGF-A polypeptide, fragment thereof or a nucleic acid molecule encoding such a polypeptide can be a modified VEGF-A as provided herein or a modified VEGF-A yet to be identified, e.g., by employing the screening method provided herein.

For example, exemplary non-human animals comprising a wild-type endogenous VEGF-A gene and comprising the modified VEGF-A polypeptide, e.g. VEGF-A K84A, are shown in the appended examples.

In this exemplary mode, the non-human animal comprises a wild-type endogenous VEGF-A and further comprises the modified VEGF-A. Again the health condition of the non-human transgenic animal is indicative for the effectiveness of the modified VEGF-A molecule. In case the modified VEGF-A molecule is an angiogenesis/vasculogenesis inhibitor, the modified VEGF-A polypeptide preferably also inhibits the naturally occurring VEGF-A present in the animal leading to a deteriorated angiogenesis and/or vasculogenesis. Thus, the resulting health condition of such an animal is deteriorated as observed in the illustrative Figure 3. In case the modified VEGF-A molecule is an angiogenesis/vasculogenesis stimulator, the health condition of the animal should preferably be unchanged.

Artificially introduced mutation includes insertion, missense, frame shift, deletion, or substitution, or replacement of DNA sequence, or any combination thereof. In preferred embodiments, the transgenic animal comprises an artificially introduced mutation, wherein said mutation is a frame shift mutation. Insertions include the insertion of entire genes, which may be of animal, plant, fungal, insect, prokaryotic, or viral origin. The endogenous vascular endothelial growth factor A (VEGF-A) comprising an artificially introduced mutation is referred herein as mutated endogenous vascular growth factor A (VEGF-A).

The term "transgenic animal" includes animals that are heterozygous and/or homozygous for endogenous vascular endothelial growth factor A (VEGF-A) gene comprising an artificially introduced mutation. For example, a transgenic animal being heterozygous for the VEGF-A gene comprising an artificially introduced mutation is herein also referred to *vegfaa*$^{+/-}$. A transgenic animal being homozygous for the VEGF-A gene comprising an artificially introduced mutation is herein also referred to *vegfaa*$^{-/-}$.

The artificially introduced mutation can be by way of human intervention, such as by transgenic techniques well known in the art. The nucleic acid is introduced into the cell, directly or indirectly by introduction into a precursor of the cell, by way of deliberate genetic manipulation, such as by microinjection or by infection with a recombinant virus. The term genetic manipulation does not include classical cross-breeding, but rather is directed to the introduction of a recombinant DNA molecule. This molecule may be integrated within a chromosome, or it may be extrachromosomally replicating DNA. As shown in the appended example, transcription activator-like effector nucleases (TALENs) (Maurice S. Brozzo et al. Thermodynamic and structural description of allosterically regulated VEGFR-2 dimerization. Blood. 16, 1781-1788 (2012)) can be employed to introduce the mutation. TALENs are artificial restriction enzymes generated by fusing a TAL effector DNA-binding domain to a DNA cleavage domain. The restriction enzymes are enzymes that cut DNA strands at a specific sequence. Transcription activator-like effectors (TALEs) can be engineered to bind at a desired DNA sequence. By combining such an engineered TALE with a DNA cleavage domain (which cuts DNA strands), one can engineer restriction enzymes that are specific for the desired DNA sequence. The skilled person is aware that any other method by way of human intervention can be used to genetically manipulate the non-human transgenic organism or one or more cells of the same, e.g. methods based on the CRISPR technology.

In certain preferred aspects, the expression of the mutated endogenous vascular endothelial growth factor A (VEGF-A) gene results in a non-functional vascular endothelial growth factor A (VEGF-A) polypeptide. A non-functional mutation can for example alter the activity of a normal naturally occurring gene product by inhibiting its activity partially or completely. The non-functional mutation can also alter the activity by enhancing the normal gene product's activity or by altering its sequence. In preferred aspects, the non-functional mutation eliminates the activity/function of the normal or wild-type VEGF-A gene product. Thus, in preferred aspects, the non-functional vascular endothelial growth factor A (VEGF-A) polypeptide has no or almost no activity/function compared to the normal or wild-type VEGF-A gene product. For instance, the non-functional vascular endothelial growth factor A (VEGF-A) polypeptide cannot bind to its receptor or cannot dimerize. In the appended Examples, said mutated endogenous vascular growth factor A (VEGF-A) gene has a deletion of 10 nucleotides. Said exemplary mutated endogenous vascular growth factor A (VEGF-A) gene has a sequence as shown in SEQ ID NO: 32. The deletion of the 10 nucleotides results in a frame shift allele. In certain aspects, the expression of the mutated endogenous vascular endothelial growth factor A (VEGF-A) gene results in a non-functional vascular endothelial growth factor A (VEGF-A) polypeptide. Such a gene product is shown in SEQ ID NO: 33. In the appended examples, the mutated endogenous VEGF-A polypeptide lacks the VEGF-A and PDGF knot motifs. The cysteines present in the knot motif are crucial for VEGF-A dimerization, which is thought to promote VEGF-A receptor mediated signaling. Hence, the exemplary mutation introduced in the endogenous VEGF-A is one example of a non-functional VEGF-A polypeptide. This suggests that the exemplary mutation of the endogenous VEGF-A gene is a null allele. As used herein, a null allele is a mutant copy of a gene at a locus that completely lacks that gene's normal function. The exemplary artificially mutation introduced leads to impaired angiogenesis and/or vasculogenesis.

The term "endogenous vascular endothelial growth factor A (VEGF-A) gene" is meant to include the naturally occurring genetic loci of VEGF-A found in the host animal. The term "mutated endogenous vascular endothelial growth factor A (VEGF-A) gene" or the like is meant to include the naturally occurring genetic loci of VEGF-A found in the animal that has an artificially introduced mutation according to the invention, and wherein preferably the artificially introduced mutation results in a non-functional gene product. The artificially introduced mutation can be introduced at only one allele or at both alleles.

It is herein understood that the transgenic animal can further, i.e., in addition to the mutated endogenous VEGF-A, comprise a transgene having a nucleic acid sequence that has been introduced into a cell by way of human intervention such as by way of the described methods herein. In preferred aspects, said transgenic animal comprises further the modified vascular endothelial growth factor A (VEGF-A) polypeptide of the present invention. The transgene can be partly or entirely heterologous, i.e., foreign, to the transgenic animal or cell into which it is introduced. A transgene can include one or more transcriptional regulatory sequences and any other nucleic acid, such as introns, that may be necessary for optimal expression of a selected nucleic acid. This transgene can comprise a fluorescent protein. An exemplary transgene can be etsrp fused to green fluorescent protein (GFP) or kdrl fused to mCherry. The term "non-human transgenic animal" or "non-human animal" is intended to include any vertebrate such as fish, mammals, birds, reptiles, and amphibians. Suitable mammals include, e.g., rodents, non-human primates, sheep, dogs and cows. Suitable fish include e.g. Danio rerio; suitable birds include, e.g., chickens, geese, and turkeys. Further non-human transgenic animals are selected from the rodent family including rat and mouse. In preferred aspects, the non-human transgenic animal is a fish, e.g., zebrafish or Danio rerio. It is herein understood that the non-human transgenic animal or the non-human animal comprising an endogenous wild-type VEGF-A and a modified VEGF-A polypeptide according to the invention includes every developmental stage of the transgenic animal. For example, most preferably the animal is a zebrafish, wherein all developmental stages are herein encompassed, e.g., zygote, cleavage, blastula, gastrula, segmentation, pharyngula, hatching, larval, juvenile and adult stage. The stages larval and juvenile are herein preferred.

In most preferred aspects, the present invention relates to a non-human transgenic animal, wherein said transgenic animal comprises an endogenous vascular endothelial growth factor A (VEGF-A) gene, wherein said endogenous vascular endothelial growth factor A (VEGF-A) gene comprises an artificially introduced mutation, wherein said transgenic animal is a zebrafish.

In certain aspects, the present invention relates to a non-human transgenic animal, wherein said transgenic animal comprises an endogenous vascular endothelial growth factor A (VEGF-A) gene, wherein said endogenous vascular endothelial growth factor A (VEGF-A) gene comprises an artificially introduced mutation, and wherein said transgenic animal comprises the modified vascular endothelial growth factor A (VEGF-A) polypeptide of the present invention. The modified VEGF-A polypeptide can be an anti-angiogenic and/or anti-vasculogenic polypeptide as described herein. In preferred aspects, the modified vascular endothelial growth factor A (VEGF-A) comprises a hydrophobic amino acid in place of: the lysine at the position corresponding to position 84 of the wild-type VEGF-A as shown in SEQ ID NO: 2; and/or the phenylalanine at the position corresponding to position 17 of the wild-type VEGF-A as shown in SEQ ID NO: 2. In the appended Examples, it is proven that the replacement of the amino acids, such as K84 or F 17, by a hydrophobic amino acid in the context of the VEGF-A 121 isoform lacking the heparin/Nrp-1 binding domain does not ameliorate the phenotype of the zebrafish carrying a VEGF-A null allele. Therefore, the herein provided inventive modified VEGF-A polypeptide demonstrates a specific and surprising effect in the herein provided *in vivo* system.

The modified vascular endothelial growth factor A (VEGF-A) can be introduced into the non-human transgenic animal or the non-human animal by way that is well known in the prior art, such as insertion of nucleic acid molecules or polypeptides. For example, mRNA encoding the modified VEGF-A polypeptide according to the invention can be injected at a tone cell stage.

In certain aspects, the present invention relates to a method for producing the non-human transgenic animal. Said method can comprise the introduction of an artificial mutation into the endogenous vascular endothelial growth factor A (VEGF-A) gene in a non-human animal at a stage no later than the 4-cell stage.
The one cell embryo undergoes a series of cleavage divisions, progressing through 2-cell, 4-cell, 8-cell and 16 cell stages. Cleavage is the division of cells in the early embryo. Spiral cleavage can vary between species, but generally the first two cell divisions result in four macromeres, also called blastomeres, (A, B, C, D) each representing one quadrant of the embryo. These first two cleavages are oriented in planes that occur at right angles parallel to the animal-vegetal axis of the zygote. At the 4-cell stage, the A and C macromeres meet at the animal pole, creating the animal cross-furrow, while the B and D macromeres meet at the vegetal pole, creating the vegetal cross-furro.

In certain aspects, the present invention relates to a method for identifying a candidate modified vascular endothelial growth factor A (VEGF-A) as a possible agent for treating an angiogenic disorder, wherein said method comprises:

(a) contacting or otherwise exposing a non-human transgenic animal to said candidate modified vascular endothelial growth factor A (VEGF-A), wherein said transgenic animal comprises an endogenous vascular endothelial growth factor A (VEGF-A) gene with an artificially introduced mutation, wherein the expression of the mutated endogenous vascular endothelial growth factor A (VEGF-A) gene results in a non-functional vascular endothelial growth factor A (VEGF-A) polypeptide;
(b) monitoring the health condition of said transgenic animal comprising the modified vascular endothelial growth factor A (VEGF-A) and the endogenous vascular endothelial growth factor A (VEGF-A) gene with an artificially introduced mutation; and
(c) selecting said modified vascular endothelial growth factor A (VEGF-A) that results in impaired vasculogenesis and/or angiogenesis of the transgenic animal, and wherein said impaired vasculogenesis and/or angiogenesis is indicative of the capacity of said selected modified vascular endothelial growth factor A (VEGF-A) to inhibit vasculogenesis and/or angiogenesis.

It is also understood herein that the assay/method for screening the non-human transgenic animal provided herein can be used to determine/identify/analyze whether a potential candidate molecule stimulates or ameliorates the angiogenesis and/or vasculogenesis. Such a candidate molecule is a stimulator of angiogenesis and/or vasculogenesis.

Furthermore, the present invention also relates to a method for identifying a candidate molecule as a possible angiogenesis and/or vasculogenesis inhibitor or stimulator, wherein said method comprises:

(a) contacting or otherwise exposing a non-human transgenic animal to said candidate molecule, wherein said transgenic animal comprises an endogenous vascular endothelial growth factor A (VEGF-A) gene with an artificially introduced mutation, wherein the expression of the mutated endogenous vascular endothelial growth factor A (VEGF-A) gene results in a non-functional vascular endothelial growth factor A (VEGF-A) polypeptide;
(b) monitoring the health condition of said transgenic animal comprising said candidate molecule; and
(c) selecting said candidate molecule that results in impaired vasculogenesis and/or angiogenesis of the transgenic animal; or
selecting said candidate molecule that results in ameliorated vasculogenesis and/or angiogenesis of the transgenic animal,
wherein said impaired vasculogenesis and/or angiogenesis is indicative of the capacity of said selected candidate molecule to inhibit vasculogenesis and/or angiogenesis; or
wherein said ameliorated vasculogenesis and/or angiogenesis is indicative of the capacity of said selected candidate molecule to stimulate vasculogenesis and/or angiogenesis.

The non-human transgenic animal or the non-human animal is contacted or otherwise exposed with a candidate molecule or preferably with a candidate modified VEGF-A polypeptide. The contacting is preferably injection, i.e. injecting the candidate molecule into the non-human transgenic animal or the non-human animal. However, it is herein understood that the candidate molecule can be inserted into the non-human animal by any other means and methods. The candidate molecule can be injected e.g. as a polypeptide, or a nucleic acid encoding such a polypeptide, e.g. mRNA.

The candidate modified VEGF-A polypeptide can be a VEGF-A carrying one or several mutation(s). It was shown in the appended examples that the herein provided modified VEGF-A molecules were surprisingly found by employing the inventive animals and the screening methods. In the appended examples, the modified or wild-type VEGF-A molecules, e.g. mRNA encoding the modified or wild-type VEGF-A polypeptide, are injected into the non-human transgenic animal, e.g. into eggs, preferably from zebrafish, that carry a homozygous frame shift mutation in the VEGF-A gene resulting in a VEGF-A null allele. It is also demonstrated in the appended examples that the modified or wild-type VEGF-A molecules are injected into the non-human transgenic animal, e.g. eggs, that carry a frame shift mutation in one allele of the VEGF-A gene. In other words, the non-human transgenic animal can also be heterozygotic for the mutated endogenous vascular endothelial growth factor A (VEGF-A) gene. For example, such a transgenic animal carries the mutation at one allele of the endogenous VEGF-A and the other allele is a naturally occurring wild-type version. It is also demonstrated in the appended examples that the modified or wild-type VEGF-A molecules are injected into the non-human animal, e.g. eggs, that is a wild-type animal.

In the method for identifying a candidate molecule, e.g. the modified vascular endothelial growth factor A (VEGF-A) and the use of the inventive animals, the health conditions of the non-human transgenic animal comprising the modified vascular endothelial growth factor A (VEGF-A) or the wild-type VEGF-A, are monitored. Such health conditions can be indicative for vasculogenesis and/or angiogenesis. Thus, the health condition is indicative for vascular abnormalities. Indications for angiogenesis and/or vasculogenesis can be the vascular patterning and/or vascular system. Impaired or deteriorated vasculogenesis and/or angiogenesis can be manifested by defects in the vascular system or pattering. In particular, pericardial edema, blood pooling and/or lack of blood circulation is indicative for impaired angiogenesis and/or vasculogenesis. Thus, the blood flow circulation or the presence of the pericardial edema is monitored. In case the non-human transgenic animal comprises an endogenous VEGF-A gene with an artificially introduced mutation resulting in a non-functional gene product, the selected candidate molecule that is an inhibitor of angiogenesis and/or vasculogenesis can also merely result in an unchanged vasculogenesis and/or angiogenesis because the transgenic animal comprising the non-functional VEGF-A gene product already has a deteriorated or impaired vasculogenesis and/or angiogenesis. Thus, in this aspect, the selected candidate molecule can also fail to improve or ameliorate angiogenesis and/or vasculogenesis. In other words, such a selected candidate molecule fails to rescue viability caused by the non-functional vascular endothelial growth factor A (VEGF-A) polypeptide. Ultimately, such animals with deteriorated, impaired or unchanged vasculogenesis and/or angiogenesis may result in lethality, e.g. at about 5 days post fertilization. In the appended examples, the rescue potential of the modified VEGF-A polypeptide is monitored/measured, which reflects the angiogenesis and/or vasculogenesis of the non-human transgenic animal. Thus, the resulting phenotype or the health condition of the non-human transgenic animal comprising the modified vascular endothelial growth factor A (VEGF-A) or other candidate molecules can be measured as a percentage of embryos showing blood flow circulation and lacking pericardial edema. It is shown in the appended examples that the modified VEGF-A polypeptides abrogate the rescue of the non-human transgenic animal, e.g., the zebrafish contain a *vegfaa*$^{-/-}$ gene. Thus, the herein provided modified VEGF-A fails to rescue the viability of the transgenic non-human animal comprising said endogenous vascular endothelial growth factor A (VEGF-A) gene with an artificially introduced mutation. Accordingly, the modified VEGF-A polypeptides, fragments thereof or the encoding nucleotide sequences are angiogenesis and/or vasculogenesis inhibitors. As used herein, "fails to rescue" or "fails to rescue viability" means that the health conditions are not improved, but remain unchanged or are even deteriorated. In other words, the viability is not rescued. This may result ultimately in lethality of the non-human transgenic animal due to the failed rescue, e.g. at day 5 post fertilization.

It is understood herein that ameliorated or improved vasculogenesis and/or angiogenesis can be manifested by improving blood flow circulation and lacking pericardial edema of e.g. the transgenic animal comprising an endogenous mutated VEGF-A gene. Ultimately, animals with improved or ameliorated vasculogenesis and/or angiogenesis show a longer survival rate and thus are rescued in comparison to transgenic animals comprising an endogenous mutated VEGF-A gene. Transgenic animals comprising an endogenous mutated VEGF-A gene die, preferably, at about day 5 post fertilization. For example, the transgenic animal comprising the vegfaa$^{-/-}$ die at around 5 days post fertilization, when no rescue is performed or no injection of a stimulator is provided. In preferred aspect, the animal with ameliorated and/or improved vasculogenesis, i.e., the rescued animal, lives until adulthood and is able to reproduce. However, it is herein understood that the life span of the animals or transgenic animal is depended on environmental conditions and stress level and thus can vary. In preferred aspects, said (candidate) modified vascular endothelial growth factor A (VEGF-A) is selected that results in impaired vasculogenesis and/or angiogenesis of the transgenic animal. Said impaired vasculogenesis and/or angiogenesis is indicative of the capacity of said selected modified vascular endothelial growth factor A (VEGF-A) to inhibit vasculogenesis and/or angiogenesis. An impaired vasculogenesis and/or angiogenesis can be manifested by defects in the vascular system or pattering, e.g. by the formation of pericardial edema, blood pooling and/or lack of circulation. Such an effect was shown in the appended examples by the exemplary modified VEGF-A, e.g., VEGF-A K84A or F17A.

In further aspects, said (candidate) modified vascular endothelial growth factor A (VEGF-A) is selected that results in ameliorated vasculogenesis and/or angiogenesis of the transgenic animal. Said ameliorated vasculogenesis and/or angiogenesis is indicative of the capacity of said selected modified vascular endothelial growth factor A (VEGF-A) to stimulate vasculogenesis and/or angiogenesis. An amelioration of vasculogenesis and/or angiogenesis can be manifested by a healthy or normal vascular system or pattering. For example, it can be manifested in the prevention of pericardial edema e.g. through the formation of a functional circulatory loop and/or by differentiation of the dorsal aorta. Such an effect was shown in the appended examples by the exemplary wild-type VEGF-A. This effect is observed in case the molecule is an angiogenesis/vasculogenesis stimulator.

In certain aspects, the health conditions of said transgenic animal comprising said modified vascular endothelial growth factor A (VEGF-A) polypeptide and said endogenous vascular endothelial growth factor A (VEGF-A) gene with an artificially introduced mutation are compared to a transgenic animal comprising a wild-type vascular endothelial growth factor A (VEGF-A) polypeptide and said endogenous vascular endothelial growth factor A (VEGF-A) gene with an artificially introduced mutation; and wherein said modified vascular endothelial growth factor A (VEGF-A) is selected that results in impaired vasculogenesis and/or angiogenesis compared to the transgenic animal comprising the wild-type vascular endothelial growth factor A (VEGF-A) polypeptide. As shown in the appended examples, the wild-type VEGF-A, i.e., the VEGF-A polypeptide as naturally occurring, rescued or ameliorated the health condition associated with the artificial mutation introduced in the endogenous VEGF-A. In particular, the health condition associated with angiogenesis and/or vasculogenesis, e.g., blood flow circulation and/or presence of pericardial edema, are ameliorated/improved.

Furthermore, the present invention also relates to a method for identifying a candidate molecule as a possible angiogenesis and/or vasculogenesis inhibitor or stimulator, wherein said method comprises:

(a) contacting or otherwise exposing a non-human transgenic animal to said candidate molecule, preferably modified VEGF-A, wherein said transgenic animal comprises an endogenous vascular endothelial growth factor A (VEGF-A) gene with an artificially introduced mutation at one allele, wherein the expression of the mutated endogenous vascular endothelial growth factor A (VEGF-A) gene results in a non-functional vascular endothelial growth factor A (VEGF-A) polypeptide, and wherein the endogenous VEGF-A at the other allele is a wild-type VEGF-A;
(b) monitoring the health condition of said transgenic animal comprising said candidate molecule; and
(c) selecting said candidate molecule that results in impaired vasculogenesis and/or angiogenesis of the transgenic animal;
wherein said impaired vasculogenesis and/or angiogenesis is indicative of the capacity of said selected candidate molecule to inhibit vasculogenesis and/or angiogenesis and, optionally, is indicative that the selected candidate molecule has a dominant negative function on the wild-type endogenous VEGF-A.

In such aspects, the non-human transgenic animal is heterozygotic for the VEGF-A gene. As explained above, said impaired vasculogenesis and/or angiogenesis are/is manifested by defects in the vascular system or pattering, e.g. by the formation of pericardial edema, blood pooling and/or lack of circulation. Due to the presence of a wild-type VEGF-A the effects can be milder compared to the transgenic animal comprising the mutation of the endogenous VEGF-A at both alleles. It is herein understood that a candidate molecule that has a dominant negative function adversely affects the naturally-occurring gene product. For example, the dominant negative polypeptide can be form a dimer with the naturally-occurring version of the polypeptide and prevent, for example, the interaction and/or activation of the receptor and thus signal mediation.
It is herein understood that any further assay/method for screening can be employed that is suitable to determine/identify/analyze whether a potential candidate molecule inhibits/prevents/decreases or stimulates/improves the angiogenesis and/or vasculogenesis.
For example, the present invention also relates to a method for identifying a candidate modified vascular endothelial growth factor A (VEGF-A) as a possible angiogenesis and/or vasculogenesis inhibitor or stimulator, wherein said method comprises:

(a) contacting or otherwise exposing a non-human animal to said candidate modified vascular endothelial growth factor A (VEGF-A), wherein said animal comprises an endogenous vascular endothelial growth factor A (VEGF-A);
(b) monitoring the health condition of said animal comprising the modified vascular endothelial growth factor A (VEGF-A) and the endogenous vascular endothelial growth factor A (VEGF-A) gene; and
(c) selecting said modified vascular endothelial growth factor A (VEGF-A) that results in impaired vasculogenesis and/or angiogenesis of said animal, or selecting said modified vascular endothelial growth factor A (VEGF-A) that results in improved vasculogenesis and/or angiogenesis of said animal,
wherein said impaired vasculogenesis and/or angiogenesis is indicative of the capacity of said selected candidate modified vascular endothelial growth factor A (VEGF-A) to inhibit vasculogenesis and/or angiogenesis; and
wherein said improved vasculogenesis and/or angiogenesis is indicative of the capacity of said selected candidate modified vascular endothelial growth factor A (VEGF-A) to stimulate vasculogenesis and/or angiogenesis.

Furthermore, the present invention also relates to a method for identifying a candidate molecule as possible angiogenesis and/or vasculogenesis inhibitor or stimulator, wherein said method comprises:

(a) contacting or otherwise exposing a non-human animal to said candidate molecule, wherein said animal comprises an endogenous vascular endothelial growth factor A (VEGF-A);
(b) monitoring the health condition of said animal comprising the candidate molecule and the endogenous vascular endothelial growth factor A (VEGF-A) gene; and
(c) selecting said candidate molecule that results in impaired vasculogenesis and/or angiogenesis of the animal; or selecting said candidate molecule that results in improved vasculogenesis and/or angiogenesis of the animal,
wherein said impaired vasculogenesis and/or angiogenesis is indicative of the capacity of said selected candidate molecule to inhibit vasculogenesis and/or angiogenesis; and
wherein said improved vasculogenesis and/or angiogenesis is indicative of the capacity of said selected candidate molecule to stimulate vasculogenesis and/or angiogenesis.

Such assays are particularly suitable to identify candidate molecules that are inhibitors of angiogenesis and/or vasculogenesis and that have a dominant negative function. A candidate molecule that has a dominant negative function adversely affects the normal, wild-type gene product within the same cell.
As mentioned above, it is herein understood that impaired or deteriorated vasculogenesis and/or angiogenesis can be manifested by defects in the vascular system or pattering. As observed in the appended examples, the injection of modified VEGF-A, such as VEGF-F K84A or F17A, into wild-type animals results in pericardial edema, blood pooling and lack of circulation. Thus, impaired or deteriorated vasculogenesis and/or angiogenesis can be manifested by pericardial edema, blood pooling and lack of circulation.

As explained above, it is understood herein that improved vasculogenesis and/or angiogenesis can be manifested by an unchanged health conditions of the animal; e.g., during development, that is observed normally in case of wild-type animals. The vasculogenesis and/or angiogenesis can also be improved in terms of blood flow circulation and lacking pericardial edema.

It is herein understood that candidate molecules can be polypeptides, fragments thereof or encoding nucleic acid sequences, small molecule drug(s), siRNA, shRNA, miRNA, dsRNA, small temporal RNA (stRNA), antisense molecules or binding proteins that result in an impaired or ameliorated vasculogenesis and/or angiogenesis. A small molecule drug to be used herein can refer to an (organic) low molecular weight (<900 Daltons) compound. A binding protein can be selected from the group consisting of antibodies, Fab fragments, Fab' fragments, $F(ab')_2$ fragments, single chain variable fragments (scFv), (single) domain antibodies, isolated variable regions of antibodies (VL and/or VH regions), CDRs, immunoglobulin domains, CDR-derived peptidomimetics and the like. Candidate molecules identified by the herein provided method and non-human transgenic animal can be either stimulators or inhibitors of angiogenesis and/or vasculogenesis. Candidate molecules can also be polypeptides or fragments thereof or encoding nucleic acid molecules that are associated with angiogenesis and/or vasculogenesis, such as, VEGF-A, PDGF ANGPT1, ANGPT2, ANPEP, TYMP, EREG, FGF1, FGF2, FIGF, FLT1, JAG1, KDR, LAMA5, NRP1, NRP2, PGF, PLXDC1, STAB1, PLGF, VEGF-B, VEGF-C, and VEGF-D. Preferred herein are polypeptides or fragments thereof or encoding nucleic acid molecules carrying (a) modification(s) in its sequence compared to the naturally occurring version. Preferred candidate molecules identified by the herein provided method are modified VEGF-A polypeptides, fragments thereof or encoding nucleic acid sequences. Most preferred candidate molecules identified by the herein provided method are modified VEGF-A polypeptides, fragments thereof or encoding nucleic acid sequences resulting in impaired vasculogenesis and/or angiogenesis and are thus inhibitors of angiogenesis and/or vasculogenesis.

Also the candidate molecule, the specific mutation of the candidate molecule yet to be identified utilizing the assay/method for screening and/or the non-human transgenic animal provided herein is envisaged in context of the present invention. The assay/method for screening and/or the non-human transgenic animal provided herein can also be used to identify a novel use of a known molecule. Such a novel use of a know molecule is also envisaged herein.

In a further embodiment, the nucleic acid molecule, the vector, the modified vascular endothelial growth factor A (VEGF-A), the functional fragment thereof or the fusion protein/polypeptide according to the invention can be used as a medicament, i.e. the modified vascular endothelial growth factor A (VEGF-A) or the functional fragment thereof provided and described herein are for use in medicine. The terms "medicament" and "pharmaceutical composition" are used interchangeably herein. Accordingly, definitions and explanations provided herein in relation to "pharmaceutical compositions", apply, mutatis mutandis, to the term "medicament".

The present invention relates to a modified vascular endothelial growth factor A (VEGF-A) polypeptide for use in treating an angiogenic disorder. Further, the present invention relates to a method of treatment for an angiogenic disorder comprising the step of administering to a subject in need of such treatment a pharmaceutical effective amount of a modified vascular endothelial growth factor A (VEGF-A) polypeptide. In preferred embodiments, it is understood herein that an angiogenic disorder/disease can be a neovascular pathology and/or macular pathology. It is understood herein that a neovascular pathology is selected from the group of age related macular degeneration, neovascular age related macular degeneration, diabetic macular edema, high risk-corneal transplantation and neovascular glaucoma. In particular preferred embodiments, the neovascular pathology is age related macular degeneration. In most preferred embodiments, the age rated macular degeneration is a neovascular age related macular degeneration. In certain aspects, angiogenic disorder can be cancer, tumor, a tumorous disease, a blood-brain barrier permeability alterations, a neuroinflammatory disorder, an inflammatory disorder, osteoporosis, psoriasis, obesity, Mycobacterial infections, and/or granuloma.

The term "treating an angiogenic disorder", "treatment of a disorder or a disease" as used herein, such as "neovascular pathology" or "treatment of cancer", is well known in the art. "Treatment of a disorder or disease" implies that a disorder or disease is suspected or has been diagnosed in a patient/subject. A patient/subject suspected of suffering from a disorder or disease typically shows specific clinical and/or pathological symptoms, which a skilled person can attribute to a specific pathological condition (i.e., diagnose a disorder or disease).

The "treatment" of a disorder or disease may, for example, lead to a halt in the progression of the disorder or disease (e.g., no deterioration of symptoms) or a delay in the progression of the disorder or disease (in case the halt in progression is of a transient nature only). In preferred aspects, the treatment of a disorder such as neovascular pathology improves the vision of a subject/patient. The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may, e.g., lead to a halt in the progression of the disorder or disease or a delay in the progression of the disorder or disease. Such a partial or complete response may be followed by a relapse. It is to be understood that a subject/patient may experience a broad range of responses to a treatment (e.g., the exemplary responses as described herein above). The treatment of a disorder or disease may, inter alia, comprise curative treatment (preferably leading to a complete response and eventually to healing of the disorder or disease) and palliative treatment (including symptomatic relief).

The present invention also relates to modified vascular endothelial growth factor A (VEGF-A) for use in the prevention of an angiogenic disorder. The term "prevention of a disorder or disease" as used herein, such as "prevention of neovascular pathology" or "prevention of cancer", is also well known in the art. For example, a patient/subject suspected of being prone to suffer from a disorder or disease as defined herein may, in particular, benefit from a prevention of the disorder or disease. The subject/patient may have a susceptibility or predisposition for a disorder or disease, including but not limited to hereditary predisposition. Such a predisposition can be determined by standard assays, using, for example, genetic markers or phenotypic indicators. It is to be understood that a disorder or disease to be prevented in accordance with the present invention has not been diagnosed or cannot be diagnosed in the patient/subject (for example, the patient/subject does not show any clinical or pathological symptoms). Thus, the term "prevention" comprises the use of compounds of the present invention before any clinical and/or pathological symptoms are diagnosed or determined or can be diagnosed or determined by the attending physician.

The present invention provides a pharmaceutical composition comprising modified vascular endothelial growth factor A (VEGF-A) polypeptide or the functional fragment thereof, the fusion protein, the nucleic acid molecule of the invention and/or a pharmaceutical excipient. It is understood herein that said pharmaceutical excipient can comprise or is a pharmaceutical carrier and/or diluent.

The modified VEGF-A polypeptide, the pharmaceutical composition, the nucleic acid molecule according to the present invention can be used for the treatment and/or prevention of angiogenic disorders. Said angiogenic disorder is e.g., a neovascular pathology, macular pathology, a cancer, a tumor, a tumorous disease, a blood-brain barrier permeability alterations, a neuroinflammatory disorder, an inflammatory disorder, osteoporosis, psoriasis, obesity, Mycobacterial infections, and/or granuloma. In preferred embodiments, the neovascular pathology is selected from the group of age related macular degeneration, neovascular age related macular degeneration, diabetic macular edema, high risk-corneal transplantation and neovascular glaucoma. In particular preferred embodiments, the pharmaceutical composition, the nucleic acid molecule, the vector and/or the modified VEGF-A polypeptide is used for the treatment or prevention of age related macular degeneration. In most preferred embodiments, the pharmaceutical composition, the nucleic acid molecule, the vector and/or the modified VEGF-A polypeptide is used for the treatment or prevention of neovascular age related macular degeneration. It is herein understood that "age related macular degeneration" can be also referred to as "macular degeneration", "AMD" or "ARMD". Age related macular degeneration is a medical condition that usually affects older adults and results in a loss of vision in the centre of the visual field (the macula) because of damage of the retina. It can occur in "dry" or "wet" forms. In the dry form, cellular debris called duress accumulates between the retina and choroid and the retina can become detached. The wet form is also referred to as "neovascular age related macular degeneration", "neovascular AMD" or "exudative age related macular degeneration". The neovascular age related macular degeneration causes vision loss due to abnormal blood vessel growth (choroidal neovascularization) in the choriocapillaris, through Bruch's membrane. Without being bound by theory, the proliferation of abnormal blood vessels in the retina is stimulated by vascular endothelial growth factor A (VEGF-A). These new vessels are fragile, ultimately leading to blood and protein leakage below the macula. Bleeding, leaking, and scarring from these blood vessels eventually cause irreversible damage to the photoreceptors and rapid vision loss.

Furthermore, the modified VEGF-A polypeptide, the pharmaceutical composition, the nucleic acid molecule according to the present invention can be used for the treatment of angiogenic disorders, wherein the blood flow circulation is reduced.

In certain aspects, the invention provides the modified VEGF-A polypeptide, the pharmaceutical composition, the nucleic acid molecule and/or the vector according to the present invention for use of the treatment of tumor, wherein the tumor is a solid tumor. Further, the invention provides the modified VEGF-A polypeptide, the pharmaceutical composition, the nucleic acid molecule and/or the vector according to the present invention for use in the treatment of cancer, wherein the cancer is selected from the group consisting of pancreatic cancer, angioma (or Cerebral cavernous malformation), cervical cancer, breast cancer, colon cancer, melanoma, prostate cancer, bladder cancer and tongue cancer. In preferred aspects, the invention provides the modified VEGF-A polypeptide, the pharmaceutical composition, the nucleic acid molecule and/or the vector according to the present invention, wherein vascular normalization and/or improvement of vision is involved. In certain aspects, the invention provides the modified VEGF-A polypeptide, the pharmaceutical composition, the nucleic acid molecule and/or the vector according to the present invention, wherein reduction of tumor growth, reduction of metastatization or survival extension is involved.

In certain aspects, the invention provides the modified VEGF-A polypeptide, the pharmaceutical composition, the nucleic acid molecule and/or the vector according to the present invention, which is to be administered in combination with a further anti-angiogenic drug. In certain aspects, the invention provides the modified VEGF-A polypeptide, the pharmaceutical composition, the nucleic acid molecule and/or the vector according to the present invention, which is to be administered in combination with a with an anti-proliferative drug, an anticancer drug, a cytostatic drug, a cytotoxic drug and/or radiotherapy. In preferred aspects, the invention provides the modified VEGF-A polypeptide, the pharmaceutical composition, the nucleic acid molecule and/or the vector according to the present invention, which is to be administered in combination with bevacizumb (Roche), ranibizumab (Roche), pegaptanib (Macugen), aflibercept (Eylea) and/or verteporfin. In particular preferred aspects, the invention provides the modified VEGF-A polypeptide, the pharmaceutical composition, the nucleic acid molecule and/or the vector according to the present invention, which is to be administered in combination with bevacizumb (Roche) and/or ranibizumab (Roche).
In certain aspects, the modified VEGF-A, the pharmaceutical composition, the nucleic acid molecule and/or the vector according to the present invention is to be administered parenterally. In preferred aspects, the modified VEGF-A, the pharmaceutical composition, the nucleic acid molecule and/or the vector according to the present invention is to be administered into the eye. It is herein understood that, for example, the modified VEGF-A, the pharmaceutical composition, the nucleic acid molecule and/or the vector according to the present invention is injected into the vitreous humor of the eye.

The modified VEGF-A, the pharmaceutical composition, the nucleic acid molecule and/or the vector according to the present invention can be used in combination with other therapeutic agents. When a compound of the invention is used in combination with a second therapeutic agent active against the same disease, the dose of each compound may differ from that when the compound is used alone. The combination of a compound of the present invention with a second therapeutic agent may comprise the administration of the second therapeutic agent with the compound of the invention. Such an administration may comprise simultaneous/concomitant administration. However, also sequential/separate administration is envisaged, as also explained below.

Preferably, the second therapeutic agent to be administered in combination with the compounds of this invention is a further anti-angiogenic drug. Said further anti-angiogenic drug can be, for example, bevacizumb (Roche), ranibizumab (Roche), pegaptanib (Macugen), aflibercept (Eylea) and/or verteporfin.

In certain aspects, said anti-angiogenic drug is an anticancer drug. The anticancer drug to be administered in combination with the pharmaceutical composition, the nucleic acid molecule, the vector and/or the modified VEGF-A polypeptide according to the present invention may be: a tumor angiogenesis inhibitor (for example, a protease inhibitor, an epidermal growth factor receptor kinase inhibitor, or a vascular endothelial growth factor A receptor kinase inhibitor); a cytotoxic drug (for example, an antimetabolite, such as purine and pyrimidine analogue antimetabolites); an antimitotic agent (for example, a microtubule stabilizing drug or an antimitotic alkaloid); a platinum coordination complex; an anti-tumor antibiotic; an alkylating agent (for example, a nitrogen mustard or a nitrosourea); an endocrine agent (for example, an adrenocorticosteroid, an androgen, an anti-androgen, an estrogen, an anti-estrogen, an aromatase inhibitor, a gonadotropin-releasing hormone agonist, or a somatostatin analogue); or a compound that targets an enzyme or receptor that is overexpressed and/or otherwise involved in a specific metabolic pathway that is misregulated in the tumor cell (for example, ATP and GTP phosphodiesterase inhibitors, histone deacetylase inhibitors, protein kinase inhibitors (such as serine, threonine and tyrosine kinase inhibitors (for example, Abelson protein tyrosine kinase)) and the various growth factors, their receptors and corresponding kinase inhibitors (such as epidermal growth factor receptor kinase inhibitors, vascular endothelial growth factor receptor kinase inhibitors, fibroblast growth factor inhibitors, insulin-like growth factor receptor inhibitors and platelet-derived growth factor receptor kinase inhibitors)); methionine, aminopeptidase inhibitors, proteasome inhibitors, cyclooxygenase inhibitors (for example, cyclooxygenase-1 or cyclooxygenase-2 inhibitors) and topoisomerase inhibitors (for example, topoisomerase I or IV inhibitors or topoisomerase II inhibitors).

An alkylating agent which can be used as an anticancer drug in combination with the pharmaceutical composition, the nucleic acid molecule, the vector and/or the modified VEGF-A polypeptide of the present invention may be, for example, a nitrogen mustard (such as cyclophosphamide, mechlorethamine (chlormethine), uramustine, melphalan, chlorambucil, ifosfamide, bendamustine, or trofosfamide), a nitrosourea (such as carmustine, streptozocin, fotemustine, lomustine, nimustine, prednimustine, ranimustine, or semustine), an alkyl sulfonate (such as busulfan, mannosulfan, or treosulfan), an aziridine (such as hexamethylmelamine (altretamine), triethylenemelamine, ThioTEPA (N,N'N'-triethylenethiophosphoramide), carboquone, or triaziquone), a hydrazine (such as procarbazine), a triazene (such as dacarbazine), or an imidazotetrazines (such as temozolomide).

A platinum coordination complex which can be used as an anticancer drug in combination with the pharmaceutical composition, the nucleic acid molecule, the vector and/or the modified VEGF-A polypeptide of the present invention may be, for example, cisplatin, carboplatin, nedaplatin, oxaliplatin, satraplatin, or triplatin tetranitrate.

A cytotoxic drug which can be used as an anticancer drug in combination with the pharmaceutical composition, the nucleic acid molecule, the vector and/or the modified VEG polypeptide of the present invention may be, for example, an antimetabolite, including folic acid analogue antimetabolites (such as aminopterin, methotrexate, pemetrexed, or raltitrexed), purine analogue antimetabolites (such as cladribine, clofarabine, fludarabine, 6-mercaptopurine (including its prodrug form azathioprine), pentostatin, or 6-thioguanine), and pyrimidine analogue antimetabolites (such as cytarabine, decitabine, 5-fluorouracil (including its prodrug forms capecitabine and tegafur), floxuridine, gemcitabine, enocitabine, or sapacitabine).

An antimitotic agent which can be used as an anticancer drug in combination with the pharmaceutical composition, the nucleic acid molecule, the vector and/or the modified VEGF-A polypeptide of the present invention may be, for example, a taxane (such as docetaxel, larotaxel, ortataxel, paclitaxel/taxol, or tesetaxel), a Vinca alkaloid (such as vinblastine, vincristine, vinflunine, vindesine, or vinorelbine), an epothilone (such as epothilone A, epothilone B, epothilone C, epothilone D, epothilone E, or epothilone F) or an epothilone B analogue (such as ixabepilone/azaepothilone B).

An anti-tumor antibiotic which can be used as an anticancer drug in combination with the pharmaceutical composition, the nucleic acid molecule and/or the modified VEGF-A polypeptide of the present invention may be, for example, an anthracycline (such as aclarubicin, daunorubicin, doxorubicin, epirubicin, idarubicin, amrubicin, pirarubicin, valrubicin, or zorubicin), an anthracenedione (such as mitoxantrone, or pixantrone) or an anti-tumor antibiotic isolated from Streptomyces (such as actinomycin (including actinomycin D), bleomycin, mitomycin (including mitomycin C), or plicamycin).

A tyrosine kinase inhibitor which can be used as an anticancer drug in combination with the pharmaceutical composition, the nucleic acid molecule, the vector and/or the modified VEGF-A polypeptide of the present invention may be, for example, axitinib, bosutinib, cediranib, dasatinib, erlotinib, gefitinib, imatinib, lapatinib, lestaurtinib, nilotinib, semaxanib, sorafenib, sunitinib, or vandetanib.

A topoisomerase-inhibitor which can be used as an anticancer drug in combination with the pharmaceutical composition, the nucleic acid molecule, the vector and/or the modified VEGF-A polypeptide of the present invention may be, for example, a topoisomerase I inhibitor (such as irinotecan, topotecan, camptothecin, belotecan, rubitecan, or lamellarin D) or a topoisomerase II inhibitor (such as amsacrine, etoposide, etoposide phosphate, teniposide, or doxorubicin).

Further anticancer drugs may be used in combination with the pharmaceutical composition, the nucleic acid molecule, the vector and/or the modified VEGF-A polypeptide of the present invention. The anticancer drugs may comprise biological or chemical molecules, like TNF-related apoptosis-inducing ligand (TRAIL), tamoxifen, amsacrine, bexarotene, estramustine, irofulven, trabectedin, cetuximab, panitumumab, tositumomab, alemtuzumab, bevacizumab, edrecolomab, gemtuzumab, trastuzumab, pertuzumab, alvocidib, seliciclib, aminolevulinic acid, methyl aminolevulinate, efaproxiral, porfimer sodium, talaporfin, temoporfin, verteporfin, alitretinoin, tretinoin, anagrelide, arsenic trioxide, atrasentan, bortezomib, carmofur, celecoxib, demecolcine, elesclomol, elsamitrucin, etoglucid, lonidamine, lucanthone, masoprocol, mitobronitol, mitoguazone, mitotane, oblimersen, omacetaxine, sitimagene, ceradenovec, tegafur, testolactone, tiazofurine, tipifarnib, and vorinostat.

Also biological drugs, like antibodies, antibody fragments, antibody constructs (for example, single-chain constructs), and/or modified antibodies (like CDR-grafted antibodies, humanized antibodies, "full humanized" antibodies, etc.) directed against cancer or tumor markers/factors/cytokines involved in proliferative diseases can be employed in co-therapy approaches with the pharmaceutical composition, the nucleic acid molecule, the vector and/or the modified VEGF-A polypeptide of the invention. Such antibodies can be anti-VEGF-A antibodies, e.g., bevacizumab and/or ranibizumab. Further examples of such biological molecules are anti-HER2 antibodies (e.g. trastuzumab, Herceptin®), anti-CD20 antibodies (e.g. Rituximab, Rituxan®, MabThera®, Reditux®), anti-CD19/CD3 constructs (see, e.g., EP-B1 1071752) and anti-TNF antibodies (see, e.g., Taylor PC. Antibody therapy for rheumatoid arthritis. Curr Opin Pharmacol. 2003. 3(3):323-328). Further antibodies, antibody fragments, antibody constructs and/or modified antibodies to be used in co-therapy approaches with the pharmaceutical composition and/or the modified VEGF-A polypeptide of the invention can be found in Taylor PC. Curr Opin Pharmacol. 2003. 3(3):323-328; Roxana A. Maedica. 2006. 1(1):63-65.

Further, mineral supplements, vitamins and/or antioxidant-vitamins can employed in co-therapy approaches with the pharmaceutical composition, the nucleic acid molecule, the vector and/or the modified VEGF-A polypeptide of the invention. An exemplary anti-oxidant vitamin is carotenoid, vitamin C or vitamin E. An exemplary mineral is selenium or zinc.

The combinations referred to above may conveniently be presented for use in the form of a pharmaceutical formulation. The individual components of such combinations may be administered either sequentially or simultaneously/concomitantly in separate or combined pharmaceutical formulations by any convenient route. When administration is sequential, either the pharmaceutical composition, the nucleic acid molecule, the vector and/or the modified VEGF-A polypeptide of the present invention or the second therapeutic agent may be administered first. When administration is simultaneous, the combination may be administered either in the same or different pharmaceutical composition. When combined in the same formulation it will be appreciated that the two compounds must be stable and compatible with each other and the other components of the formulation. When formulated separately, they may be provided in any convenient formulation.

The pharmaceutical composition, the nucleic acid molecule, the vector and/or the modified VEGF-A polypeptide can also be administered in combination with physical therapy, such as radiotherapy. Radiotherapy may commence before, after, or simultaneously with administration of the compounds of the invention. For example, radiotherapy may commence 1-10 minutes, 1-10 hours or 24-72 hours after administration of the compounds. Yet, these time frames are not to be construed as limiting. The subject is exposed to radiation, preferably gamma radiation, whereby the radiation may be provided in a single dose or in multiple doses that are administered over several hours, days and/or weeks. Gamma radiation may be delivered according to standard radiotherapeutic protocols using standard dosages and regimens.

In certain aspects, the present invention relates to a nucleic acid molecule, a modified VEGF-A polypeptide and/or a pharmaceutically acceptable salt, solvate, or prodrug thereof, or a pharmaceutical composition comprising any of the aforementioned entities in combination with a pharmaceutically acceptable excipient, for use in the treatment or prevention of neovascular pathology or cancer, in particular the treatment or prevention of age related macular degeneration, wherein the compound or the pharmaceutical composition is to be administered in combination with a further anti-angiogenic drug such as an anticancer drug and/or in combination with radiotherapy.

The term "Angiogenesis" means that a vascular endothelial cell (EC) germinates from a pre-existing vessel and a capillary vessel is formed in a way that goes into a tissue. A formative process is the digestion of the vascular basement membrane by a protease, the migration/growth of a vascular EC, and the lumen formation. "Angiogenic disorder" is a vascular disease such as neovascular pathology/disorder or macular pathology, e.g., age related macular degeneration, diabetic retinopathy, or occlusion of retinal vein; arterial sclerosis, hypertonia, angina pectoris, obstructive arteriosclerosis, myocardial infarction, cerebral infarction, diabetic angiopathy or vascular malformation; inflammatory disease such as hepatitis, pneumonitis, glomerular nephritis, thyroiditis, osteitis, arthromeningitis, osteoclasia, chondrolysis, rheumatism, bronchial asthma, sarcoidosis, Crow-Fukase syndrome, pannus, allergic oedema, ulcers, hydroperitoneum, peritoneal sclerosis or tissular conglutination; reproductive system disease such as uterus dysfunction, placental dysfunction, ovarian hyperergasia or follicle cyst; central nervous system disease such as retinosis, cerebral apoplexy, vascular dementia or Alzheimer disease; cancer such as solid cancer, angiomatous, hemangioendothelioma, sarcomas, Kaposi's sarcoma or hematopoietic organic ulcer.

"Cancer", in accordance with the present invention, refers to a class of diseases or disorders characterized by uncontrolled division of cells and the ability of these to spread, either by direct growth into adjacent tissue through invasion, or by implantation into distant sites by metastasis, where cancer cells are transported through the bloodstream or lymphatic system. The tumorous disease can be any form of a cancer, a tumor or is chosen from pancreas cancer, breast cancer, epithelial cancer, hepatocellular carcinoma, cholangiocellular cancer, stomach cancer, colon cancer, prostate cancer, bladder cancer, tongue cancer, head and neck cancer, skin cancer (melanoma), a cancer of the urogenital tract, e.g., ovarian cancer, endometrial cancer, cervix cancer, and kidney cancer; lung cancer, gastric cancer, a cancer of the small intestine, liver cancer, gall bladder cancer, a cancer of the bile duct, esophagus cancer, a cancer of the salivary glands or a cancer of the thyroid gland.

The invention relates to a modified vascular endothelial growth factor A (VEGF-A) polypeptide for use in treating an angiogenic disorder. In certain aspects, the invention relates to a method of treatment for an angiogenic disorder comprising the step of administering to a subject in need of such treatment a pharmaceutical effective amount of a modified vascular endothelial growth factor A (VEGF-A) polypeptide. Further, the invention provides a method of treatment for angiogenic disorder and/or neovascular pathology and/or tumorous disease and/or cancer comprising the step of administering to a subject in need of such treatment a pharmaceutical active amount of the nucleic acid molecule of the present invention, or the modified VEGF-A polypeptide of the invention, the pharmaceutical composition of the present invention or as produced by the method as described herein.

The subject or patient to be treated in accordance with the invention may be an animal (e.g., a non-human animal), a vertebrate animal, a mammal, a rodent (e.g., a guinea pig, a hamster, a rat, a mouse), a murine (e.g., a mouse), a canine (e.g., a dog), a feline (e.g., a cat), a porcine (e.g., a pig), an equine (e.g., a horse), a primate, a simian (e.g., a monkey or ape), a monkey (e.g., a marmoset, a baboon), an ape (e.g., a gorilla, chimpanzee, orang-utan, gibbon), a human or a fish. In the context of this invention, it is particularly envisaged that animals are to be treated which are economically, agronomically or scientifically important. Scientifically important organisms include, but are not limited to, mice, rats, rabbits and fish (such as *Danio rerio* or zebrafish. Lower organisms such as, e.g., fruit flies like *Drosophila melanogaster* and nematodes like *Caenorhabditis elegans* may also be used in scientific approaches. Non-limiting examples of agronomically important animals are sheep, cattle and pigs, while, for example, cats and dogs may be considered as economically important animals. Preferably, the subject/patient is a mammal; more preferably, the subject/patient is a human or a non-human mammal (such as, e.g., a guinea pig, a hamster, a rat, a mouse, a rabbit, a dog, a cat, a horse, a monkey, an ape, a marmoset, a baboon, a gorilla, a chimpanzee, an orang-utan, a gibbon, a sheep, cattle, or a pig); most preferably, the subject/patient is a human.

The pharmaceutical effective amount can be higher than 10 mg/kg of body weight. Further, the pharmaceutical effective amount can be lower than 0.5 mg/kg of body weight. In particular preferred aspects, the invention provides a method of treatment according to the invention, wherein the pharmaceutical effective amount is the range of 0.5 to 10 mg/kg of body weight.

It is envisaged herein that the content of DNA of a preparation is different depending on a disease of therapeutic purpose, administration site, number of doses, desired duration of therapy, an age or body weight of a patient or the like and can be suitably adjusted. It is usually about 0.01 - 2000 mg and preferably 0.1 - 100 mg of DNA encoding a protein of this invention for a patient (the body weight is 60 kg).

The pharmaceutical composition will be formulated and dosed in a fashion consistent with good medical practice, taking into account the clinical condition of the individual patient, the site of delivery of the pharmaceutical composition, the method of administration, the scheduling of administration, and other factors known to practitioners. The "effective amount" of the pharmaceutical composition for purposes herein is thus determined by such considerations.

The skilled person knows that the effective amount of pharmaceutical composition administered to an individual will, inter alia, depend on the nature of the compound.

The administration of the herein provided compositions may, inter alia, comprise an administration twice daily, every day, every other day, every third day, every fourth day, every fifth day, once a week, once every second week, once every third week, once every month, or every two months etc.

For example, if said compound is a (poly)peptide or protein the total pharmaceutically effective amount of pharmaceutical composition administered parenterally per dose will be in the range of about 1 μg protein /kg/day to 15 mg protein /kg/day of patient body weight, although, as noted above, this will be subject to therapeutic discretion. More preferably, this dose is at least 0.01 mg protein/kg/day, and most preferably for humans between about 0.01 and 1 mg protein /kg/day. If given continuously, the pharmaceutical composition is typically administered at a dose rate of about 1 μg/kg/hour to about 50 μg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect. The particular amounts may be determined by conventional tests which are well known to the person skilled in the art.

Pharmaceutical compositions of the invention may be administered parenterally, ocular, orally, rectally, intracisternally, intravaginally, intraperitoneally, topically (as by powders, ointments, drops or transdermal patch) or bucally. In preferred embodiments, the pharmaceutical composition is administered parenterally. In particular preferred embodiments, the pharmaceutical composition is injected into the eye. In most preferred embodiments, the pharmaceutical composition is injected into the humor of the eye.

By "pharmaceutically acceptable carrier" or "pharmaceutically carrier" is meant a virus, a non-toxic solid, semisolid or liquid filler, diluent, encapsulating material or formulation auxiliary of any type. The term "parenteral" as used herein refers to modes of administration which include intravitreal, intravenous, intramuscular, intraperitoneal, intratracheal, intranasal, intrasternal, subcutaneous and intraarticular injection and infusion.

The pharmaceutical composition is also suitably administered by sustained release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Biopolymers 22:547-556 (1983)), poly (2-hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al., Id.) or poly-D-(-)-3-hydroxybutyric acid (EP 133,988). Sustained release pharmaceutical composition also include liposomally entrapped compound. Liposomes containing the pharmaceutical composition are prepared by methods known per se: DE 3,218,121; Epstein et al., Proc. Natl. Acad. Sci. (USA) 82:3688-3692 (1985); Hwang et al., Proc. Natl. Acad. Sci. (USA) 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal therapy.

For parenteral administration, the pharmaceutical composition is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation.

Generally, the formulations are prepared by contacting the components of the pharmaceutical composition uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) (poly)peptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

The components of the pharmaceutical composition to be used for therapeutic administration must be sterile. Sterility is readily accomplished by filtration through sterile filtration membranes (e.g., 0.2 micron membranes). Therapeutic components of the pharmaceutical composition generally are placed into a container having a sterile access port, for example, an intravenous solution bag or vial having a stopper pierceable by a hypodermic injection needle.

The components of the pharmaceutical composition ordinarily will be stored in unit or multidose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized compound(s) using bacteriostatic Water-for-Injection.

The term "nucleic acid molecule" in accordance with the present invention comprises coding and, wherever applicable, non-coding sequences (like promoters, enhancers etc.).

The terms "polypeptide", "(poly)peptide", "peptide" and "protein" are used herein interchangeably and refer to a polymer of two or more amino acids linked via amide bonds that are formed between an amino group of one amino acid and a carboxyl group of another amino acid. The amino acids comprised in the peptide or protein, which are also referred to as amino acid residues, may be selected from the 20 standard proteinogenic $\alpha$-amino acids (i.e., Ala, Arg, Asn, Asp, Cys, Glu, Gln, Gly, His, Ile, Leu, Lys, Met, Phe, Pro, Ser, Thr, Trp, Tyr, and Val) but also from non-proteinogenic and/or non-standard $\alpha$-amino acids (such as, e.g., ornithine, citrulline, homolysine, pyrrolysine, or 4--hydroxyproline) as well as $\beta$-Amino acids (e.g., $\beta$-alanine), $\gamma$-amino acids and $\delta$-amino acids. Preferably, the amino acid residues comprised in the peptide or protein are selected from $\alpha$-amino acids, more preferably from the 20 standard proteinogenic $\alpha$-amino acids (which can be present as the L-isomer or the D-isomer, and are preferably all present as the L-isomer). The peptide or protein may be unmodified or may be modified, e.g., at its N-terminus, at its C-terminus and/or at a functional group in the side chain of any of its amino acid residues (particularly at the side chain functional group of one or more Lys, His, Ser, Thr, Tyr, Cys, Asp, Glu, and/or Arg residues). Such modifications may include, e.g., the attachment of any of the protecting groups described for the corresponding functional groups in: Wuts PG & Greene TW, Greene's protective groups in organic synthesis, John Wiley & Sons, 2006. Such modifications may also include the covalent attachment of one or more polyethylene glycol (PEG) chains (forming a PEGylated peptide or protein), the glycosylation and/or the acylation with one or more fatty acids (e.g., one or more $C_{8-30}$ alkanoic or alkenoic acids; forming a fatty acid acylated peptide or protein). The amino acid residues comprised in the peptide or protein may, e.g., be present as a linear molecular chain (forming a linear peptide or protein) or may form one or more rings (corresponding to a cyclic peptide or protein). The peptide or protein may also form oligomers consisting of two or more identical or different molecules. As used herein, the term "domain" relates to any region/part of an amino acid sequence that is capable of autonomously adopting a specific structure and/or function. In the context of the present invention, accordingly, a "domain" may represent a functional domain or a structural domain.

The term "consensus sequence" or "consensus sequence motif' is the calculated order of most frequent residues, either nucleotide or amino acid, found at each position in a sequence alignment. It represents the results of a multiple sequence alignments in which related sequences are compared to each other and similar sequence motifs are calculated.

As used herein, the terms "comprising" and "including" or grammatical variants thereof are to be taken as specifying the stated features, integers, steps or components but do not preclude the addition of one or more additional features, integers, steps, components or groups thereof. This term encompasses the terms "consisting of' and "consisting essentially of."

Thus, the terms "comprising"/"including"/"having" mean that any further component (or likewise features, integers, steps and the like) can/may be present.

The term "consisting of' means that no further component (or likewise features, integers, steps and the like) is present.

The term "consisting essentially of" or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed composition, device or method.

Thus, the term "consisting essentially of" means those specific further components (or likewise features, integers, steps and the like) can be present, namely those not materially affecting the essential characteristics of the composition, device or method. In other words, the term "consisting essentially of" (which can be interchangeably used herein with the term "comprising substantially"), allows the presence of other components in the composition, device or method in addition to the mandatory components (or likewise features, integers, steps and the like), provided that the essential characteristics of the device or method are not materially affected by the presence of other components.

The term "method" refers to manners, means, techniques and procedures for accomplishing a given task including, but not limited to, those manners, means, techniques and procedures either known to, or readily developed from known manners, means, techniques and procedures by practitioners of the chemical, biological and biophysical arts.

The term "about" preferably refers to ±10% of the indicated numerical value, more preferably to ±5% of the indicated numerical value, and in particular to the exact numerical value indicated.

As used herein, the term "about" refers to ±10% of the indicated numerical value, and in particular to ±5% of the indicated numerical value. Whenever the term "about" is used, a specific reference to the exact numerical value indicated is also included. If the term "about" is used in connection with a parameter that is quantified in integers, such as the number of nucleotides in a given nucleic acid, the numbers corresponding to ±10% or ±5% of the indicated numerical value are to be rounded to the nearest integer. For example, the expression "about 25 nucleotides" refers to the range of 23 to 28 nucleotides, in particular the range of 24 to 26 nucleotides, and preferably refers to the specific value of 25 nucleotides.

The present invention is further described by reference to the following non-limiting figures and examples. Unless otherwise indicated, established methods of recombinant gene technology were used as described, for example, in Sambrook, Russell "Molecular Cloning, A Laboratory Manual", Cold Spring Harbor Laboratory, N.Y. (2001)) which is incorporated herein by reference in its entirety.

The present invention is further described by reference to the following non-limiting figures and examples.

Unless otherwise indicated, established methods of recombinant gene technology were used as described, for example, in Sambrook, Russell "Molecular Cloning, A Laboratory Manual", Cold Spring Harbor Laboratory, N.Y. (2001)) which is incorporated herein by reference in its entirety.

The Figures show:

Figure 1. Zebrafish ***vegfaa*** mutant allele shows severe vascular defects.
(a) The most abundant Vegfaa isoforms are Vegfaa-121 and Vegfaa-165. Vegfaa-121, 144 amino acids long, contains a signal peptide (SP) and a PDGF/VEGF domain (PDGF/VEGF). The Vegfaa-165 is characterized by NRP/heparin domain in between the PDGF/VEGF and C-terminal domain. (b) Sequence alignment of the wild-type (wt) and *vegfaa* Δ10 version of the *vegfaa* gene (181-190), in which the *vegfaa* Δ10 mutant comprises the deletion of ten nucleotides in exon 3. The *vegfaa* Δ10 mutation (encoding p.Glu61Metfs*54) results in a frameshift leading to a premature stop codon and therefore the loss of the knot motif (indicated by an asterisk). (c, right) Brightfield micrographs of 72 hpf wt and *vegfaa* -/- larvae in lateral view; (c, let) Confocal micrographs of 72 hpf *Tg(kdrl:HRAS-mCherry)* wt and *vegfaa* -/- larvae in lateral views; PCV refers to post cardinal vein.

**Figure 2. *vegfaa* mRNA injections rescue the viability of vegfaa-/- phenotype**
(left panel) Brightfield micrographs of 72 hpf uninjected, *vegfaa*-121 and -165 mRNA injected larvae in lateral views. Arrow points to area of pericardial edema (top). (middle panel) Confocal micrographs of 72 hpf *Tg(etsrp*:eGFP*)* uninjected *vegfaa*-/- and vegfaa-121 and -165 mRNA injected larvea in lateral views. (right panel) Magnified Confocal micrographs of 72 hpf *Tg(etsrp:*eGFP*)* uninjected and *vegfaa*-121 and -165 mRNA injected larvea in lateral views. Arrow points to area of collapse (top) and functional (middle, bottom) circulatory loop.

**Figure 3. Vegfaa F17A and K84A dominant negative isoforms block angiogenesis**
a) Structure of VEGF-A binding to VEGFR-2 (F17 and K84 are highlighted) (Muller et al., 1997, Structure) (b) Brightfield and (c) confocal micrographs of 72 hpf uninjected, *vegfaa*-121 F17A and K84A injected larvae. Arrow points to area of pericardial edema (d) Cartoon detailing the method used for transgenic transplant. H2B:RFP and Vegf-A-121 F17A expressing donor cells were transplanted into *Tg(etsrp:*eGFP*)* host embryos. (e) confocal micrographs of 48 hpf H2B-RFP and Vegfaa-121 F17A expressing cells transplanted in *Tg(etsrp:*eGFP*)* embyros.

**Figure 4. HRMA detects mutations in TALEN injected embryos**
Melting curves of the PCR products from genomic DNA samples isolated from *vegfaa* +/+, -/+ and -/-. The presence of mutations produces a homoduplex peak and a separate heteroduplex peak.

**Figure *5. pgfb and vegfbb* mRNA injections rescue the viability of vegfaa-/- phenotype.**
(left panel) Brightfield and (middle panel) confocal micrographs of 72 hpf, *pgfb* and *vegfbb* mRNA injected larvae in lateral views. Arrow points to the lack of pericardial edema. (right panel) Magnified Confocal micrographs of 72 hpf *Tg(etsrp:*eGFP*) pgfb* and *vegfbb* mRNA injected larvea in lateral views. Arrow points to area of functional circulatory loop.

The following non-binding Examples illustrate the invention.

## Example 1: The replacement of K84 and F17 by alanine in Vegfaa-121 abrogates the ability to rescue mutant zebrafish having a Vegfa null allele

### Materials and methods:

### Fish stocks

Zebrafish were maintained at 28°C and propagated as previously described. The following transgenic lines were used for this work: *Tg(etsrp:*eGFP*)* (Veldman et al., 2012), and *Tg(kdrl:HRAS*-mCherry*)*$^{s896}$ (Chi et al., 2008).

### Confocal Microscopy

A LSM 700 (Zeiss, Germany) and 780 were used for live imaging. Embryos and larvae were anesthetized with a low dose of tricaine, placed in a glass bottom Petri dish (MatTek) with a layer of 1.2% low melt agarose and imaged using a Plan-Apochromat 10x/0.45 and LCI Plan-Neofluar 25x/0.8.

### Plasmids

Total RNA extraction was performed using TRIZOL on whole zebrafish embryos or HUVEC cells (Life Technologies, Carlsbad, California) and used for cDNA synthesis using SuperScript second strand (Life Technologies). cDNAs encoding the *vegfaa* 121, 165, 189, vegfab, vegfbb, pgf, VEGF-A-121 and 165 were PCR-amplified using whole embryo cDNA as template. PCR fragments were ligated into the mammalian expression vector pcDNA3.1 myc-HIS tag between ecoRI and xhoI. All constructs were verified by sequencing. Table 6 shows the primers used for these experiments.

Table 6. Used primers and purpose thereof

| SEQ ID NO: | Primer and corres ponding purpose | |
|---|---|---|
| | These primers were used to PCR the zebrafish vegfaa-121 and -165 and clone it in a pcDNA 3.1 plasmid inside hindIII and ecoRI restriction sites. Normal PCR condition were used. | |
| SEQ ID NO: 42 | vegfaa fw<br>xpcDNA | ccaagcttATGAACTTGGTTGTTTATTTG |
| SEQ ID NO: 43 | vegfaa rv<br>xpcDNA | ccgaattcTTATCTTGGCTTTTCACAT |
| | These primers were used to mutagenize the aminoacids F17 and K84 in alanines. The standard protocol for site directed mutagenesis was followed for the PCR conditions. | |
| SEQ ID NO: 44 | F17A vegfaa fw | AATGATGTGATTCCCgcgATGGATGTGTATAAA |
| SEQ ID NO: 45 | F17A vegfaa rv | TTTATACACATCCATcgcGGGAATCACATCATT |
| SEQ ID NO: 46 | K84A zvegfaa fw | GGTGCTGCGGGTCGCGCAACGCGTATCGC |
| SEQ ID NO: 47 | K84A zvegfaa rv | GCGATACGCGTTGCGCGACCCGCAGCACC |
| | These primers were used to PCR the zebrafish vegfaa-121 and -165 introducing the mutation of the NRP-1 binding amino acid (R165E). The product was cloned in a pcDNA 3.1 plasmid in the hindIII-ecoRI restriction sites. | |

(continued)

| SEQ ID NO: | Primer and corres ponding purpose | |
|---|---|---|
| SEQ ID NO: 48 | R165E vegfaa-165fw | ccAAGCTTatgaacttggttgtttat |
| SEQ ID NO: 49 | R165E vegfaa-165rv | ccGAATTCttaTTCtggcttttcaca |
| | These primers were used to PCR the zebrafish Pgfb and Vegfbb and clone it in a pcDNA 3.1 plasmid inside hindIII and ecoRI restriction sites. Normal PCR condition were used. | |
| SEQ ID NO: 50 | zPgf fw | ccaagcttATGAGTTATTTGACGTCACTG |
| SEQ ID NO: 51 | zPgf rv | ccgaattTTACCTGCGGGGAGGCTGACAC |
| SEQ ID NO: 52 | Vegfbb fw | ccaagcttatgcggggttttcttctgttt |
| SEQ ID NO: 53 | Vegfbb rv | ccgaattttagtctctgaacctgcactg |

**Cell culture**

HUVEC (Human Umbilical Vein Endothelial Cells) cell cultures were maintained at 37 °C in 5% $CO_2$, 95% air in appropriate medium containing 10% fetal bovine serum, 100 units/ml penicillin and 100 μg/ml streptomycin.

**Genome editing**

TALENs were designed targeting *vegfaa* (Figure 4) using TALEN targeter (https://talent.cac.cornell.edu/) (Doyle et al., 2013) and constructed using Golden Gate assembly (Cermak et al., 2011). *vegfaa* exon 3 TALEN recognition sites are: TTTGTTAGGTGGCTGTC and TACGTGTGCTCGATCTC. Zebrafish embryos were injected into the cell at the one-cell stage with 100 pg total TALEN RNA.

**Genotyping**

Embryos or fin-clips were placed in PCR tubes, with 50 μl of elution buffer (10 mM Tris-Cl, pH 8.5) and 1 mg/ml Proteinase K added to each well and then incubated at 55° C for 2 hrs. The samples were then heated to 95°C for 10 min to inactivate Proteinase K. Primers were designed using primer3: http://biotools.umassmed.edu/bioapps/primer3_www.cgi. Eco Real-Time PCR System (Illumina, San Diego, CA) was used for PCR reaction and high-resolution melt analysis. DyNAmo SYBR green (Thermo Fisher Scientific, Waltham, MA) was used in these experiments. PCR reaction protocols was 95°C for 15 sec, then 40 cycles of 95°C for 2 sec, 60°C for 2 sec, and 72°C for 2 sec. Following the PCR, a high resolution melting curve was generated by collecting SYBR-green fluorescence data in the 65-95°C range. The analyses were performed on normalised derivative plots.

**Site-directed mutagenesis**

Site-directed mutagenesis was performed using PfuUltra Fusion HS (Agilent, Santa Clara, CA). PCR reaction protocol: 95° C for 1 minute, 95°C for 50 seconds, 60°C for 50 seconds, 68°C for 1 minute/kb of plasmid length x 18 times, 68°C for 7 minutes, 4 °C hold. 1μL of Dpn1 was added to the PCR reaction and incubated at 37 °C for 1 hour to digest parental DNA and transform into competent cells.

**Transplantation**

Wild-type embryos were injected with H2B-mCherry and vegfaa-121 F17A mRNAs at one-cell stage. About 20-40 cells were transferred to the margin of etsrp-GFP expressing wild-type host embryos which were subsequently grown at 28.5 °C until the indicated stages. Endothelial cell contribution of transplanted cells was assayed by virtue of mCherry (H2B-mCherry) expression in chimeric embryos using a dissection scope equipped with fluorescence (Axiocam, Zeiss).

**Results**

In zebrafish, *vegfaa* encodes for two major isoforms, a freely diffusible 121-amino acid isoform (Vegfa-121) and an extracellular matrix interacting 165-amino acid protein (Vegfa-165) (Figure 1) characterized by an NRP/heparin binding domain. In order to understand the role of *vegfaa* during zebrafish development, *vegfaa* mutants were generated using TALENs (Cermak et al., 2011) targeting exon 3. A frameshift allele, *vegfaa* Δ10 (Figure 1b) was identified and recovered using high resolution melt analysis (HRMA) (Figure 4). The *vegfaa* mutant sequence gives rise to a peptide of 119 amino acids (*vegfaa Δ10* or Q60fs59), in which the VEGF and PDGF knot motifs are lost (Fig.1b, bottom). The cysteines present in the knot motif are crucial for Vegfa dimerization, suggesting that the *vegfaa*$^{-/-}$ Δ10 is a null allele. *Vegfaa*$^{-/+}$ fish were crossed into the *Tg(kdrl: hRas-mCherry)* and *Tg(etsrp: GFP)* backgrounds. Both kdrl:hRas and etsrp:GFP backgrounds allow staining of the plasma membrane of endothelial cells. Brightfield micrographs show a severe pericardial edema and blood pooling in *vegfaa*$^{-/-}$ larvae at 72 hpf, due to the lack of circulation. (Figure 1c, left). Defective formation of the dorsal aorta (DA) and lack of intersegmental vessels (ISV) were evident in *vegfaa*$^{-/-}$ larvae at 72 hpf using confocal microscopy (Figure 1c, right). Moreover, *vegfaa*$^{-/-}$ mutants do not show defects in angioblast migration, however tubulogenesis is impaired. As in mice (Carmeliet et al., 1996, Ferrara et al., 1996), the deletion of Vegfa and its downstream signaling lead to dramatic defects in the vascular system formation and patterning followed by embryonic lethality. The vegfaa mutants die around day 6-7. We next wanted to address the question whether VEGF-A mRNA injections could rescue *vegfaa*$^{-/-}$ mutants. *vegfaa*-121 and *vegfaa-165* were cloned, in vitro transcribed and injected in *vegfaa*$^{-/-}$ embryos as previously described (Lawson et al., 2002). Brightfield micrographs show that mRNA injections of both *vegfaa* isoforms prevented the formation of a pericardial edema (Figure 2a) through the formation of a functional circulatory loop. Confocal image of *Tg(etsrp: GFP)* larvae show that DA differentiation in mutant embryos (Figure 2b) was visible only after injection of *Vegfaa*-121 and *Yegfaa-165* mRNA, but ISVs formation did not take place and the length of the circulatory loop was variable in the rescued larvae (Figure 2c). However, a functional circulatory loop was sufficient for the embryos survival (table 1) indicating that *vegfaa* is dispensable for late embryonic and larval development.

Table 1. Rescued embryos percentage after *vegfaa* 121 and 165 mRNA injection in *vegfaa* mutants.

| Ligand | Rescue percentage |
|---|---|
| Vegf-aa 121 | 57.7 |
| Vegf-aa 165 | 76.8 |

Rescued *vegfaa* homozygous mutants survive to become fertile adults making these a unique and powerful *in vivo* test tube.

Neuropillin-1 (Nrp-1) is a transmembrane receptor for the Vegfa-165 isoform and semaphorin family members (Herzog et al., 2011). Though NRP1 is essential for developmental angiogenesis, the contribution of NRP1-VEGF interaction to physiological and pathological angiogenesis is still not fully understood. Thus, structural determinants of Vegfaa binding to Vegf receptor 2 (Vegfr-2) and Nrp-1 *in vivo* was investigated. The available crystal structures and biochemical data from previous *in vitro* experiments (Muller et al., 1997, Brozzo et al., 2012 and Muller et al., 1997) and amino acid conservation among species were assessed to identify key residues for targeting. The codon for phenylalanine 17 (F17), aspartic acid 63 (D63), glutamic acid 64 (E64) or lysine 84 (K84) was mutagenized to encode for alanine and the resulting *vegfaa* alleles were *in vitro* transcribed. These isoforms were injected into eggs from *vegfaa*$^{-/-}$ incrosses and their rescue potential was measured as a percentage of embryos showing blood flow circulation and lacking pericardial edema. It was surprisingly found that the substitution of each of these two amino acids in *vegfaa*-121 abrogates the ability of Vegfa-121 to rescue *vegfaa*$^{-/-}$ mutants (table 2). Interestingly, the replacement of F17 and K84 by alanine in the context of the Vegfaa-165 isoform did not significantly affect *vegfaa-165* mediated rescue of the *vegfaa*$^{-/-}$ mutants (table 2).

In summary, wild-type Vegfaa-121 and Vegfaa-165 rescued vasculogenesis and angiogenesis in zebrafish with a *vegfaa* null-allele. It was surprisingly found that the replacement of K84 and F17 by alanine in the context of the Vegfa-121 abrogate the rescue ability of this mutant fish. Furthermore, theses mutations in the Vegfa-165 isoform do not eliminate the rescue ability of mutant zebrafish. These results prove the anti-angiogenic and anti-vasculogenic activity of Vegfa-121 K84A and Vegfa-121 F17A.

Table 2 Rescued embryos percentage after *vegfaa* 121 F17A or K84A and *vegfaa* 165 F17A or K84A or R165E mRNA injection in vegfaa mutants.

| Ligand | Rescue percentage |
|---|---|
| Vegf-aa 121 F17A | 0 |
| Vegf-aa 121 K84A | 0 |
| Vegf-aa 165 F17A | 88.9 |
| Vegf-aa 165 K84A | 88 |
| Vegf-aa R165E | 3 |

**Example 2:**

Vegfa-165 carrying the alanine mutations at K84 or F17 rescued the zebrafish with a Vegfa null allele. Yet, the mutated Vegfa-121 isoform (K84A or F17A) failed to rescue the mutant zebrafish. The Nrp-1 binding domain present in *vegfaa-165* could account for this functional difference. To further investigate this, the final arginine 165 (R165) in *vegfaa-165* was mutagenized to glutamic acid. R165 is responsible for Neuropilin-1 binding (Fantin et al., 2014). Surprisingly, the point mutation R165E eliminated *vegfaa-165* rescue indicating the importance of the Nrp-1 interaction over Vegfr-2 (Table 2). To understand whether Nrp-1 can signal independently of Vegfr-2, Vegfb (vegfbb) and Placental growth factor (Pgfb) were analyzed. These two molecules share common features like the ability to bind Nrp-1 (Makinen et al., 1999 and Christinger et al., 2004) but not Vegfr-2. (Olofsson et al., 1998 and Errico et al., 2004). Both isoforms were able to rescue the *vegfaa*$^{-/-}$mutant viability based on DA differentiation, formation of a functional circulatory loop and lack of pericardial edema (Figure 5 and Table 5).

Table 5. Rescued embryos percentage after *pgfb* and *vegfbb* mRNA injection in vegfaa mutants.

| Ligand | Rescue percentage |
|---|---|
| Pgf | 97.9 |
| Vegf-bb | 84.4 |

These data indicate that Nrp-1can promote vasculogenesis independently of Vegfr-2. Endothelial cell survival might be implemented through Nrp-1-Vegfr-1 interaction or Nrp-1 itself. However, F17A is predicted to prevent the interaction between Vegfaa and Vegfr-1 suggesting that the rescue of vegfaa-/- embryos is mediated by Nrp-1 itself or the interaction with other molecules (lack of the TK domain in VegfR1 does not lead to any vascular phenotype). In case of the longer isoforms encompassing the Nrp1-binding site, i.e., Vegfa-165, the regulation of vasculogenesis can be mediated solely by Vegfa-Nrp1 binding in zebrafish.

**Example 3: Vegfa-121 K84A and Vegfa-121 F17A are diffusible** antagonists **of endogenous Vegfa that act in a non-cell autonomous fashion**

Interestingly, injection of embryos, obtained from *vegfaa*$^{+/-}$ incrosses, with *vegfaa-121* F17A or K84A mutant isoforms leads to a phenotype that did not follow the Mendelian segregation seen in controls (uninjected). In fact, all heterozygotic embryos expressing these mutant Vegfa-121 isoforms showed the vascular abnormalities seen in the *vegfaa*$^{-/-}$ mutants. Thus, it might be speculated that the mutant isoforms form a dimer with wild-type Vegfa and thus trap the endogenous wild-type isoforms.

As Vegfa functions by dimerizing or aggregating its target receptors, it might be sufficient to change one of the two Vegfa molecules to modulate receptor binding and/or activity (Figure 3a). To further investigate this hypothesis, mRNA coding for the two *vegfaa-121* mutant isoforms, i.e., K84A or F17A, were injected in wild-type embryos. Importantly, brightfield micrographs of 72 hpf larvae injected with either 50 pg of vegfaa-121 F17A or K84A show pericardial edema, blood pooling and lack of circulation (Figure 3b). Furthermore, *Tg(etsrp:GFP)* embryos injected with these two mutant isoforms showed the vascular defects reminiscent of the a *vegfaa*$^{-/-}$ phenotype at 72 hpf (Figure 3c). The observed phenotype indicates that Vegfa-121 K84A or F17A acts antagonistically to endogenous wild-type Vegfa rendering the mutant isoforms as dominant-negative inhibitors.

In addition, transplantation experiments (Figure 3d) were performed to characterize the diffusion and cell type specificity of these mutant molecules. Wild-type embryos were injected with H2B-RFP and *vegfaa-121* F17A or K84A mRNA. The marker H2B-RFP allows the tracking of the expression of the modified Vegfa isoforms. Cells were then transplanted into *Tg(etsrp:GFP*) embryos. In both cases (F17A or K84A), donor embryos (Fig3d) show vascular defects in the proximity of red (transplanted) cells, indicating that these molecules work in a non-cell autonomous fashion. Moreover, the vascular defects are several micrometers (scale on the picture) far away from the red cells expressing *vegfaa-121* F17A, indicating the ability of this molecule to diffuse through the extracellular matrix. In summary, it was documented herein that Vegfa-121 K84A and Vegfa-121 F17A are anti-angiogenic/anti-vasculogenic molecules that inhibit endogenous wild-type Vegfa in a non-cell autonomous fashion.

These data indicate that this molecule might be helpful to inhibit pathological angiogenesis like age related macular degeneration (AMD). In the treatment of macular pathologies, it is in particular important to employ molecules that have high retinal penetration properties. Small proteins can readily diffuse from the vitreous of the eye to the back of the eye where the choroidal vasculature resides. Next to the full antibody Bevacizumab (150 kDa), the Fab fragment, Ranibizumab with a molecular weight of 50 kDa is employed in the treatment of AMD (Rosenfeld et al., 2006). Both of the commercial available molecules block Vegfa. The *vegfaa-121* mutant alleles described here are 15 and 5 times smaller than the full antibody and fragment version, respectively, making them potentially more diffusible and bio-available than the commercially available drugs. In summary, two new anti-angiogenic molecules with high *in vivo* diffusibility were identified.

The present invention refers to the following nucleotide and amino acid sequences:

The sequences provided herein are available in the NCBI database and can be retrieved from www.ncbi.nlm.nih.gov/sites/entrez?db=gene; Theses sequences also relate to annotated and modified sequences. The present invention also provides techniques and methods wherein homologous sequences, and variants of the concise sequences provided herein are used. Preferably, such "variants" are genetic variants.

**SEQ ID NO: 1**

Nucleotide sequence encoding Homo Sapiens VEGF-A isoform 121 (cDNA). The encoded polypeptide is herein also referred to wild-type VEGF-A. A corresponding mRNA molecule encoding such nucleotide sequence has this nucleotide sequence with the exception that the thymidine (T) residue(s) is/are replaced by (a) uracil (U) residue(s).

Gcacccatggcagaaggaggagggcagaatcatcacgaagtggtgaagttcatggatgtctatcagcgcagctactgccatccaatc
gagaccctggtggacatcttccaggagtaccctgatgagatcgagtacatcttcaagccatcctgtgtgcccctgatgcgatgcggggg
ctgctgcaatgacgagggcctggagtgtgtgcccactgaggagtccaacatcaccatgcagattatgcggatcaaacctcaccaaggc
cagcacataggagagatgagcttcctacagcacaacaaatgtgaatgcagaccaaagaaagatagagcaagacaagaaaaatgtgac
aagccgaggcggtga

**SEQ ID NO: 2**

Amino acid sequence of Homo Sapiens VEGF-A isoform 121. This VEGF-A is herein also referred to wild-type VEGF-A.

APMAEGGGQNHHEVVK**F**MDVYQRSYCHPIETLVDIFQEYPDEIEYIFKPSCVPLMRCG
GCCNDEGLECVPTEESNITMQIMRI**K**PHQGQHIGEMSFLQHNKCECRPKKDRARQEK
CDKPRR

**SEQ ID NO: 3**

Nucleotide sequence encoding modified Homo Sapiens VEGF-A isoform 121 (cDNA), wherein the nucleotides (nnn) 250 to 252 encode alanine. Exemplary codons encoding alanine are GCT, GCC, GCA, and GCG. The encoded polypeptide is herein also referred to VEGF-A 121 K84A. A corresponding mRNA molecule encoding such nucleotide sequence has this nucleotide sequence, with the exception that the thymidine (T) residue(s) is/are replaced by (a) uracil (U).

**nnn** = GCT, GCC, GCA, GCG

Gcacccatggcagaaggaggagggcagaatcatcacgaagtggtgaagttcatggatgtctatcagcgcagctactgccatccaatc gagaccctggtggacatcttccaggagtaccctgatgagatcgagtacatcttcaagccatcctgtgtgcccctgatgcgatgcggggg ctgctgcaatgacgagggcctggagtgtgtgcccactgaggagtccaacatcaccatgcagattatgcggatc**nnn**cctcaccaagg ccagcacataggagagatgagcttcctacagcacaacaaatgtgaatgcagaccaaagaaagatagagcaagacaagaaaaatgtga caagccgaggcggtga

**SEQ ID NO: 4**

Amino acid sequence of modified Homo Sapiens VEGF-A isoform 121. The modified VEGF-A has alanine in place of the lysine at the position 84 (bold). This modified VEGF-A is herein also referred to VEGF-A K84A.

APMAEGGGQNHHEVVKFMDVYQRSYCHPIETLVDIFQEYPDEIEYIFKPSCVPLMRCG GCCNDEGLECVPTEESNITMQIMRI**A**PHQGQHIGEMSFLQHNKCECRPKKDRARQEK CDKPRR

**SEQ ID NO: 5**

Nucleotide sequence encoding modified Homo Sapiens VEGF-A isoform 121 (cDNA), wherein the nucleotides (nnn) 49 to 51 encode alanine. Exemplary codons encoding alanine are GCT, GCC, GCA, and GCG. The encoded polypeptide is herein also referred to VEGF-A 121 F17A. A corresponding mRNA molecule encoding such nucleotide sequence has this nucleotide sequence, with the exception that the thymidine (T) residue(s) is/are replaced by (a) uracil (U).
**nnn** = GCT, GCC, GCA, GCG

Gcacccatggcagaaggaggagggcagaatcatcacgaagtggtgaag**nnn**atggatgtctatcagcgcagctactgccatccaat cgagaccctggtggacatcttccaggagtaccctgatgagatcgagtacatcttcaagccatcctgtgtgcccctgatgcgatgcgggg gctgctgcaatgacgagggcctggagtgtgtgcccactgaggagtccaacatcaccatgcagattatgcggatcaaacctcaccaagg ccagcacataggagagatgagcttcctacagcacaacaaatgtgaatgcagaccaaagaaagatagagcaagacaagaaaaatgtga caagccgaggcggtga

**SEQ ID NO: 6**

Amino acid sequence of modified Homo Sapiens VEGF-A isoform 121. The modified VEGF-A has alanine in place of the phenylalanine at the position 17 (bold). This modified VEGF-A is herein also referred to VEGF-A F17A.

APMAEGGGQNHHEVVK**A**MDVYQRSYCHPIETLVDIFQEYPDEIEYIFKPSCVPLMRC GGCCNDEGLECVPTEESNITMQIMRIKPHQGQHIGEMSFLQHNKCECRPKKDRARQE KCDKPRR

**SEQ ID NO: 7**

Nucleotide sequence encoding Danio rerio VEGF-A isoform 121 (cDNA). A corresponding mRNA molecule encoding such nucleotide sequence has this nucleotide sequence with the exception that the thymidine (T) residue(s) is/are replaced by (a) uracil (U) residue(s). This nucleotide sequence is herein also referred to Vegfaa-121.

GCCCACATACCCAAAGAAGGGGGAAAGAGCAAAAATGATGTGATTCCCTTCATG
GATGTGTATAAAAAGAGTGCGTGCAAGACCCGAGAGCTGCTGGTAGACATCATCC
AGGAGTATCCCGATGAGATCGAGCACACGTACATCCCGTCCTGTGTGGTTCTCAT
GCGCTGTGCAGGATGCTGTAATGATGAGGCGCTCGAATGCGTCCCGACAGAGACA
CGAAACGTCACTATGGAGGTGCTGCGGGTCAAGCAACGCGTATCGCAGCATAATT
TTCAGCTGAGTTTCACAGAACACACCAAGTGTGAATGCAGGCCAAAGGCAGAAGT
CAAAGCAAAGAAAGAAAAATGTGAAAAGCCAAGATGA

**SEQ ID NO: 8**

Amino acid sequence of Danio rerio VEGF-A isoform 121. This VEGF-A is herein also referred to wild-type VEGF-A or Vegfaa-121.

AHIPKEGGKSKNDVIPFMDVYKKSACKTRELLVDIIQEYPDEIEHTYIPSCVVLMRCAG
CCNDEALECVPTETRNVTMEVLRVKQRVSQHNFQLSFTEHTKCECRPKAEVKAKKE
KCEKPR

**SEQ ID NO: 9**

Nucleotide sequence encoding modified Danio rerio VEGF-A isoform 121 (cDNA), wherein the nucleotides (xxx) 250 to 252 encode alanine. Exemplary codons encoding alanine are GCT, GCC, GCA, and GCG. The encoded polypeptide is herein also referred to VEGF-A 121 K84A or Vegfaa-121 K84A. A corresponding mRNA molecule encoding such nucleotide sequence has this nucleotide sequence, with the exception that the thymidine (T) residue(s) is/are replaced by (a) uracil (U).
**nnn** = GCT, GCC, GCA, GCG

GCCCACATACCCAAAGAAGGGGGAAAGAGCAAAAATGATGTGATTCCCTTCATG
GATGTGTATAAAAAGAGTGCGTGCAAGACCCGAGAGCTGCTGGTAGACATCATCC
AGGAGTATCCCGATGAGATCGAGCACACGTACATCCCGTCCTGTGTGGTTCTCAT
GCGCTGTGCAGGATGCTGTAATGATGAGGCGCTCGAATGCGTCCCGACAGAGACA
CGAAACGTCACTATGGAGGTGCTGCGGGTC**nnn**CAACGCGTATCGCAGCATAATTT
TCAGCTGAGTTTCACAGAACACACCAAGTGTGAATGCAGGCCAAAGGCAGAAGTC
AAAGCAAAGAAAGAAAAATGTGAAAAGCCAAGATGA

**SEQ ID NO: 10**

Amino acid sequence of modified Danio rerio VEGF-A isoform 121. The modified VEGF-A has alanine in place of the lysine at the position 84 (bold). This modified VEGF-A is herein also referred to VEGF-A K84A or Vegfaa-121 K84A.

AHIPKEGGKSKNDVIPFMDVYKKSACKTRELLVDIIQEYPDEIEHTYIPSCVVLMRCAG
CCNDEALECVPTETRNVTMEVLRV**A**QRVSQHNFQLSFTEHTKCECRPKAEVKAKKE
KCEKPR

**SEQ ID NO: 11**

Nucleotide sequence encoding modified Danio rerio VEGF-A isoform 121 (cDNA), wherein the nucleotides (nnn) 49 to 51 encode alanine. Exemplary codons encoding alanine are GCT, GCC, GCA, and GCG. The encoded polypeptide is herein also referred to VEGF-A 121 F17A or Vegfaa-121 F17A. A corresponding mRNA molecule encoding such nucleotide sequence has this nucleotide sequence, with the exception that the thymidine (T) residue(s) is/are replaced by (a) uracil (U).

GCCCACATACCCAAAGAAGGGGGAAAGAGCAAAAATGATGTGATTCCC**nnn**ATGG
ATGTGTATAAAAAGAGTGCGTGCAAGACCCGAGAGCTGCTGGTAGACATCATCCA
GGAGTATCCCGATGAGATCGAGCACACGTACATCCCGTCCTGTGTGGTTCTCATGC
GCTGTGCAGGATGCTGTAATGATGAGGCGCTCGAATGCGTCCCGACAGAGACACG
AAACGTCACTATGGAGGTGCTGCGGGTCAAGCAACGCGTATCGCAGCATAATTTT
CAGCTGAGTTTCACAGAACACACCAAGTGTGAATGCAGGCCAAAGGCAGAAGTC
AAAGCAAAGAAAGAAAAATGTGAAAAGCCAAGATGA

**SEQ ID NO: 12**

Amino acid sequence of modified Danio rerio VEGF-A isoform 121. The modified VEGF-A has alanine in place of the phenylalanine at the position 17 (bold). This modified VEGF-A is herein also referred to VEGF-A K84A or Vegfaa-121 F17A.

AHIPKEGGKSKNDVIP**A**MDVYKKSACKTRELLVDIIQEYPDEIEHTYIPSCVVLMRCA
GCCNDEALECVPTETRNVTMEVLRVKQRVSQHNFQLSFTEHTKCECRPKAEVKAKK
EKCEKPR

**SEQ ID NO: 13**

Amino acid sequence of modified Homo Sapiens VEGF-A isoform 121. The modified VEGF-A has alanine in place of the lysine at the position 84 and the phenylalanine at the position 17 (bold). This modified VEGF-A is herein also referred to VEGF-A K84A and F17A.

APMAEGGGQNHHEVVK**A**MDVYQRSYCHPIETLVDIFQEYPDEIEYIFKPSCVPLMRC
GGCCNDEGLECVPTEESNITMQIMRI**A**PHQGQHIGEMSFLQHNKCECRPKKDRARQE
KCDKPRR

**SEQ ID NO: 14**

Amino acid sequence of modified Danio Rerio VEGF-A isoform 121. The modified VEGF-A has alanine in place of the lysine at the position 84 and the phenylalanine at the position 17 (bold). This modified VEGF-A is herein also referred to Vegfaa-121 K84A and F17A.

AHIPKEGGKSKNDVIP**A**MDVYKKSACKTRELLVDIIQEYPDEIEHTYIPSCVVLMRCA
GCCNDEALECVPTETRNVTMEVLRV**A**QRVSQHNFQLSFTEHTKCECRPKAEVKAKK
EKCEKPR

**SEQ ID NO: 15**

Nucleotide sequence encoding Homo Sapiens VEGF-A isoform 121 with the signal peptide (cDNA). A corresponding mRNA molecule encoding such nucleotide sequence has this nucleotide sequence with the exception that the thymidine (T) residue(s) is/are replaced by (a) uracil (U) residue(s).

atgaactttctgctgtcttgggtgcattggagccttgccttgctgctctacctccaccatgccaagtggtcccaggctgcacccatggcaga
aggaggagggcagaatcatcacgaagtggtgaagttcatggatgtctatcagcgcagctactgccatccaatcgagaccctggtggac
atcttccaggagtaccctgatgagatcgagtacatcttcaagccatcctgtgtgcccctgatgcgatgcgggggctgctgcaatgacgag
ggcctggagtgtgtgcccactgaggagtccaacatcaccatgcagattatgcggatcaaacctcaccaaggccagcacataggagag
atgagcttcctacagcacaacaaatgtgaatgcagaccaaagaaagatagagcaagacaagaaaaatgtgacaagccgaggcggtg
a

**SEQ ID NO: 16**

Amino acid sequence of Homo Sapiens VEGF-A isoform 121 with the signal peptide.

MNFLLSWVHWSLALLLYLHHAKWSQAAPMAEGGGQNHHEVVK**F**MDVYQRSYCHP
IETLVDIFQEYPDEIEYIFKPSCVPLMRCGGCCNDEGLECVPTEESNITMQIMRI**K**PHQG
QHIGEMSFLQHNKCECRPKKDRARQEKCDKPRR

**SEQ ID NO: 17**

Nucleotide sequence encoding modified Homo Sapiens VEGF-A isoform 121 with the signal peptide (cDNA), wherein the nucleotides 328 to 330 encode alanine (bold). A corresponding mRNA molecule encoding such nucleotide sequence has this nucleotide sequence, with the exception that the thymidine (T) residue(s) is/are replaced by (a) uracil (U).

atgaactttctgctgtcttgggtgcattggagccttgccttgctgctctacctccaccatgccaagtggtcccaggctgcacccatggcaga
aggaggagggcagaatcatcacgaagtggtgaagttcatggatgtctatcagcgcagctactgccatccaatcgagaccctggtggac
atcttccaggagtaccctgatgagatcgagtacatcttcaagccatcctgtgtgcccctgatgcgatgcgggggctgctgcaatgacgag
ggcctggagtgtgtgcccactgaggagtccaacatcaccatgcagattatgcggatc**GCA**cctcaccaaggccagcacataggag
agatgagcttcctacagcacaacaaatgtgaatgcagaccaaagaaagatagagcaagacaagaaaaatgtgacaagccgaggcgg
tga

**SEQ ID NO: 18**

Amino acid sequence of modified Homo Sapiens VEGF-A isoform 121 with the signal peptide. The modified VEGF-A has the inventive alanine in place of the lysine at the position 110 (corresponding to position 84 in SEQ ID NO: 2) (bold).

MNFLLSWVHWSLALLLYLHHAKWSQAAPMAEGGGQNHHEVVKFMDVYQRSYCHP
IETLVDIFQEYPDEIEYIFKPSCVPLMRCGGCCNDEGLECVPTEESNITMQIMRI**A**PHQG
QHIGEMSFLQHNKCECRPKKDRARQEKCDKPRR

**SEQ ID NO: 19**

Nucleotide sequence encoding modified Homo Sapiens VEGF-A isoform 121 with the signal peptide (cDNA), wherein the nucleotides 127 to 129 encode alanine (bold). A corresponding mRNA molecule encoding such nucleotide sequence has this nucleotide sequence, with the exception that the thymidine (T) residue(s) is/are replaced by (a) uracil (U).

atgaactttctgctgtcttgggtgcattggagccttgccttgctgctctacctccaccatgccaagtggtcccaggctgcacccatggcaga
aggaggagggcagaatcatcacgaagtggtgaag**GCC**atggatgtctatcagcgcagctactgccatccaatcgagaccctggtg
gacatcttccaggagtaccctgatgagatcgagtacatcttcaagccatcctgtgtgcccctgatgcgatgcgggggctgctgcaatgac
gagggcctggagtgtgtgcccactgaggagtccaacatcaccatgcagattatgcggatcaaacctcaccaaggccagcacatagga
gagatgagcttcctacagcacaacaaatgtgaatgcagaccaaagaaagatagagcaagacaagaaaaatgtgacaagccgaggcg
gtga

**SEQ ID NO: 20**

Amino acid sequence of modified Homo Sapiens VEGF-A isoform 121 with the signal peptide. The modified VEGF-A has the inventive alanine in place of the phenylalanine at the position 43 (corresponding to position 17 in SEQ ID NO: 2) (bold).

MNFLLSWVHWSLALLLYLHHAKWSQAAPMAEGGGQNHHEVVK**A**MDVYQRSYCHP
IETLVDIFQEYPDEIEYIFKPSCVPLMRCGGCCNDEGLECVPTEESNITMQIMRIKPHQG
QHIGEMSFLQHNKCECRPKKDRARQEKCDKPRR

**SEQ ID NO: 21**

Nucleotide sequence encoding Danio rerio VEGF-A isoform 121 with the signal peptide (cDNA). A corresponding mRNA molecule encoding such nucleotide sequence has this nucleotide sequence with the exception that the thymidine (T) residue(s) is/are replaced by (a) uracil (U) residue(s). This nucleotide sequence is herein also referred to Vegfaa-121.

ATGAACTTGGTTGTTTATTTGATACAGTTATTTCTCGCGGCTCTCCTCCATCTGTCT
GCTGTAAAGGCTGCCCACATACCCAAAGAAGGGGGAAAGAGCAAAAATGATGTG
ATTCCCTTCATGGATGTGTATAAAAAGAGTGCGTGCAAGACCCGAGAGCTGCTGG
TAGACATCATCCAGGAGTATCCCGATGAGATCGAGCACACGTACATCCCGTCCTG
TGTGGTTCTCATGCGCTGTGCAGGATGCTGTAATGATGAGGCGCTCGAATGCGTCC
CGACAGAGACACGAAACGTCACTATGGAGGTGCTGCGGGTCAAGCAACGCGTAT
CGCAGCATAATTTTCAGCTGAGTTTCACAGAACACACCAAGTGTGAATGCAGGCC
AAAGGCAGAAGTCAAAGCAAAGAAAGAAAAATGTGAAAAGCCAAGATGA

**SEQ ID NO: 22**

Amino acid sequence of Danio rerio VEGF-A isoform 121 with the signal peptide. This VEGF-A is herein also referred to VEGF-A or Vegfaa-121.

MNLVVYLIQLFLAALLHLSAVKAAHIPKEGGKSKNDVIP**F**MDVYKKSACKTRELLVD
IIQEYPDEIEHTYIPSCVVLMRCAGCCNDEALECVPTETRNVTMEVLRV**K**QRVSQHNF
QLSFTEHTKCECRPKAEVKAKKERCEKPR

**SEQ ID NO: 23**

Nucleotide sequence encoding modified Danio rerio VEGF-A isoform 121 with the signal peptide (cDNA), wherein the nucleotides 319 to 321 encode alanine (bold). A corresponding mRNA molecule encoding such nucleotide sequence has this nucleotide sequence, with the exception that the thymidine (T) residue(s) is/are replaced by (a) uracil (U).

ATGAACTTGGTTGTTTATTTGATACAGTTATTTCTCGCGGCTCTCCTCCATCTGTCT
GCTGTAAAGGCTGCCCACATACCCAAAGAAGGGGGAAAGAGCAAAAATGATGTG
ATTCCCTTCATGGATGTGTATAAAAAGAGTGCGTGCAAGACCCGAGAGCTGCTGG
TAGACATCATCCAGGAGTATCCCGATGAGATCGAGCACACGTACATCCCGTCCTG
TGTGGTTCTCATGCGCTGTGCAGGATGCTGTAATGATGAGGCGCTCGAATGCGTCC
CGACAGAGACACGAAACGTCACTATGGAGGTGCTGCGGGTC**GCG**CAACGCGTAT
CGCAGCATAATTTTCAGCTGAGTTTCACAGAACACACCAAGTGTGAATGCAGGCC
AAAGGCAGAAGTCAAAGCAAAGAAAGAAAAATGTGAAAAGCCAAGATGA

**SEQ ID NO: 24**

Amino acid sequence of modified Danio rerio VEGF-A isoform 121 with the signal peptide. The modified VEGF-A has the inventive alanine in place of the lysine at the position 107 (corresponding to position 84 in SEQ ID NO: 8) (bold).

MNLVVYLIQLFLAALLHLSAVKAAHIPKEGGKSKNDVIPFMDVYKKSACKTRELLVD
IIQEYPDEIEHTYIPSCVVLMRCAGCCNDEALECVPTETRNVTMEVLRV**A**QRVSQHNF
QLSFTEHTKCECRPKAEVKAKKERCEKPR

**SEQ ID NO: 25**

Nucleotide sequence encoding modified Danio rerio VEGF-A isoform 121 with the signal peptide (cDNA), wherein the nucleotides 118 to 120 (bold) encode alanine. A corresponding mRNA molecule encoding such nucleotide sequence has this nucleotide sequence, with the exception that the thymidine (T) residue(s) is/are replaced by (a) uracil (U).

ATGAACTTGGTTGTTTATTTGATACAGTTATTTCTCGCGGCTCTCCTCCATCTGTCT
GCTGTAAAGGCTGCCCACATACCCAAAGAAGGGGAAAGAGCAAAAATGATGTG
ATTCCC**GCC**ATGGATGTGTATAAAAAGAGTGCGTGCAAGACCCGAGAGCTGCTGG

TAGACATCATCCAGGAGTATCCCGATGAGATCGAGCACACGTACATCCCGTCCTG
TGTGGTTCTCATGCGCTGTGCAGGATGCTGTAATGATGAGGCGCTCGAATGCGTCC
CGACAGAGACACGAAACGTCACTATGGAGGTGCTGCGGGTCAAGCAACGCGTAT
CGCAGCATAATTTTCAGCTGAGTTTCACAGAACACACCAAGTGTGAATGCAGGCC
AAAGGCAGAAGTCAAAGCAAAGAAAGAAAAATGTGAAAA
GCCAAGATGA

**SEQ ID NO: 26**

Amino acid sequence of modified Danio rerio VEGF-A isoform 121 with the signal peptide. The modified VEGF-A has the inventive alanine in place of the phenylalanine at the position 40 (corresponding to position 17 in SEQ ID NO: 8) (bold).

MNLVVYLIQLFLAALLHLSAVKAAHIPKEGGKSKNDVIP**A**MDVYKKSACKTRELLVD
IIQEYPDEIEHTYIPSCVVLMRCAGCCNDEALECVPTETRNVTMEVLRVKQRVSQHNF
QLSFTEHTKCECRPKAEVKAKKERCEKPR

**SEQ ID NO: 27**

Amino acid sequence of Homo Sapiens Heparin binding domain/Nrp-1 binding domain.
NPCGPCSERRKHLFVQDPQTCKCSCKNTDSRCKARQLELNERTC

**SEQ ID NO: 28**

Amino acid sequence of Danio rerio Heparin binding domain/Nrp-1 binding domain.
NHCEPCSERRKRLYVQDPLTCKCSCKFTQMQCKSRQLELNERTC

**SEQ ID NO: 29**

Amino acid sequence of Homo Sapiens VEGF-A isoform 165.

MNFLLSWVHWSLALLLYLHHAKWSQAAPMAEGGGQNHHEVVKFMDVYQRSYCHP
IETLVDIFQEYPDEIEYIFKPSCVPLMRCGGCCNDEGLECVPTEESNITMQIMRIKPHQG
QHIGEMSFLQHNKCECRPKKDRARQENPCGPCSERRKHLFVQDPQTCKCSCKNTDSR
CKARQLELNERTCRCDKPRR

**SEQ ID NO: 30**

Amino acid sequence of Danio rerio VEGF-A isoform 165.

MNLVVYLIQLFLAALLHLSAVKAAHIPKEGGKSKNDVIPFMDVYKKSACKTRELLVD
IIQEYPDEIEHTYIPSCVVLMRCAGCCNDEALECVPTETRNVTMEVLRVKQRVSQHNF
QLSFTEHTKCECRPKAEVKAKKENHCEPCSERRKRLYVQDPLTCKCSCKFTQMQCKS
RQLELNERTCRCEKPR

**SEQ ID NO: 31**

Amino acid sequence of Homo Sapiens VEGF-A isoform 111.

MNFLLSWVHWSLALLLYLHHAKWSQAAPMAEGGGQNHHEVVKFMDVYQRSYCHP
IETLVDIFQEYPDEIEYIFKPSCVPLMRCGGCCNDEGLECVPTEESNITMQIMRIKPHQG
QHIGEMSFLQHNKCECRCDKPRR

**SEQ ID NO: 32**

Mutated endogenous vascular growth factor A (VEGF-A) herein also referred to modified Zebrafish vegfaa delta 10.

ATGAACTTGGTTGTTTATTTGATACAGTTATTTCTCGCGGCTCTCCTCCATCTGTCT
GCTGTAAAGGCTGCCCACATACCCAAAGAAGGGGGAAAGAGCAAAAATGATGTG
ATTCCCTTCATGGATGTGTATAAAAAGAGTGCGTGCAAGACCCGAGAGCTGCTGG
TAGACATCATCCAGATGAGATCGAGCACACGTACATCCCGTCCTGTGTGGTTCTCA
TGCGCTGTGCAGGATGCTGTAATGATGAGGCGCTCGAATGCGTCCCGACAGAGAC
ACGAAACGTCACTATGGAGGTGCTGCGGGTCAAGCAACGCGTATCGCAGCATAAT
TTTCAGCTGAGTTTCACAGAACACACCAAGTGTGAATGCAGGCCAAAGGCAGAAG
TCAAAGCAAAGAAAGAAAAATGTGAAAAGCCAAGATGA

**SEQ ID NO: 33**

Gene product of mutated endogenous vascular growth factor A (VEGF-A).

MNLVVYLIQLFLAALLHLSAVKAAHIPKEGGKSKNDVIPFMDVYKKSACKTRELLVD IIQMRSSTRTSRPVWFSCAVQDAVMMRRSNASRQRHETSLWRCCGSSNAYRSIIFS

**SEQ ID NO: 34**

Amino acid stretch of Homo Sapiens VEGF-A.
MRIKPHQ

**SEQ ID NO: 35**

Amino acid stretch of mouse VEGF-A.
MRIKPHQ

**SEQ ID NO: 36**

Amino acid stretch of Danio rerio VEGF-A.
LRVKQRV

**SEQ ID NO: 37**

Amino acid stretch of frog VEGF-A.
MKIKPHI

**SEQ ID NO: 38**

Amino acid stretch of Homo Sapiens VEGF-A.
WKF MDV

**SEQ ID NO: 39**

Amino acid stretch of mouse VEGF-A.
VIKFMDV

**SEQ ID NO: 40**

Amino acid stretch of Danio rerio VEGF-A.
VIPFMDV

**SEQ ID NO: 41**

Amino acid stretch of frog VEGF-A.
VVKFLKV

**SEQ ID NO: 42**

vegfaa fw xpcDNA
ccaagcttATGAACTTGGTTGTTTATTTG

**SEQ ID NO: 43**

vegfaa rv xpcDNA
ccgaattcTTATCTTGGCTTTTCACAT

**SEQ ID NO: 44**

F17A vegfaa fw

AATGATGTGATTCCCgcgATGGATGTGTATAAA

**SEQ ID NO: 45**

F17A vegfaa rv
TTTATACACATCCATcgcGGGAATCACATCATT

**SEQ ID NO: 46**

K84A zvegfaa fw
GGTGCTGCGGGTCGCGCAACGCGTATCGC

**SEQ ID NO: 47**

K84A zvegfaa rv
GCGATACGCGTTGCGCGACCCGCAGCACC

**SEQ ID NO: 48**

R165E vegfaa-165fw
ccAAGCTTatgaacttggttgtttat

**SEQ ID NO: 49**

R165E vegfaa-165rv
ccGAATTCttaTTCtggcttttcaca

**SEQ ID NO: 50**

zPgf fw
ccaagcttATGAGTTATTTGACGTCACTG

**SEQ ID NO: 51**

zPgf rv
ccgaattTTACCTGCGGGGAGGCTGACAC

**SEQ ID NO: 52**

Vegfbb fw
ccaagcttatgcggggttttcttctgttt

**SEQ ID NO: 53**

Vegfbb rv
ccgaattttagtctctgaacctgcactg

All references cited herein are fully incorporated by reference. Having now fully described the invention, it will be understood by a person skilled in the art that the invention may be practiced within a wide and equivalent range of conditions, parameters and the like, without affecting the spirit or scope of the invention or any embodiment thereof.

N. Ferrara et al., (2003) Nature Medicine, 9, 669 - 676
Yves A. Muller et al. (1997) Proc Natl Acad Sci USA. 94, 7192-7197
Maurice S. Brozzo et al. (2012) Blood. 16, 1781-1788
T. Cermak, et al. (2011) Nucleic Acids Res. 39, e82
N. D. Lawson, et al. (2002) Dev Cell. 3, 127-136.
B. Herzog, et al. (2011) Development. 141, 556-562
Yves A. Muller et al. (2014) Development. 141, 556-562
T. Makinen et al. (1999) J Biol Chem. 274, 21217-21222

Hans W. Christinger, et al. (2004) J Biol Chem. 279,10382-10388
B. Olofsson et al. (1998) Proc. Natl. Acad. Sci. USA 95, 11709- USA
M. Errico et al. (2004) J Biol Chem. 279, 43929-43939
Philip J. Rosenfeld et al. (2006) N Engl J Med 355, 1419-1431
M. B. Veldman and S.Lin. (2012) Circ Res. 110, 220-229
N. C. Chi et al. (2008) Plos Biol. 13;6(5):e109
E. L. Doyle et al. (2013) Plos One 8, e82120
J J Chen, et al. (2011) Eye 25, 1504-1511; doi:10.1038/eye.2011.225; published online 16 September 2011

## Claims

1. A modified vascular endothelial growth factor A (VEGF-A) polypeptide for use in treating an angiogenic disorder, wherein the modified vascular endothelial growth factor A (VEGF-A) is the VEGF-A 121 isoform,
wherein said modified vascular endothelial growth factor A (VEGF-A) comprises a hydrophobic amino acid in place of the lysine at the position corresponding to position 84 of the wild-type VEGF-A as shown in SEQ ID NO: 2, and/or wherein said modified vascular endothelial growth factor A (VEGF-A) comprises a hydrophobic amino acid in place of the phenylalanine at the position corresponding to position 17 of the wild-type VEGF-A as shown in SEQ ID NO: 2.

2. The polypeptide for use of claim 1, wherein said angiogenic disorder is neovascular pathology.

3. The polypeptide for use of claim 1 or 2, wherein said neovascular pathology is selected from the group of age related macular degeneration, neovascular age related macular degeneration, diabetic macular edema, high risk-corneal transplantation and neovascular glaucoma.

4. The polypeptide for use of any one of claims 1 to 3, wherein said hydrophobic amino acid is selected from the group consisting of alanine, valine, isoleucine, leucine and methionine.

5. The polypeptide for use of any one of claims 1 to 4, wherein said hydrophobic amino acid is alanine.

6. The polypeptide for use of any one of claims 1 to 5, wherein said modified vascular endothelial growth factor A (VEGF-A) is selected from the group consisting of:

   (a) a polypeptide encoded by a nucleic acid molecule given in SEQ ID NO: 1,
   wherein the nucleotides AAA at position 250 to 252 of SEQ ID NO: 1 are replaced by nucleotides encoding said hydrophobic amino acid, and/or
   wherein the nucleotides TTC at position 49 to 51 of SEQ ID NO: 1 are replaced by nucleotides encoding said hydrophobic amino acid;
   (b) a polypeptide given in SEQ ID NO: 2, wherein the lysine residue corresponding to position 84 of SEQ ID NO: 2; and/or wherein the phenylalanine corresponding to position 17 of SEQ ID NO: 2 is replaced by said hydrophobic amino acid;
   (c) a polypeptide encoded by a nucleic acid molecule hybridizing under stringent conditions to the complementary strand of a nucleic acid molecule encoding the polypeptide as defined in (a) or (b);
   (d) a polypeptide having at least 55% identity to the polypeptide of any one of (a) to (c) and functioning as an inhibitor of angiogenesis; and
   (e) a polypeptide that functions as an inhibitor of angiogenesis comprising an amino acid sequence encoded by a nucleic acid molecule being degenerate as a result of the genetic code to the nucleotide sequence of a nucleic acid molecule as defined in any one of (a), (c) and (d).

7. The polypeptide for use of any one of claims 1 to 6, wherein said modified vascular endothelial growth factor A (VEGF-A) is selected from the group consisting of SEQ ID NO: 4, SEQ ID NO: 6, SEQ ID NO: 10, SEQ ID NO: 12, SEQ ID NO: 13 and SEQ ID NO: 14.

8. A modified vascular endothelial growth factor A (VEGF-A) polypeptide, wherein the modified vascular endothelial growth factor A (VEGF-A) is the VEGF-A 121 isoform, wherein said modified vascular endothelial growth factor A (VEGF-A) comprises a hydrophobic amino acid in place of the lysine at the position corresponding to position 84 of the wild-type VEGF-A as shown in SEQ ID NO: 2, and/or wherein said modified vascular endothelial growth factor A (VEGF-A) comprises a hydrophobic amino acid in place of the phenylalanine at the position corresponding to position 17 of the wild-type VEGF-A as shown in SEQ ID NO: 2.

**9.** The polypeptide of claim 8, wherein said hydrophobic amino acid is selected from the group consisting of alanine, valine, isoleucine, leucine and methionine.

**10.** The polypeptide of claim 8 or 9, wherein said hydrophobic amino acid is alanine.

**11.** The polypeptide of any one of claims 8 to 10, wherein said modified vascular endothelial growth factor A (VEGF-A) is selected from the group consisting of:

(a) a polypeptide encoded by a nucleic acid molecule given in SEQ ID NO: 1,
wherein the nucleotides AAA at position 250 to 252 of SEQ ID NO: 1 are replaced by nucleotides encoding said hydrophobic amino acid, and/or wherein the nucleotides TTC at position 49 to 51 of SEQ ID NO: 1 are replaced by nucleotides encoding said hydrophobic amino acid;
(b) a polypeptide given in SEQ ID NO: 2, wherein the lysine residue corresponding to position 84 of SEQ ID NO: 2; and/or wherein the phenylalanine corresponding to position 17 of SEQ ID NO: 2 is replaced by said hydrophobic amino acid;
(c) a polypeptide encoded by a nucleic acid molecule hybridizing under stringent conditions to the complementary strand of a nucleic acid molecule encoding the polypeptide as defined in (a) or (b);
(d) a polypeptide having at least 55% identity to the polypeptide of any one of (a) to (c) and functioning as an inhibitor of angiogenesis; and
(e) a polypeptide that functions as an inhibitor of angiogenesis comprising an amino acid sequence encoded by a nucleic acid molecule being degenerate as a result of the genetic code to the nucleotide sequence of a nucleic acid molecule as defined in any one of (a), (c) and (d).

**12.** The polypeptide of any one of claims 8 to 11, wherein said modified vascular endothelial growth factor A (VEGF-A) is selected from the group consisting of SEQ ID NO: 4, SEQ ID NO: 6, SEQ ID NO: 10, SEQ ID NO: 12, SEQ ID NO: 13 and SEQ ID NO: 14.

**13.** A non-human transgenic animal, wherein said transgenic animal comprises the modified vascular endothelial growth factor A (VEGF-A) polypeptide of any one of claims 8 to 12.

**14.** The non-human transgenic animal of claim 13, wherein said transgenic animal comprises an endogenous vascular endothelial growth factor A (VEGF-A) gene, wherein said endogenous vascular endothelial growth factor A (VEGF-A) gene comprises an artificially introduced mutation, wherein the expression of the mutated endogenous vascular endothelial growth factor A (VEGF-A) gene results in a non-functional vascular endothelial growth factor A (VEGF-A) polypeptide.

**15.** The non-human transgenic animal of claim 13 or 14, wherein said transgenic animal is a zebrafish.

**16.** The non-human transgenic animal of any one of claims 13 to 15, wherein said mutated endogenous vascular growth factor-A (VEGF-A) gene has a sequence as shown in SEQ ID NO: 32.

Figure 1.

a

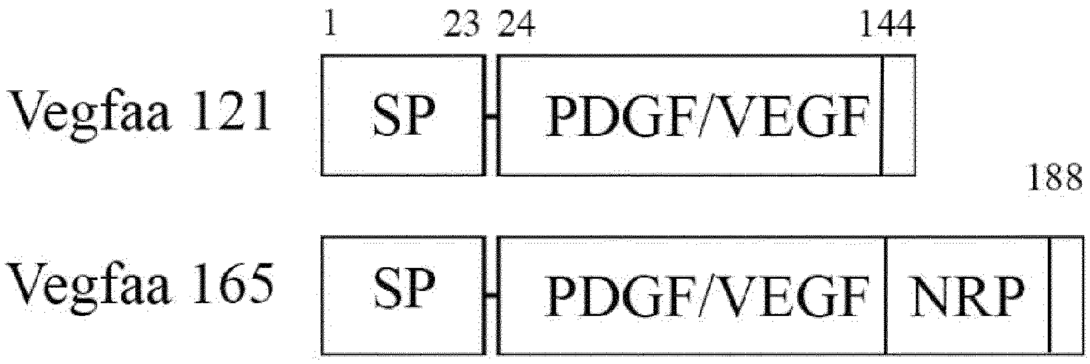


b

indel in zebrafish *vegfaa* locus

```
wt  ACATCATCCAGGAGTATCCCGATGAGATCG
Δ10 ACATCATCCAG----------ATGAGATCG
```

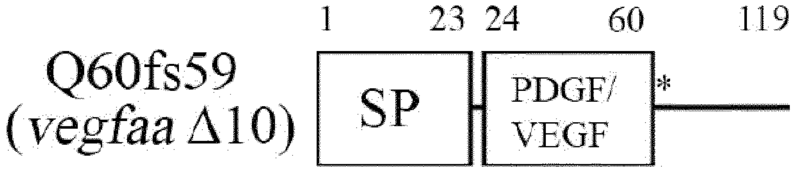

**Figure 1. continued 1.**

**C**

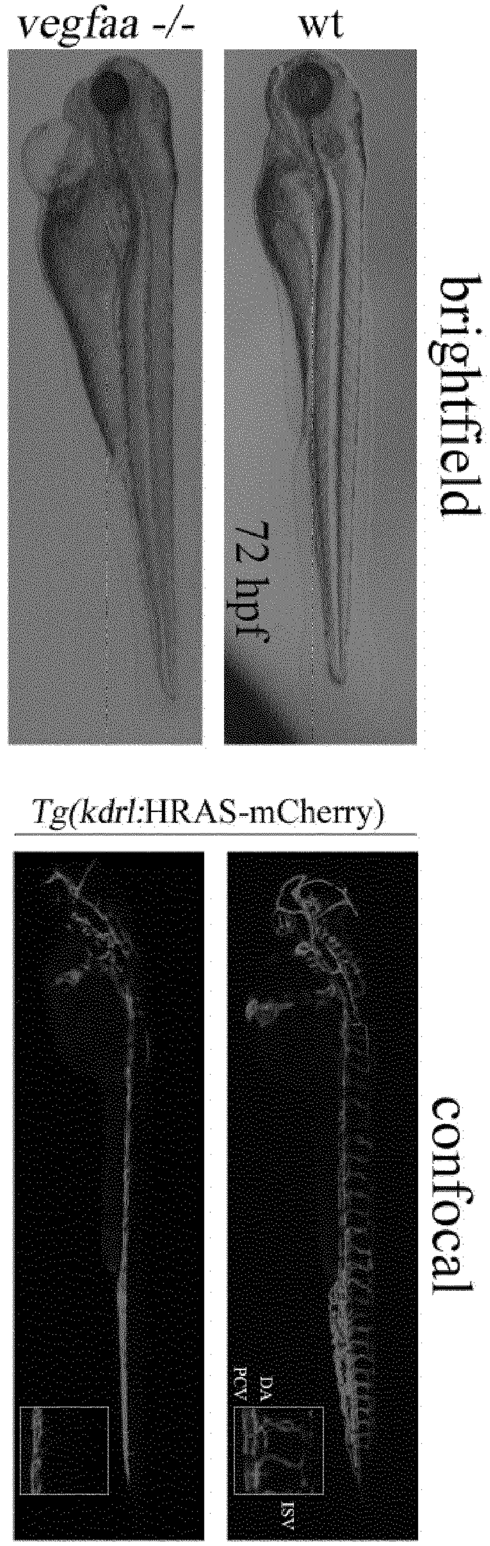

Figure 2.

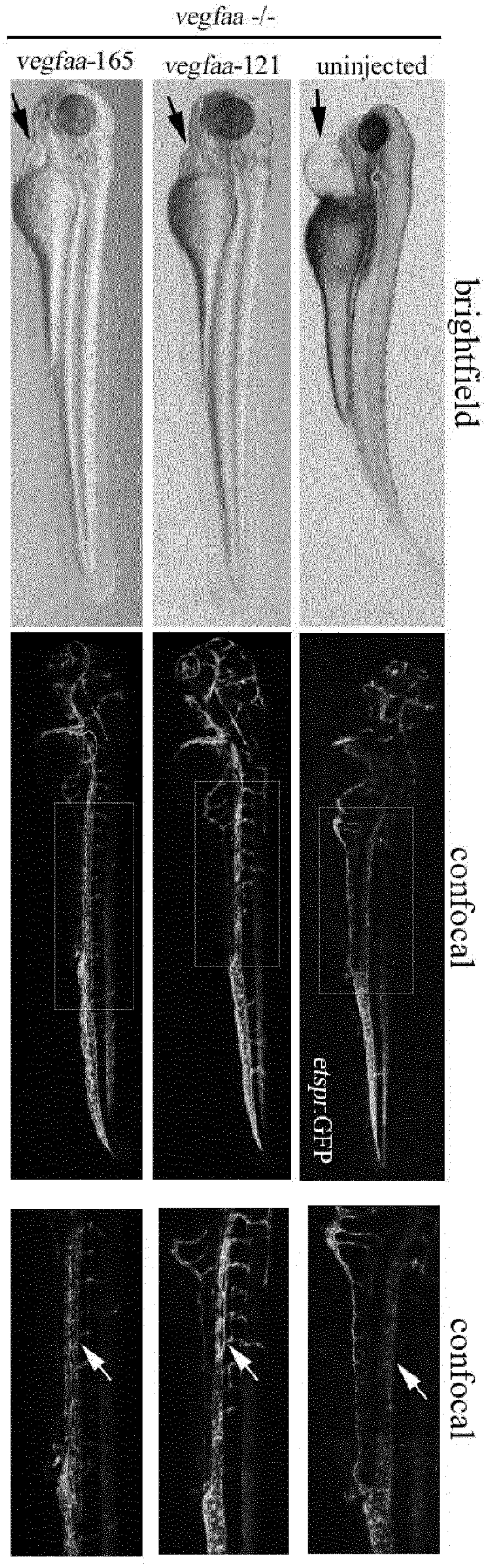
vegfaa -/-
vegfaa-165
vegfaa-121
uninjected
brightfield
confocal
etspr:GFP
confocal

Figure 3.

A

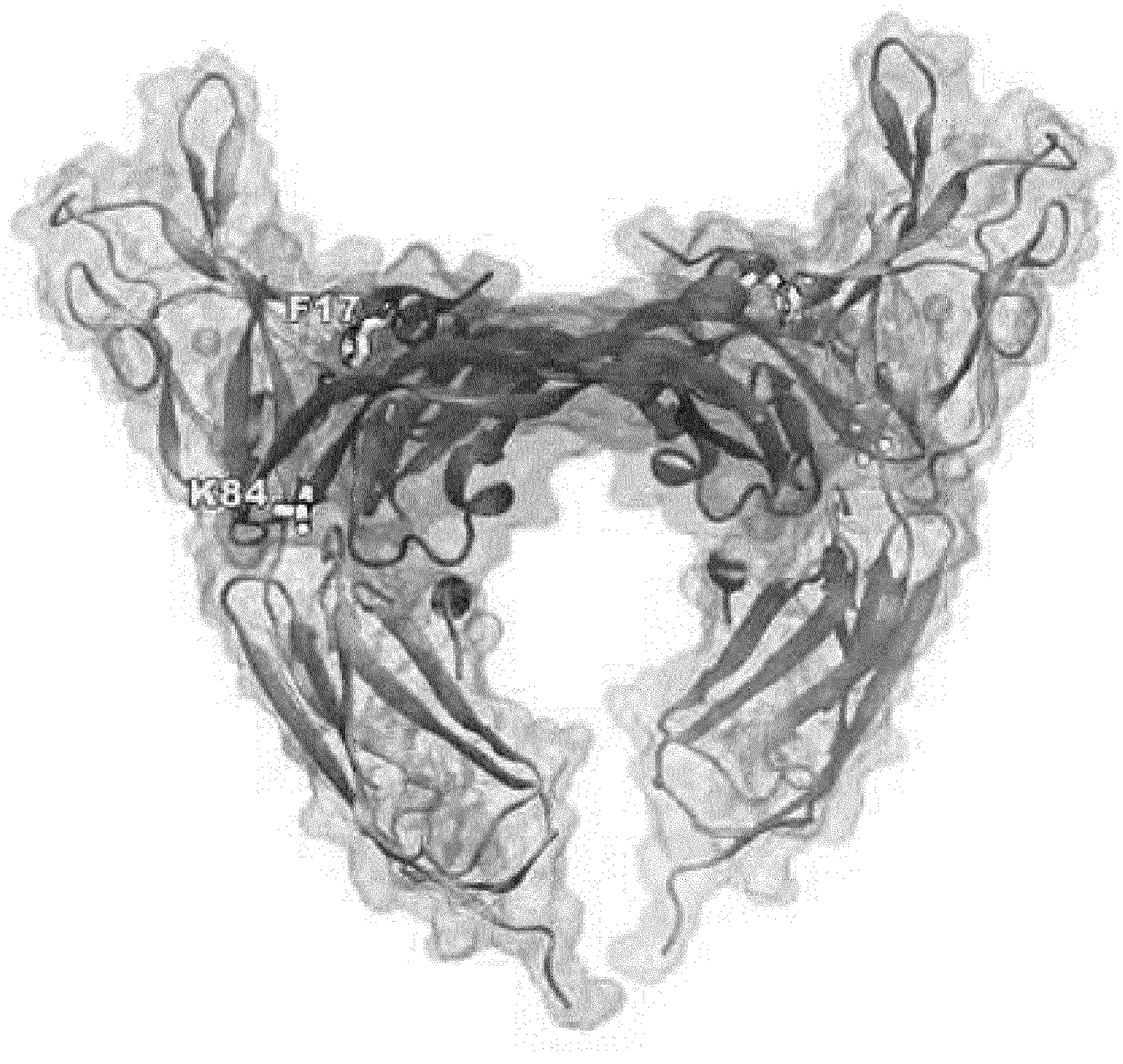

**Figure 3. continued 1.**

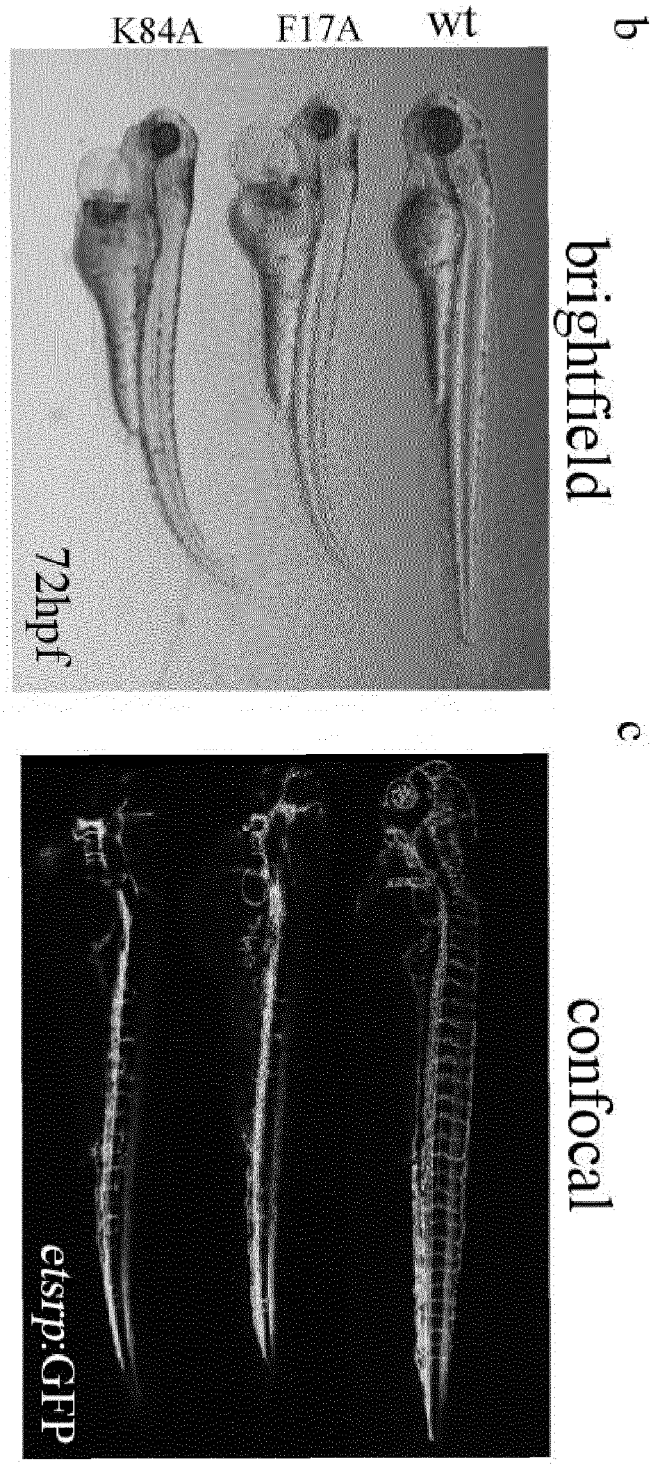

**Figure 3. continued 2.**

**D**

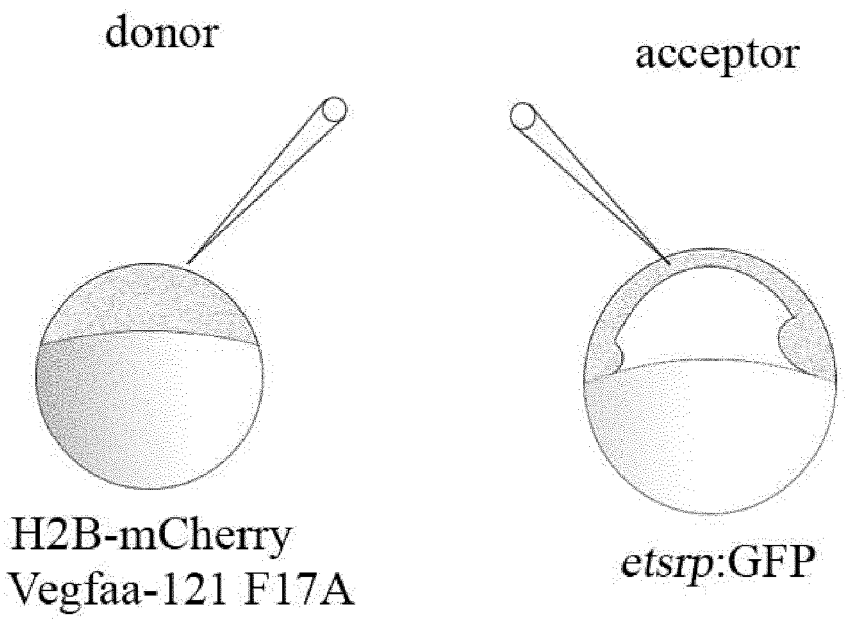


**E**

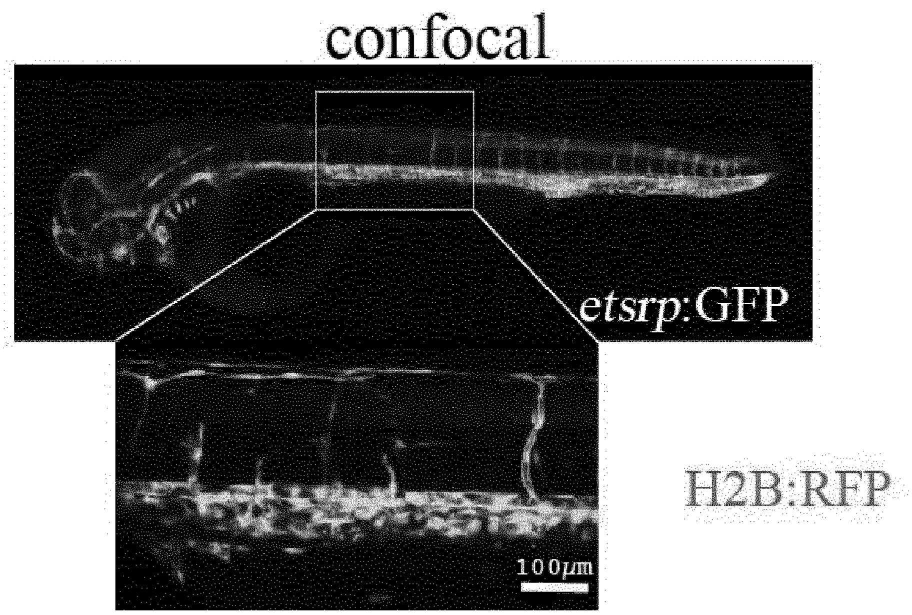

Figure 4.

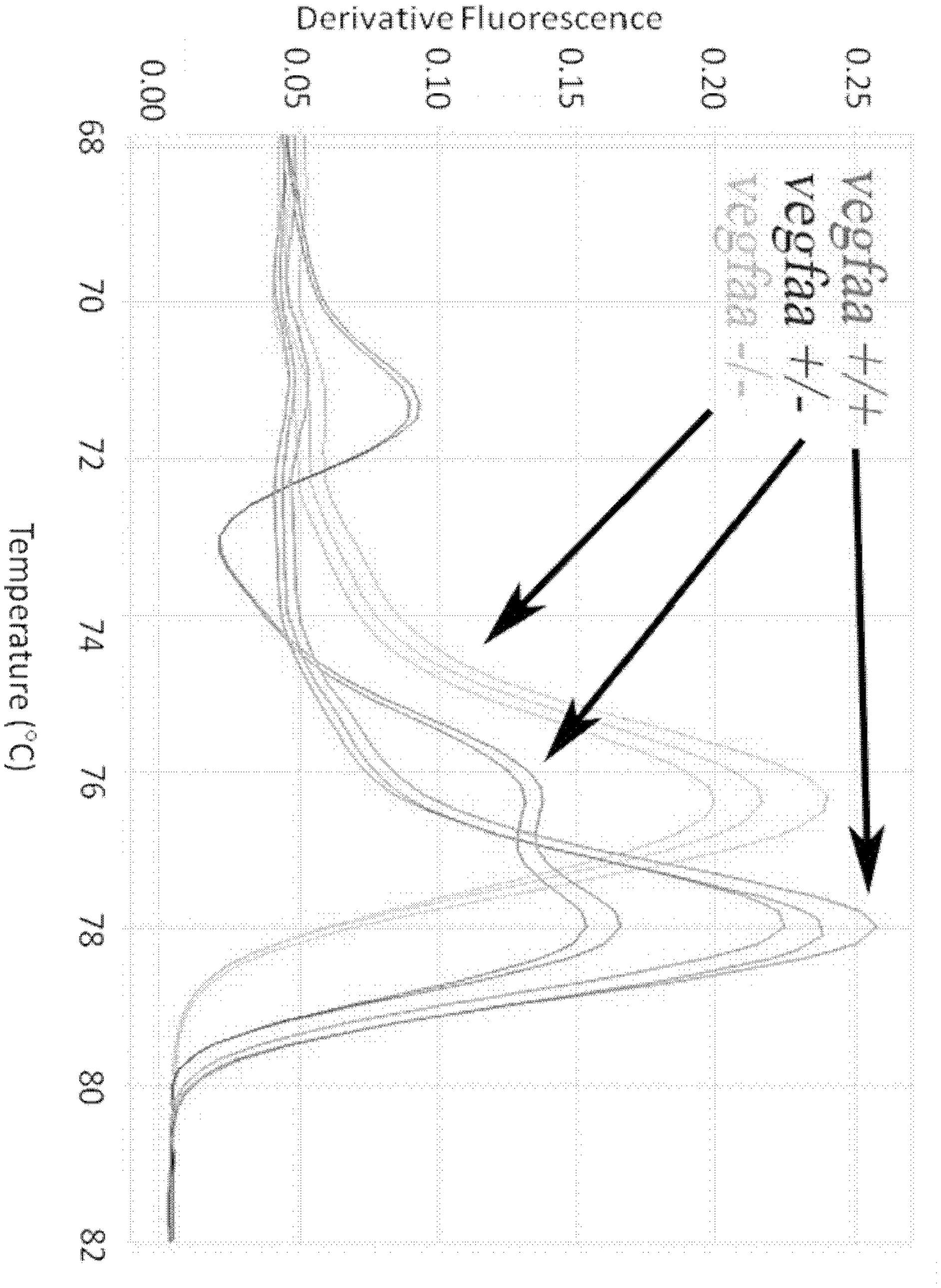
Derivative Fluorescence
0.00
0.05
0.10
0.15
0.20
0.25
68
70
72
74
76
78
80
82
Temperature (°C)
vegfaa +/+
vegfaa +/-
vegfaa -/-

**Figure 5.**

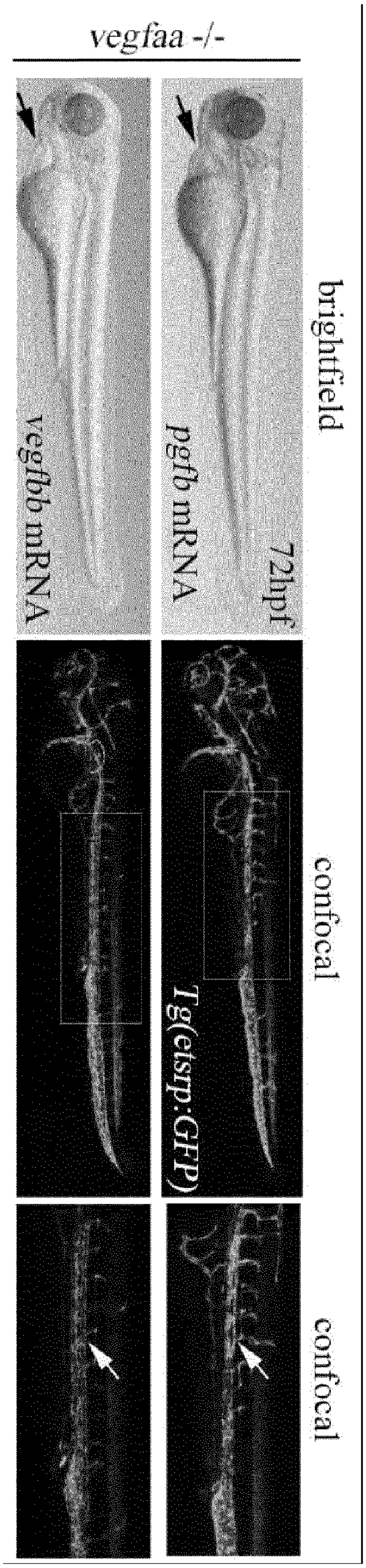
vegfaa -/-
brightfield
confocal
confocal
72hpf
pgfb mRNA
vegfb mRNA
Tg(etsrp:GFP)

Europäisches Patentamt
European Patent Office
Office européen des brevets

# EUROPEAN SEARCH REPORT

Application Number
EP 16 15 9048

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br><br><br>Y | WO 97/08313 A1 (GENENTECH INC [US]; KEYT BRUCE [US]; NGUYEN FRANCIS HUNG [US]; FERRARA) 6 March 1997 (1997-03-06)<br>* page 8; figures 1A,5,10,16-18; sequence K84A *<br>----- | 1-6,8-16<br><br><br>7,12 | INV.<br>A61K38/18<br>C07K14/515 |
| X<br><br><br><br><br><br>Y | LI B ET AL: "Receptor-selective Variants of Human Vascular Endothelial Growth Factor", JOURNAL OF BIOLOGICAL CHEMISTRY, AMERICAN SOCIETY FOR BIOCHEMISTRY AND MOLECULAR BIOLOGY, US, vol. 275, no. 38, 22 September 2000 (2000-09-22), pages 29823-29828, XP002993616, ISSN: 0021-9258, DOI: 10.1074/JBC.M002015200<br>* table I *<br>----- | 1-16<br><br><br><br><br><br>7,12 | |
| X<br><br><br><br><br><br>Y | FUH G ET AL: "REQUIREMENTS FOR BINDING AND SIGNALLING OF THE KINASE DOMAIN RECEPTOR FOR VASCULAR ENDOTHELIAL GROWTH FACTOR", JOURNAL OF BIOLOGICAL CHEMISTRY, AMERICAN SOCIETY FOR BIOCHEMISTRY AND MOLECULAR BIOLOGY, US, vol. 273, no. 18, 1 May 1998 (1998-05-01), pages 11197-11204, XP000876741, ISSN: 0021-9258, DOI: 10.1074/JBC.273.18.11197<br>* figure 1; table III *<br>-----<br>-/-- | 1-16<br><br><br><br><br><br>7,12 | TECHNICAL FIELDS SEARCHED (IPC)<br>A61K<br>C07K |

The present search report has been drawn up for all claims

1

| Place of search | Date of completion of the search | Examiner |
|---|---|---|
| Munich | 1 July 2016 | Hars, Jesko |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 15 9048

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SIEMEISTER G ET AL: "AN ANTAGONISTIC VASCULAR ENDOTHELIAL GROWTH FACTOR (VEGF) VARIANT INHIBITS VEGF-STIMULATED RECEPTOR AUTOPHOSPHORYLATION AND PROLIFERATION OF HUMAN ENDOTHELIAL CELLS", PROCEEDINGS OF THE NATIONAL ACADEMY OF SCIENCES, NATIONAL ACADEMY OF SCIENCES, US, vol. 95, no. 8, 14 April 1998 (1998-04-14), pages 4625-4629, XP000876737, ISSN: 0027-8424, DOI: 10.1073/PNAS.95.8.4625 | 1-16 | |
| Y | * figure 1B * | 7,12 | |
| | ----- | | |
| X | MULLER Y A ET AL: "VASCULAR ENDOTHELIAL GROWTH FACTOR: CRYSTAL STRUCTURE AND FUNCTIONAL MAPPING OF THE KINASE DOMAIN RECEPTOR BINDING SITE", PROCEEDINGS OF THE NATIONAL ACADEMY OF SCIENCES, NATIONAL ACADEMY OF SCIENCES, US, vol. 94, no. 14, 1 July 1997 (1997-07-01), pages 7192-7197, XP000918906, ISSN: 0027-8424, DOI: 10.1073/PNAS.94.14.7192 | 1-16 | TECHNICAL FIELDS SEARCHED (IPC) |
| Y | * tables 2,3 * | 7,12 | |
| | ----- | | |
| | -/-- | | |

The present search report has been drawn up for all claims

1

| Place of search | Date of completion of the search | Examiner |
|---|---|---|
| Munich | 1 July 2016 | Hars, Jesko |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 15 9048

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GONG B ET AL: "Characterization of the zebrafish vascular endothelial growth factor A gene: comparison with vegf-A genes in mammals and Fugu", BIOCHIMICA ET BIOPHYSICA ACTA . GENE STRUCTURE AND EXPRESSION, ELSEVIER, AMSTERDAM, NL, vol. 1676, no. 1, 5 January 2004 (2004-01-05), pages 33-40, XP004485618, ISSN: 0167-4781, DOI: 10.1016/J.BBAEXP.2003.10.006 * figure 2 * ----- | 7,12 | |

TECHNICAL FIELDS SEARCHED (IPC)

The present search report has been drawn up for all claims

1

| Place of search | Date of completion of the search | Examiner |
|---|---|---|
| Munich | 1 July 2016 | Hars, Jesko |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 16 15 9048

This annex lists the patent family members relating to the patent documents cited in the above-mentioned European search report.
The members are as contained in the European Patent Office EDP file on
The European Patent Office is in no way liable for these particulars which are merely given for the purpose of information.

01-07-2016

| Patent document cited in search report | Publication date | Patent family member(s) | Publication date |
|---|---|---|---|
| WO 9708313 A1 | 06-03-1997 | AT 399203 T | 15-07-2008 |
| | | AU 6857296 A | 19-03-1997 |
| | | CA 2230144 A1 | 06-03-1997 |
| | | DK 0851920 T3 | 22-09-2008 |
| | | EP 0851920 A1 | 08-07-1998 |
| | | ES 2309990 T3 | 16-12-2008 |
| | | JP 3456706 B2 | 14-10-2003 |
| | | JP H10511557 A | 10-11-1998 |
| | | US 6020473 A | 01-02-2000 |
| | | US 6057428 A | 02-05-2000 |
| | | US 2006024785 A1 | 02-02-2006 |
| | | US 2007082847 A1 | 12-04-2007 |
| | | WO 9708313 A1 | 06-03-1997 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 1998016551 A
- WO 2007044534 A2
- WO 0153345 A1
- WO 2006119035 A2
- WO 2010102380 A1
- WO 1997008313 A
- WO 2007062466 A
- US 4946778 A
- US 5225539 A
- US 5693761 A
- US 6407213 B
- US 7129084 B
- US 07290975 B
- US 07310252 B
- US 20030229208 A
- WO 9316185 A
- US 5571894 A
- US 5587458 A
- US 5641870 A
- US 20020004586 A
- WO 0177342 A
- WO 9429469 A
- WO 9700957 A
- WO 9420627 A
- US 5580859 A
- US 5589466 A
- EP 1071752 B1
- US 3773919 A
- EP 58481 A
- EP 133988 A
- DE 3218121
- EP 52322 A
- EP 36676 A
- EP 88046 A
- EP 143949 A
- EP 142641 A
- JP 58118008 A
- US 4485045 A
- US 4544545 A
- EP 102324 A

### Non-patent literature cited in the description


- **ALTSCHUL.** *Nucl. Acids Res.,* 1997, vol. 25, 3389-3402
- **ALTSCHUL.** *J. Mol. Evol.,* 1993, vol. 36, 290-300
- **ALTSCHUL.** *J. Mol. Biol.,* 1990, vol. 215, 403-410
- **SIEVERS.** *Curr Protoc Bioinformatics,* 2014, vol. 48, 3.13.1-3.13.16
- **SAMBROOK.** Molecular Cloning; A Laboratory Manual. Cold Spring Harbour Laboratory Press, Cold Spring Harbour, 1989
- **AUSUBEL.** Current Protocols in Molecular Biology. Green Publishing Associates; and Wiley Interscience, 1989
- **SAMBROOK, RUSSELL.** Molecular Cloning, A Laboratory Manual. Cold Spring Harbor Laboratory, 2001
- **AUSUBEL.** Current Protocols in Molecular Biology. Green Publishing Associates and Wiley Interscience, 1989
- Nucleic acid hybridization, a practical approach. IRL Press Oxford, 1985
- **THOMPSON.** *Nucl. Acids Res.,* 1994, vol. 2, 4673-4680
- *Brutlag Comp. App. Biosci.,* 1990, vol. 6, 237-245
- **HENIKOFF.** *PNAS,* 1989, vol. 89, 10915
- **STRYER.** Biochemistry. Freeman and Company, 1995
- **MULLER et al.** *Structure,* 1997, vol. 5, 1325-1338
- **STAUFFER et al.** *Journal of Biomolecular NMR,* 2002, vol. 23, 57-61
- **HARLOW ; LANE.** Antibodies, A Laboratory Manual. Cold Spring Harbor Laboratory Press, 1988
- **HARLOW ; LANE.** Using Antibodies: A Laboratory Manual. Cold Spring Harbor Laboratory Press, 1999
- **ALTSHULER.** *Biochemistry,* 2010, vol. 75, 1584-605
- **HOLLIGER.** *Nature Biotechnology,* 2005, vol. 23, 1126-36
- **KÖHLER ; MILSTEIN.** *Nature,* 1975, vol. 256, 495
- **KOZBOR.** *Immunology Today,* 1983, vol. 4, 72
- **COLE et al.** Monoclonal Antibodies and Cancer Therapy. Alan R. Liss, Inc, 1985, 77
- **JONES.** *Nature,* 1986, vol. 321, 522-525

- **REICHMANN.** *Nature,* 1998, vol. 332, 323-327
- *Presta Curr Op Struct Biol,* 1992, vol. 2, 593-596
- **QUEEN.** *PNAS,* 1989, 10029-10033
- **SCHIER.** *Human Antibodies Hybridomas,* 1996, vol. 7, 97
- **MALMBORG.** *J. Immunol. Methods,* 1995, vol. 183, 7
- **SELA.** *Science,* 1969, vol. 166, 1365
- **LAVER.** *Cell,* 1990, vol. 61, 553
- **MORIMOTO et al.** *Journal of Biochemical and Biophysical Methods,* 1992, vol. 24, 107-117
- **BRENNAN et al.** *Science,* 1985, vol. 229, 81
- **CARTER et al.** *Bio/Technology,* 1992, vol. 10, 163-167
- **MILLSTEIN et al.** *Nature,* 1983, vol. 305, 537-539
- **SHALABY et al.** *J. Exp. Med.,* 1992, vol. 175, 217-225
- **HOLLINGER et al.** *Proc. Natl. Acad. Sci. USA,* 1993, vol. 90, 6444-6448
- **TUTT et al.** *J. Immunol.,* 1991, vol. 147, 60
- **GIORDANO.** *Nature Medicine,* 1996, vol. 2, 534-539
- **SCHAPER.** *Circ. Res.,* 1996, vol. 79, 911-919
- **ANDERSON.** *Science,* 1992, vol. 256, 808-813
- **ISNER.** *Lancet,* 1996, vol. 348, 370-374
- **MUHLHAUSER.** *Circ. Res.,* 1995, vol. 77, 1077-1086
- **WANG.** *Nature Medicine,* 1996, vol. 2, 714-716
- **SCHAPER.** *Current Opinion in Biotechnology,* 1996, vol. 7, 635-640
- **VERMA.** *Nature,* 1997, vol. 389, 239-242
- **STEWART et al.** Solid Phase Peptide Synthesis. WH Freeman Co, 1969
- **MERRIFIELD.** *J. Am. Chem. Soc.,* 1963, vol. 85, 2149-2154
- **MACK et al.** *PNAS,* 1995, vol. 92, 7021-7025
- **RAUM et al.** *Cancer Immunol Immunother,* 2001, vol. 50 (3), 141-150
- **SMITH.** *J. Virol.,* 1983, vol. 46, 584
- **ENGELHARD.** *Proc. Nat. Acad. Sci. USA,* 1994, vol. 91, 3224-3227
- **REISS.** *Plant Physiol. (Life Sci. Adv.),* 1994, vol. 13, 143-149
- **HERRERA-ESTRELLA.** *EMBO J.,* 1983, vol. 2, 987-995
- **MARSH.** *Gene,* 1984, vol. 32, 481-485
- **HARTMAN.** *Proc. Natl. Acad. Sci. USA,* 1988, vol. 85, 8047
- **TAMURA.** *Biosci. Biotechnol. Biochem.,* 1995, vol. 59, 2336-2338
- **GIACOMIN.** *Pl. Sci.,* 1996, vol. 116, 59-72
- **SCIKANTHA.** *J. Bact.,* 1996, vol. 178, 121
- **GERDES.** *FEBS Lett.,* 1996, vol. 389, 44-47
- **JEFFERSON.** *EMBO J.,* 1987, vol. 6, 3901-3907
- **VERMA.** *Nature,* 1994, vol. 389, 239
- **ONODERA.** *Blood,* 1998, vol. 91, 30-36
- **VERMA.** *Gene Ther.,* 1998, vol. 5, 692-699
- **NABEL.** *Ann. N.Y. Acad. Sci.,* 1997, vol. 811, 289-292
- **VERZELETTI.** *Hum. Gene Ther.,* 1998, vol. 9, 2243-51
- **NAGY.** *Proc. Natl. Acad. Sci. USA,* 1993, vol. 90, 8424-8428
- **SCOPES.** Protein Purification. Springer-Verlag, 1982
- **MAURICE S. BROZZO et al.** Thermodynamic and structural description of allosterically regulated VEGFR-2 dimerization. *Blood,* 2012, vol. 16, 1781-1788
- **TAYLOR PC.** Antibody therapy for rheumatoid arthritis. *Curr Opin Pharmacol.,* 2003, vol. 3 (3), 323-328
- **TAYLOR PC.** *Curr Opin Pharmacol.,* 2003, vol. 3 (3), 323-328
- **ROXANA A.** *Maedica.,* 2006, vol. 1 (1), 63-65
- **SIDMAN, U. et al.** *Biopolymers,* 1983, vol. 22, 547-556
- **R. LANGER et al.** *J. Biomed. Mater. Res.,* 1981, vol. 15, 167-277
- **R. LANGER.** *Chem. Tech.,* 1982, vol. 12, 98-105
- **EPSTEIN et al.** *Proc. Natl. Acad. Sci. (USA),* 1985, vol. 82, 3688-3692
- **HWANG et al.** *Proc. Natl. Acad. Sci. (USA),* 1980, vol. 77, 4030-4034
- **WUTS PG ; GREENE TW.** Greene's protective groups in organic synthesis. John Wiley & Sons, 2006
- **N. FERRARA et al.** *Nature Medicine,* 2003, vol. 9, 669-676
- **YVES A. MULLER et al.** *Proc Natl Acad Sci USA.,* 1997, vol. 94, 7192-7197
- **MAURICE S. BROZZO et al.** *Blood,* 2012, vol. 16, 1781-1788
- **T. CERMAK et al.** *Nucleic Acids Res.,* 2011, vol. 39, e82
- **N. D. LAWSON et al.** *Dev Cell.,* 2002, vol. 3, 127-136
- **B. HERZOG et al.** *Development,* 2011, vol. 141, 556-562
- **YVES A. MULLER et al.** *Development,* 2014, vol. 141, 556-562
- **T. MAKINEN et al.** *J Biol Chem.,* 1999, vol. 274, 21217-21222
- **HANS W. CHRISTINGER et al.** *J Biol Chem.,* 2004, vol. 279, 10382-10388
- **B. OLOFSSON et al.** *Proc. Natl. Acad. Sci. USA,* 1998, vol. 95, 11709

- **M. ERRICO et al.** *J Biol Chem.,* 2004, vol. 279, 43929-43939
- **PHILIP J. ROSENFELD et al.** *N Engl J Med,* 2006, vol. 355, 1419-1431
- **M. B. VELDMAN ; S.LIN.** *Circ Res.,* 2012, vol. 110, 220-229
- **N. C. CHI et al.** *Plos Biol.,* 2008, vol. 6 (5), e109
- **E. L. DOYLE et al.** *Plos One,* 2013, vol. 8, e82120
- **J J CHEN et al.** *Eye,* 2011, vol. 25, 1504-1511